# EUROPEAN PATENT APPLICATION

(11) **EP 4 541 804 A1**
(43) Date of publication of application: **23.04.2025**
(21) Application number: 23823155.9
(22) Date of filing: 13.06.2023
(51) Int. Cl.: C07H 21/02, C07H 19/20, A61K 31/713

(54) **CYCLIC PHOSPHONATE-MODIFIED NUCLEOTIDE**

(30) Priority: 14.06.2022 CN 202210678937; 13.01.2023 CN 202310072481
(71) Applicant: Rona Bioscience, Limited, Admiralty, Hong Kong (HK)
(72) Inventor: HUANG, Jinyu, Shanghai 201315 (CN); ZOU, Hao, Shanghai 201315 (CN); LIU, Junkai, Shanghai 201315 (CN)
(74) Representative: Cabinet Becker et Associés
(86) International application number: PCT/CN2023/099987
(87) International publication number: WO 2023/241587

(57) **Abstract**

The present invention provides an oligonucleotide having a cyclic phosphonate modification. The oligonucleotide of the present invention exhibits one or more among enhanced stability, reduced off-target toxicity, and enhanced effectiveness.

## Description

### FIELD OF THE INVENTION

The present invention relates to the technical field of medical and pharmaceutical science, and particularly relates to a double-stranded RNA having a cyclic phosphonate structure.

### BACKGROUND OF THE INVENTION

RNA interference is a phenomenon of specific and highly efficient degradation of the target mRNA induced by a double-stranded RNA (dsRNA, also known as siRNA).

The 5'-phosphate of the guide strand (antisense strand) of siRNA stabilizes the complex formed by the guide strand and Ago2 through electrostatic interactions with cationic amino acid residues near the interface between the MID and PIWI domains of the Ago2 protein. Therefore, the 5'-phosphate of the guide strand of siRNA is essential for RNAi-based gene silencing. However, this 5'-phosphate can be cleaved by endogenous phosphatases, resulting in the loss of the phosphate group at the 5' end and a significant reduction in siRNA activity.

The *in vivo* activity of siRNA can be significantly improved by introducing a phosphonate-modified nucleotide at the ends of the small interfering RNA (siRNA), particularly at the 5' end of the antisense strand. For example, both WO2011139702A2 and WO2013033230A1 disclose a nucleotide comprising 5'-vinylphosphonate (E-VP or VP), which can bind to Ago2 to replace 5'-phosphate and is highly resistant to endogenous phosphatases, thereby improving the activity and/or stability of siRNA.

WO2017214112A1 discloses a nucleotide comprising 5'-cyclo-phosphonate.

However, the inventors have found that 5'-E-VP is connected to the sugar moeity via a single C-C bond, and the C-C bond can still freely rotate, allowing the phosphonate group to be at different positions relative to the sugar moeity (see the figure below, where the *ap* position represents the conformation in which the unmodified 5'-phosphate binds to the Ago2 protein).

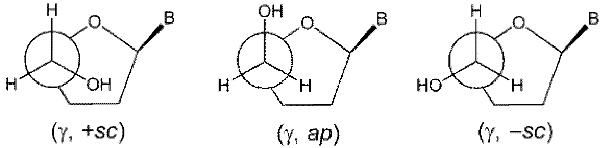

A substantial proportion of the conformations therein (e.g., *+sc* and *-sc*) may be unfavorable for binding to the cationic region of Ago2. Therefore, there is a need in this field to develop phosphonate modifications that can lock the Ago2 binding conformation.

### SUMMARY OF THE INVENTION

The inventors have surprisingly discovered that restricting rotation of the terminal phosphonate group using a rigid loop structure and locking it in the Ago2 binding conformation is advantageous for further improving siRNA activity.

In one aspect, the present invention relates to an oligonucleotide, or a pharmaceutically acceptable salt, tautomer or stereoisomer thereof, wherein the oligonucleotide comprises at its 5' end a structure of formula (I): Wherein, 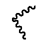
represents attachment to the remainder of the oligonucleotide, preferably to a modified or unmodified nucleoside;
X and Y are each independently selected from CRₐR_{b} or (CRₐR_{b})₂, preferably CRₐR_{b};
Z is CRₐ;
Rₐ and R_{b} are independently selected from H, D, halogen, OH, NH₂, CN, C₁₋₆ alkyl, C₁₋₆ haloalkyl, C₁₋₆ alkoxyl, C₁₋₆ haloalkoxyl, C₂₋₆ alkenyl, or C₂₋₆ alkynyl; wherein Rₐ and R_{b} are optionally further substituted with 1, 2, 3, 4, or 5 independently selected R#;
R₁ is selected from O or S;
R₂ and R₃ are independently selected from OH, SH, NH₂, C₁₋₆ alkyl, C₁₋₆ haloalkyl, C₁₋₆ alkoxyl, or C₁₋₆ haloalkoxyl; wherein the R₂ and R₃ are optionally further substituted with 1, 2, 3, 4, or 5 independently selected R#;
R# is selected from H, D, halogen, C₁₋₆ alkyl, C₁₋₆ haloalkyl, C₂₋₆ alkenyl, or C₂₋₆ alkynyl;
wherein each of the aforementioned groups is optionally substituted by 1, 2, 3, 4, 5, or more deuterium atoms or fully deuterated.

Without wishing to be bound by any particular theory, the structure of the present invention contains a four-membered carbocyclic ring. This relatively rigid structure enables the oligonucleotide (particularly the phosphonate group at its 5' end) of the present invention to better bind to the Ago2 protein, thereby improving the activity of the oligonucleotide.

Through molecular simulation, we have observed that the compounds of the present invention lock the phosphonate in an *ap* conformation highly similar to the unmodified 5'-phosphate-bound Ago2, wherein the phosphonate group perfectly matches Arg812, Lys570, Lys566, and Lys533, forming multiple salt bridges and hydrogen bonds, as well as two hydrogen bonds with Tyr529 and Cys526, respectively. In addition, the base (uracil) in the spirocyclic phosphonate compound forms two hydrogen bonds with the backbone amides of Thr526 and Gly524, respectively. The combined action of these structures stabilizes the binding of the compounds of the present invention to the Ago2 protein.

The molecular simulation has shown that, similar to spiro compounds, the sugar moeity fused with a highly rigid aromatic ring (including five-membered and six-membered aromatic rings) locks the phosphonate group at the 5' end in a conformation that closely mimics the *ap* conformation of natural 5'-phosphate, with the phosphonate group perfectly matching Arg812, Lys570, Lys566, and Lys533, forming multiple salt bridges and hydrogen bonds, as well as two hydrogen bonds with Tyr529 and Cys526, respectively. Likewise, the base uracil on the sugar moeity forms two hydrogen bond with the backbone amides of Thr526 and Gly524, respectively. The combined action of these structures stabilizes the binding of the compounds of the present invention to the Ago2 protein.

### DETAILED DESCRIPTION OF THE INVENTION

### Definitions

### Chemical Definition

Definitions of specific functional groups and chemical terms are described in more detail as follows.

When a numerical range is provided, it is intended that a particular numerical point and sub-range within said range be included. For example, "C₁₋₆ alkyl" includes alkyls C₁, C₂, C₃, C₄, C₅, C₆, C₁₋₆, C₁₋₅, C₁₋₄, C₁₋₃, C₁₋₂, C₂₋₆, C₂₋₅, C₂₋₄, C₂₋₃, C₃₋₆, C₃₋₅, C₃₋₄, C₄₋₆, C₄₋₅, and C₅₋₆.

"C₁₋₆ alkyl" refers to any straight-chain or branched saturated hydrocarbon group with 1 to 6 carbon atoms. In some embodiments, C₁₋₄ alkyl and C₁₋₂ alkyl are preferred. Examples of C₁₋₆ alkyl described herein include, but are not limited to: methyl (C₁), ethyl (C₂), n-propyl (C₃), isopropyl (C₃), n-butyl (C₄), tert-butyl (C₄), sec-butyl (C₄), isobutyl (C₄), n-pentyl (C₅), 3-pentyl (C₅), pentyl (C₅), neopentyl (C₅), 3-methyl-2-butyl (C₅), tert-pentyl (C₅) and n-hexyl (C₆). The term "C₁₋₆ alkyl" also includes any heteroalkyl in which one or more (e.g., 1, 2, 3, or 4) carbon atoms are replaced with heteroatoms (e.g., oxygen, sulfur, nitrogen, boron, silicon, phosphorus). The alkyls may be optionally substituted by one or more substituents, for example, 1 to 5 substituents, 1 to 3 substituents, or 1 substituent. The conventional abbreviations for alkyl include: Me(-CH₃), Et(-CH₂CH₃), iPr(-CH(CH₃)₂), nPr(-CH₂CH₂CH₃), n-Bu(-CH₂CH₂CH₂CH₃) or i-Bu(-CH₂CH(CH₃)₂).

"C₂₋₆ alkenyl" refers to a straight-chain or branched hydrocarbon group with 2 to 6 carbon atoms and at least one carbon-carbon double bond. In some embodiments, C₂₋₄ alkenyl is preferred. Examples of C₂₋₆ alkenyl include, but are not limited to: vinyl (C₂), 1-propenyl (C₃), 2-propenyl (C₃), 1-butenyl (C₄), 2-butenyl (C₄), butadienyl (C₄), pentenyl (C₅), pentadienyl (C₅), and hexenyl (C₆). The term "C₂₋₆ alkenyl" also includes any heteroalkenyl in which one or more (e.g., 1, 2, 3, or 4) carbon atoms are replaced with heteroatoms (e.g., oxygen, sulfur, nitrogen, boron, silicon, phosphorus). The alkenyls may be optionally substituted by one or more substituents, for example, 1 to 5 substituents, 1 to 3 substituents, or 1 substituent.

"C₂₋₆ alkynyl" refers to a straight-chain or branched hydrocarbon group with 2 to 6 carbon atoms and at least one carbon-carbon triple bond and optionally one or more carbon-carbon double bonds. In some embodiments, C₂₋₄ alkynyl are preferred. Examples of C₂₋₆ alkynyl include, but are not limited to: ethynyl (C₂), 1-propynyl (C₃), 2-propynyl (C₃), 1-butynyl (C₄), 2-butynyl (C₄), pentynyl (C₅), and hexynyl (C₆). The term "C₂₋₆ alkynyl" also includes any heteroalkynyl in which one or more (e.g., 1, 2, 3, or 4) carbon atoms are replaced with heteroatoms (e.g., oxygen, sulfur, nitrogen, boron, silicon, phosphorus). The alkynyls may be optionally substituted by one or more substituents, for example, 1 to 5 substituents, 1 to 3 substituents, or 1 substituent.

"Halo-" or "halogen" refers to (substitution by) fluorine (F), chlorine (Cl), bromine (Br) and iodine (I).

Accordingly, "C₁₋₆ haloalkyl" refers to the aforementioned "C₁₋₆ alkyl", with one or more halogen groups. In some embodiments, a C₁₋₄ haloalkyl is particularly preferred, and a C₁₋₂ haloalkyl is even more preferred. Exemplary haloalkyls include, but are not limited to: -CF₃, -CH₂F, -CHF₂, -CHFCH₂F, -CH₂CHF₂, -CF₂CF₃, -CCl₃, -CH₂Cl, -CHCl₂, and 2,2,2-trifluoro-1,1-dimethyl-ethyl. The haloalkyls may be substituted at any substitutable connection site, for example 1 to 5 substituents, 1 to 3 substituents or 1 substituent.

"C₁₋₆ alkoxyl" refers to an -O-R group, wherein R is as defined above for "C₁₋₆ alkyl".

"C₁₋₆ haloalkoxyl" refers to an -O-R group, wherein R is as defined above for "C₁₋₆ alkyl".

"C₅₋₆ cycloalkyl" refers to a non-aromatic cyclic hydrocarbon group with 5 to 6 ring carbon atoms and no heteroatoms. A cycloalkyl herein also includes a ring system in which an aforementioned cycloalkyl ring is fused with one or more aryls or heteroaryls through any connection site(s) on the cycloalkyl ring; in this context, the number of carbons still represents the number of carbons in the cycloalkyl system. Exemplary cycloalkyls include, but are not limited to: cyclopentyl (C₅), cyclopentenyl (C₅), cyclohexyl (C₆), cyclohexenyl (C₆), and cyclohexadienyl (C₆). The cycloalkyls may be optionally substituted by one or more substituents, for example, 1 to 5 substituents, 1 to 3 substituents, or 1 substituent.

The term herein "5- to 6-membered heterocyclyl" refers to a group of a 5- to 6-membered non-aromatic ring system with ring carbon atom(s) and 1 to 3 ring heteroatoms, wherein each heteroatom is independently selected from nitrogen, oxygen, sulfur, boron, phosphorus and silicon. In said heterocyclyl containing one or more nitrogen atoms, the connection site may be a carbon or nitrogen atom as long as the valence permits. A heterocyclyl herein also includes a ring system in which an aforementioned heterocyclyl ring is fused to one or more cycloalkyls through any connection site(s) on the cycloalkyl ring, or said heterocyclyl includes a ring system in which an aforementioned heterocyclyl ring is fused to one or more aryls or heteroaryls through any connection site(s) on the heterocyclyl ring; in these contexts, the number of ring members still represents the number of ring members in the heterocyclyl ring system. Exemplary 5-membered heterocyclyls containing one heteroatom include, but are not limited to: tetrahydrofuryl, dihydrofuryl, tetrahydrothienyl, dihydrothienyl, pyrrolidinyl, dihydropyrrolyl, and pyrroli-2,5-dione. Exemplary 5-membered heterocyclyls containing two heteroatoms include, but are not limited to: dioxolanyl, oxasulfuranyl, disulfuranyl, and oxazolidin-2-one. Exemplary 5-membered heterocyclyls containing three heteroatoms include, but are not limited to: triazolinyl, oxadiazolinyl and thiadiazolinyl. Exemplary 6-membered heterocyclyls containing one heteroatom include, but are not limited to: piperidinyl, tetrahydropyranyl, dihydropyridyl and thianyl. Exemplary 6-membered heterocyclyls containing two heteroatoms include, but are not limited to: piperazinyl, morpholinyl, dithianyl, and dioxanyl. Exemplary 6-membered heterocyclyls containing three heteroatoms include, but are not limited to: triazinanyl. The heterocyclyls may be optionally substituted by one or more substituents, for example, 1 to 5 substituents, 1 to 3 substituents, or 1 substituent.

Alkyl, alkenyl, alkynyl, cycloalkyl, and heterocyclylas defined herein are optionally substituted groups.

Exemplary substituents on carbon atoms include, but are not limited to: halogen, -CN, -NO₂, -N₃, -SO₂H, -SO₃H, -OH, -OR^{aa}, -ON(R^{bb})₂, -N(R^{bb})₂, -N(R^{bb})₃⁺X⁻, -N(OR^{cc})R^{bb}, -SH, -SR^{aa}, -SSR^{cc}, -C(=O)R^{aa}, -CO₂H, -CHO, -C(OR^{cc})₂, -CO₂R^{aa}, -OC(=O)R^{aa}, -OCO₂R^{aa}, -C(=O)N(R^{bb})₂, -OC(=O)N(R^{bb})₂, -NR^{bb}C(=O)R^{aa}, -NR^{bb}CO₂R^{aa}, -NR^{bb}C(=O)N(R^{bb})₂, -C(=NR^{bb})R^{aa}, -C(=NR^{bb})OR^{aa}, -OC(=NR^{bb})R^{aa}, -OC(=NR^{bb})OR^{aa}, -C(=NR^{bb})N(R^{bb})₂, -OC(=NR^{bb})N(R^{bb})₂, -NR^{bb}C(=NR^{bb})N(R^{bb})₂, -C(=O)NR^{bb}SO₂R^{aa}, -NR^{bb}SO₂R^{aa}, -SO₂N(R^{bb})₂, -SO₂R^{aa}, -SO₂OR^{aa}, -OSO₂R^{aa}, -S(=O)R^{aa}, -OS(=O)R^{aa}, -Si(R^{aa})₃, -OSi(R^{aa})₃, -C(=S)N(R^{bb})₂, -C(=O)SR^{aa}, -C(=S)SR^{aa}, -SC(=S)SR^{aa}, -SC(=O)SR^{**a**a}, -OC(=O)SR^{aa}, -SC(=O)OR^{aa}, -SC(=O)R^{aa}, -P(=O)₂R^{aa}, -OP(=O)₂R^{aa}, -P(=O)(R^{aa})₂, -OP(=O)(R^{aa})₂, -OP(=O)(OR^{cc})₂, -P(=O)₂N(R^{bb})₂, -OP(=O)₂N(R^{bb})₂, -P(=O)(NR^{bb})₂, -OP(=O)(NR^{bb})₂, -NR^{bb}P(=O)(OR^{cc})₂, -NR^{bb}P(=O)(NR^{bb})₂, -P(R^{cc})₂, -P(R^{cc})₃, -OP(R^{cc})₂, -OP(R^{cc})₃, -B(R^{aa})₂, -B(OR^{cc})₂, -BR^{aa}(OR^{cc}), alkyl, haloalkyl, alkenyl, alkynyl, cycloalkyl, heterocyclyl, aryl, and heteroaryl, wherein each of alkyl, alkenyl, alkynyl, cycloalkyl, heterocyclyl, aryl, and heteroaryl is independently substituted with 0, 1, 2, 3, 4, or 5 R^{dd} groups;
or wherein two geminal hydrogens at a carbon atom are substituted by a group, such as =O, =S, =NN(R^{bb})₂, =NNR^{bb}C(=O)R^{aa}, =NNR^{bb}C(=O)OR^{aa}, =NNR^{bb}S(=O)₂R^{aa}, =NR^{bb} or =NOR^{cc};
wherein each of R^{aa} is independently selected from alkyl, haloalkyl, alkenyl, alkynyl, cycloalkyl, heterocyclyl, aryl and heteroaryl, or two R^{aa} groups are connected each other to form a heterocyclyl ring or heteroaryl ring, wherein each alkyl, alkenyl, alkynyl, cycloalkyl, heterocyclyl, aryl and heteroaryl is independently substituted with 0, 1, 2, 3, 4 or 5 R^{dd} groups;

Each of R^{bb} is independently selected from: hydrogen, -OH, -OR^{aa}, -N(R^{cc})₂, -CN, -C(=O)R^{aa}, -C(=O)N(R^{cc})₂, -CO₂R^{aa}, -SO₂R^{aa}, -C(=NR^{cc})OR^{aa}, -C(=NR^{cc})N(R^{cc})₂, -SO₂N(R^{cc})₂, -SO₂R^{cc}, -SO₂OR^{cc}, -SOR^{aa}, -C(=S)N(R^{cc})₂, -C(=O)SR^{cc}, -C(=S)SR^{cc}, -P(=O)₂R^{aa}, -P(=O)(R^{aa})₂, -P(=O)₂N(R^{cc})₂, -P(=O)(NR^{cc})₂, alkyl, haloalkyl, alkenyl, alkynyl, cycloalkyl, heterocyclyl, aryl and heteroaryl, or two R^{bb} groups are connected each other to form a heterocyclyl ring or heteroaryl ring, wherein each alkyl, alkenyl, alkynyl, cycloalkyl, heterocyclyl, aryl and heteroaryl group is independently substituted with 0, 1, 2, 3, 4 or 5 R^{dd} groups;
wherein each of R^{cc} is independently selected from hydrogen, alkyl, haloalkyl, alkenyl, alkynyl, cycloalkyl, heterocyclyl, aryl and heteroaryl, or two Rcc groups are connected to form a heterocyclyl ring or heteroaryl ring, wherein each alkyl, alkenyl, alkynyl, cycloalkyl, heterocyclyl, aryl and heteroaryl is independently substituted with 0, 1, 2, 3, 4 or 5 R^{dd} groups;

Each of R^{dd} is independently selected from: halogen, -CN, -NO₂, -N₃, -SO₂H, -SO₃H, -OH, -OR^{ee}, -ON(R^{ff})₂, -N(R^{ff})₂, -N(R^{ff})₃⁺X⁻, -N(OR^{ee})R^{ff}, -SH, -SR^{ee}, -SSR^{ee}, -C(=O)R^{ee}, -CO₂H, -CO₂R^{ee}, -OC(=O)R^{ee}, -OCO₂R^{ee}, -C(=O)N(R^{ff})₂, -OC(=O)N(R^{ff})₂, -NR^{ff}C(=O)R^{ee}, -NR^{ff}CO₂R^{ee}, -NR^{ff}C(=O)N(R^{ff})₂, -C(=NR^{ff})OR^{ee}, -OC(=NR^{ff})R^{ee}, -OC(=NR^{ff})OR^{ee}, -C(=NR^{ff})N(R^{ff})_{2,} -OC(=NR^{ff})N(R^{ff})₂, -NR^{ff}C(=NR^{ff})N(R^{ff})_{2,} -NR^{ff}SO₂R^{ee}, -SO₂N(R^{ff})₂, -SO₂R^{ee}, -SO₂OR^{ee}, -OSO₂R^{ee}, -S(=O)R^{ee}, -Si(R^{ee})₃, -OSi(R^{ee})₃, -C(=S)N(R^{ff})₂, -C(=O)SR^{ee}, -C(=S)SR^{ee}, -SC(=S)SR^{ee}, -P(=O)₂R^{ee}, -P(=O)(R^{ee})₂, -OP(=O)(R^{ee})₂, -OP(=O)(OR^{ee})₂, alkyl, haloalkyl, alkenyl, alkynyl, cycloalkyl, heterocyclyl, aryl, and heteroaryl, wherein each of alkyl, alkenyl, alkynyl, cycloalkyl, heterocyclyl, aryl, and heteroaryl is independently substituted with 0, 1, 2, 3, 4, or 5 R^{gg} groups, or two geminal R^{dd} substituents may be combined to form =O or =S;

Each of R^{ee} is independently selected from: alkyl, haloalkyl, alkenyl, alkynyl, cycloalkyl, aryl, heterocyclyl and heteroaryl, wherein each alkyl, alkenyl, alkynyl, cycloalkyl, heterocyclyl, aryl and heteroaryl is independently substituted with 0, 1, 2, 3, 4 or 5 R^{gg} groups;
wherein each of R^{ff} is independently selected from hydrogen, alkyl, haloalkyl, alkenyl, alkynyl, cycloalkyl, heterocyclyl, aryl and heteroaryl, or two Rff groups are connected each other to form a heterocyclyl ring or heteroaryl ring, wherein each alkyl, alkenyl, alkynyl, cycloalkyl, heterocyclyl, aryl and heteroaryl is independently substituted with 0, 1, 2, 3, 4 or 5 R^{gg} groups;

Each of R^{gg} is independently selected from: halogen, -CN, -NO₂, -N₃, -SO₂H, -SO₃H, -OH, -OC₁₋₆Alkyl, -ON(C₁₋₆Alkyl)₂, -N(C₁₋₆Alkyl)₂, -N(C₁₋₆Alkyl)₃⁺X⁻, -NH(C₁₋₆Alkyl)₂⁺X, -NH₂(C₁₋₆Alkyl)⁺X⁻, -NH₃⁺X⁻, -N(OC₁₋₆Alkyl)(C₁₋₆Alkyl), -N(OH)(C₁₋₆Alkyl), -NH(OH), -SH, -SC₁₋₆Alkyl, -SS(C₁₋₆Alkyl), -C(=O)(C₁₋₆Alkyl), -CO₂H, -CO₂(C₁₋₆Alkyl), -OC(=O)(C₁₋₆Alkyl), -OCO₂(C₁₋₆Alkyl), -C(=O)NH₂, -C(=O)N(C₁₋₆Alkyl)₂, -OC(=O)NH(C₁₋₆Alkyl), -NHC(=O)(C₁₋₆Alkyl), -N(C₁₋₆Alkyl)C(=O)(C₁₋₆Alkyl), -NHCO₂(C₁₋₆Alkyl), -NHC(=O)N(C₁₋₆Alkyl)₂, -NHC(=O)NH(C₁₋₆Alkyl), -NHC(=O)NH₂, -C(=NH)O(C₁₋₆Alkyl), -OC(=NH)(C₁₋₆Alkyl), -OC(=NH)OC₁₋₆Alkyl, -C(=NH)N(C₁₋₆Alkyl)₂, -C(=NH)NH(C₁₋₆Alkyl), -C(=NH)NH₂, -OC(=NH)N(C₁₋₆Alkyl)₂, -OC(NH)NH(C₁₋₆Alkyl), -OC(NH)NH₂, -NHC(NH)N(C₁₋₆Alkyl)₂, -NHC(=NH)NH₂, -NHSO₂(C₁₋₆Alkyl), -SO₂N(C₁₋₆Alkyl)₂, -SO₂NH(C₁₋₆Alkyl), -SO₂NH₂, -SO₂C₁₋₆Alkyl, -SO₂OC₁₋₆Alkyl, -OSO₂C₁₋₆Alkyl, -SOC₁₋₆Alkyl, -Si(C₁₋₆Alkyl)₃, -OSi(C₁₋₆Alkyl)₃, -C(=S)N(C₁₋₆Alkyl)₂, C(=S)NH(C₁₋₆Alkyl), C(=S)NH₂, -C(=O)S(C₁₋₆Alkyl), -C(=S)SC₁₋₆Alkyl, -SC(=S)SC₁₋₆Alkyl, -P(=O)₂(C₁₋₆Alkyl), -P(=O)(C₁₋₆Alkyl)₂, -OP(=O)(C₁₋₆Alkyl)₂, -OP(=O)(OC₁₋₆Alkyl)₂, C₁₋₆Alkyl, C₁₋₆Haloalkyl, C₂-C₆Alkenyl, C₂-C₆Alkynyl, C₃-C₇Cycloalkyl, C₆-C₁₀Aryl, C₃-C₇Heterocyclyl, and C5-C₁₀Heteroaryl; or two geminal R^{gg} substituents may be connected to form =O or =S; wherein, X⁻ is a counterion.

Exemplary substituents on a nitrogen atom include, but are not limited to: hydrogen, -OH, -OR^{aa}, -N(R^{cc})₂, -CN, -C(=O)R^{aa}, -C(=O)N(R^{cc})₂, -CO₂R^{aa}, -SO₂R^{aa}, -C(=NR^{bb})R^{aa}, -C(=NR^{cc})OR^{aa}, -C(=NR^{cc})N(R^{cc})₂, -SO₂N(R^{cc})₂, -SO₂R^{cc}, -SO₂OR^{cc}, -SOR^{aa}, -C(=S)N(R^{cc})₂, -C(=O)SR^{cc}, -C(=S)SR^{cc}, -P(=O)₂R^{aa}, -P(=O)(R^{aa})₂, -P(=O)₂N(R^{cc})₂, -P(=O)(NR^{cc})₂, alkyl, haloalkyl, alkenyl, alkynyl, cycloalkyl, heterocyclyl, aryl and heteroaryl, or two R^{cc} groups connecting to said nitrogen atom are connected to form a heterocyclyl ring or heteroaryl ring, wherein each alkyl, alkenyl, alkynyl, cycloalkyl, heterocyclyl, aryl and heteroaryl is independently substituted with 0, 1, 2, 3, 4 or 5 R^{dd} groups, and wherein R^{aa}, R^{bb}, R^{cc} and R^{dd} are as described above.

### Some additional definitions

The term "siRNA" herein is a class of double-stranded RNA molecules which can mediate the silencing of target RNA (e.g., mRNA, e.g., transcript of a gene encoding a protein) complementary thereto. A siRNA is generally double-stranded, including an antisense strand complementary to the target RNA thereof and a sense strand complementary to this antisense strand. For the sake of convenience, such an mRNA is also referred to herein as mRNA to be silenced, and such gene is also called target gene. Usually, the RNA to be silenced herein is an endogenous gene or a pathogen gene. In addition, RNAs other than mRNA (e.g. tRNA) as well as viral RNA may also be targeted.

The term "antisense strand" herein refers to a strand of a siRNA, wherein said strand contains a region that is completely, sufficiently or substantially complementary to the target sequence thereof. The term "sense strand" herein refers to a strand of a siRNA, wherein said strand contains a region that completely, sufficiently or substantially complementary to a region of an antisense strand as defined herein.

The term "complementary region" herein refers to a region on an antisense strand that is completely, sufficiently or substantially complementary to the target mRNA sequence thereof. In cases where a complementary region is incompletely complementary to the target sequence thereof, a mismatch may exist in an internal or terminal region of the molecule. Typically, the most tolerant mismatch is in the terminal region, e.g., within 5, 4, 3, 2 or 1 nucleotide at the 5' and/or 3' end. The region with the most sensitive to mismatch in the antisense strand is called "seed region". For example, in a siRNA containing a strand of 19 nt, the 19^{th} site (counting from the 5' end to the 3' end) can tolerate some mismatches.

The term "complementary" refers to the ability of a first polynucleotide to hybridize with a second polynucleotide under certain conditions, such as stringent conditions. For example, stringent conditions may include 400 mM NaCl, 40 mM PIPES pH 6.4, 1 mM EDTA, and 50°C or 70°C for 12-16 hours. With respect to fulfilling the above required capabilities related to the hybridization ability thereof, said "complementary" sequences may also include or be entirely composed of non-Watson-Crick base pairs and/or base pairs formed from non-natural as well as modified nucleotides. Such non-Watson-Crick base pairs include, but are not limited to, G:U wobble base pairing or Hoogsteen base pairing.

A polynucleotide that is "at least partially complementary", "sufficiently complementary" or "substantially complementary" to a messenger RNA (mRNA) refers to a polynucleotide that is substantially complementary to a continuous portion of the mRNA of interest. For example, in case that a polynucleotide is at least partially complementary to an mRNA encoding PCSK9, the sequence thereof is substantially complementary to an uninterrupted portion of said PCSK9 mRNA. The terms "complementary," "completely complementary," "sufficiently complementary" and "substantially complementary" as used herein may be applied to base pairing between the sense strand and antisense strand of a siRNA, or between the antisense strand of a siRNA reagent and the target sequence thereof.

"Sufficiently complementary" refers to the extent to which the sense strand only needs to be complementary to the antisense strand to maintain the overall double-stranded character of the molecule. In other words, although perfect complementarity is generally desired, in some cases, particularly in the antisense strand, one or more, e.g., 6, 5, 4, 3, 2, or 1 mismatch (relative to the target mRNA) may be included, but the sense and antisense strands can still maintain the overall double-stranded character of the molecule.

The term "shRNA" herein refers to short hairpin RNA. An shRNA comprises two short inverted repeat sequences. An shRNA cloned into an shRNA expression vector comprises two short inverted repeat sequences, separated by a loop sequence, forming a hairpin structure and controlled by the RNA polymerase III (pol III) promoter. Subsequently, 5 to 6 Ts are ligated as transcription terminators of pol III.

"Nucleoside" is a compound comprising two substances: one is a purine base or a pyrimidine base, and the other is a ribose or a deoxyribose; "nucleotide" is a compound comprising three substances: one is a purine base or a pyrimidine base, another is a ribose or deoxyribose, and the third is a phosphoric acid; and "oligonucleotide" refers to, for example, a nucleic acid molecule (RNA or DNA) with a length of less than 100, 200, 300 or 400 nucleotides, as either a single chain or a double chain.

The term "base" is a fundamental building block of nucleosides, nucleotides and nucleic acids; as always containing nitrogen, said base is also referred to as "nitrogenous base." Unless otherwise specified, the capital letters herein, i.e., A, U, T, G and C, denote the bases of nucleotides, which is adenine, uracil, thymine, guanine and cytosine, respectively.

As used herein, the "modification" of nucleotides includes, but is not limited to: methoxyl substitution (methoxy-modified), fluorine substitution (fluoro-modified), connection with a phosphorothioate group, or protection with a conventional protecting group. For example, a fluoro-modified nucleotide refers to a nucleotide formed by substituting the hydroxyl at the 2' position of the ribosyl of the nucleotide with a fluorine atom, while a methoxy-modified nucleotide refers to a nucleotide formed by substituting the 2'-hydroxyl of the ribosyl with a methoxyl.

"Modified nucleotides" herein include, but are not limited to: a 2'-O-methyl modified nucleotide, a 2'-fluoro modified nucleotide, a 2'-deoxy-modified nucleotide, an inosine ribonucleotide, an abasic nucleotide, an inverted abasic deoxyribonucleotide, a nucleotide comprising a phosphorothioate group, a nucleotide modified by vinylphosphonate, a locked nucleotide, 2'-amino-modified nucleotide, 2'-alkyl-modified nucleotide, a morpholino nucleotide, a phosphoramidate, a non-natural base comprising nucleotide, and a terminal nucleotide linked to a cholesteryl derivative or a dodecanoic acid bisdecylamide group, deoxyribonucleotide, or a nucleotide with protection of a conventional protecting group. For example, a 2'-fluoro modified nucleotide refers to a nucleotide formed by substituting the hydroxyl at the 2' position of the ribosyl in a nucleotide with a fluorine atom. Said 2'-deoxy-modified nucleotide refers to a nucleotide formed by substituting the 2'-hydroxyl of the ribosyl with a methoxyl.

The term "reactive phosphorus group" refers to a phosphorus-containing group included within a nucleotide unit or a nucleotide analogue unit, wherein the group can undergo a nucleophilic attack to react with a hydroxyl or amine group in another molecule, especially another nucleotide unit or nucleotide analogue unit. Typically, such a reaction generates an ester-type internucleoside bond connecting a said first nucleotide unit or a said first nucleotide analogue unit with a said second nucleotide unit or a said second nucleotide analogue unit. A reactive phosphorus group can be selected from phosphoramidite, H-phosphonate, alkyl-phosphonate, phosphate or phosphate mimics, including but not limited to: natural phosphate, phosphorothioate, phosphorodithioate, borano phosphate, borano thiophosphate, phosphonate, halogen substituted phosphonates and phosphates, phosphoramidates, phosphodiester, phosphotriester, thiophosphodiester, thiophosphotriester, diphosphates and triphosphates, preferably P(OCH_{2C}H_{2C}N)(N(iPr)₂).

"Protecting group" refers to any atom or group of atoms added to a molecule to prevent undesired chemical reactions of existing groups within the molecule. A "protecting group" may be an unstable chemical moiety known in the art, which is used to protect reactive groups such as hydroxyl, amino and thiol groups to prevent undesired or premature reactions during chemical synthesis. Protecting groups are typically used selectively and/or orthogonally to protect sites during the reactions of other reactive sites, which can then be removed to leave the unprotected groups intact or available for further reactions.

A non-limiting list of protecting groups include benzyl; substituted benzyl; alkylcarbonyls and alkoxycarbonyls (e.g., t-butoxycarbonyl (BOC), acetyl, or isobutyryl); arylalkylcarbonyls and arylalkoxycarbonyls (e.g., benzyloxycarbonyl); substituted methyl ether (e.g. methoxymethyl ether); substituted ethyl ether; a substituted benzyl ether; tetrahydropyranyl ether; silyls (e.g., trimethylsilyl, triethylsilyl, triisopropylsilyl, t-butyldimethylsilyl, tri-iso-propylsilyloxymethyl, [2-(trimethylsilyl)ethoxy]methyl or t-butyldiphenylsilyl); esters (e.g. benzoate ester); carbonates (e.g. methoxymethylcarbonate); sulfonates (e.g. tosylate or mesylate); acyclic ketal (e.g. dimethyl acetal); cyclic ketals (e.g., 1,3-dioxane, 1,3-dioxolanes, and those described herein); acyclic acetal; cyclic acetal (e.g., those described herein); acyclic hemiacetal; cyclic hemiacetal; cyclic dithioketals (e.g., 1,3-dithiane or 1,3-dithiolane); orthoesters (e.g., those described herein) and triarylmethyl groups (e.g., trityl; monomethoxytrityl (MMTr); 4,4'-dimethoxytrityl (DMTr); 4,4',4"-trimethoxytrityl (TMTr); and those described herein). Preferred protecting groups are selected from acetyl (Ac), benzoyl (Bzl), benzyl (Bn), isobutyryl (iBu), phenylacetyl, benzyloxymethyl acetal (BOM), beta-methoxyethoxymethyl ether (MEM), methoxymethylether (MOM), p-methoxybenzyl ether (PMB), methylthiomethyl ether, pivaloyl (Piv), tetrahydropyranyl (THP), triphenylmethyl (Trt), methoxytrityl [(4-methoxyohenyl)diphenylmethyl-] (MMT), dimethoxytrityl, [bis-(4-methoxyphenyl)phenylmethyl (DMT), trimethylsilyl ether (TMS), tert-butyldimethylsilyl ether (TBDMS), tri-iso-propylsilyloxymethyl ether (TOM), tri-isopropylsilyl ether (TIPS), methyl ethers, ethoxyethyl ethers (EE) N,N-dimethylformamidine and 2-cynaonethyl (CE).

"Hydroxy-protecting group" refers to a group that can prevent a hydroxyl from undergoing chemical reactions and can be removed under specific conditions to restore the hydroxyl. The main hydroxy-protecting groups include silane-type, acyl-type or ether-type protecting groups, preferably the following:
trimethylsilyl (TMS), triethylsilyl (TES), dimethylisopropylsilyl (DMIPS), diethylisopropylsilyl (DEIPS), tert-butyldimethylsilyl (TBDMS), tert-butyldiphenylsilyl (TBDPS), triisopropylsilyl (TIPS), acetyl (Ac), chloroacetyl, dichloroacetyl, trichloroacetyl, trifluoroacetyl (TFA), benzoyl, p-methoxybenzoyl, 9-fluorenylmethoxycarbonyl (Fmoc), allyloxycarbonyl (Alloc), 2,2,2-trichloroethoxycarbonyl (Troc), benzyloxycarbonyl (Cbz), tert-butoxycarbonyl (Boc), benzyl (Bn), p-methoxybenzyl (PMB), allyl, triphenylmethyl (Tr), di-p-methoxytrityl (DMTr), methoxymethyl (MOM), benzyloxymethyl (BOM), 2,2,2-trichloroethoxymethyl, 2-methoxyethoxymethyl (MEM), methylthiomethyl (MTM), p-methoxybenzyloxymethyl (PMBM), -C(O)CH₂CH₂C(O)OH or 4,4'-dimethoxytrityl, preferably -C(O)CH₂CH₂C(O)OH or 4,4'-dimethoxytrityl, more preferably -C(O)CH₂CH₂C(O)OH.

As used herein, the term "pharmaceutically acceptable salt" represents carboxylates or amino acid salts of a compound of the present invention, which are suitable for contact with patient tissues within a scope of sound medical judgment without causing excessive toxicity, irritation, allergic reactions, etc., and are effective in terms of the intended use with a reasonable benefit/risk ratio; said salt includes, where applicable, the zwitterionic form of a compound of the present invention.

The present invention includes tautomers, which are functional-group isomers resulting from the rapid migration of an atom in a molecule between two positions. A compound with different tautomeric forms, said herein, refers to all the tautomers and does not be restricted to any specific tautomeric form.

A compound of the present invention may include one or more asymmetric centers, and thus may exist in various stereoisomeric forms, such as enantiomers and/or diastereomers. For example, a compound of the present invention may be one of the forms of enantiomer, diastereoisomer or geometric isomer (e.g. a cis isomer or a trans isomer), or may be a mixture of any type of stereoisomerism, including a racemic mixture and a mixture enriched with one or more forms of stereoisomer. An isomer herein may be achieved by separating from a mixture via any method known to those skilled in the art, wherein the method includes chiral high-pressure liquid chromatography (HPLC) and the formation and crystallization of chiral salts; alternatively, a preferred isomer may be prepared through asymmetric synthesis.

The present invention also includes isotopically labeled compounds (isotopic variants) which are equivalent to those described by formula (I), except that one or more atoms are replaced with atoms with an atomic mass or mass number different from that common in nature. Examples of isotopes which may be incorporated into a compound of the present invention include isotopes of hydrogen, carbon, nitrogen, oxygen, phosphorus, sulfur, fluorine and chlorine, such as ²H, ³_{H}, 13C, ¹¹C, ¹⁴C, ¹⁵N, ¹⁸O, ¹⁷O, ³¹P, ³²P, ³⁵S, ¹⁸F and ³⁶Cl. Any compounds based on a compound of the present invention and containing any aforementioned isotope and/or any isotope of other atoms, the prodrugs thereof and the pharmaceutically acceptable salts of said compounds or said prodrugs all fall in the scope of the present invention. Certain isotopically labeled compounds of the present invention, such as a compound into which any radioisotope (e.g. ³H and ¹⁴C) is introduced, may be used for distribution determinations of a drug and/or the substrate tissue thereof. Tritium, i.e. ³H and carbon-14, i.e. ¹⁴C isotopes are particularly preferred, because they can be easily prepared and detected. Furthermore, substitution with an isotope heavier, such as deuterium, i.e. ²H, may in some cases be preferred because resultant increased metabolic stability may provide therapeutic benefits such as prolonged in vivo half-life or reduced dosage. An isotopically labeled compound of formula (I) of the present invention and the prodrug thereof may generally be prepared with any readily available isotopically labeled reagent instead of any non-isotopically labeled reagent, in a procedure described below and/or in a process disclosed in any of the Examples and Preparations.

### Compounds of the Present Invention

The present invention relates to an oligonucleotide, or a pharmaceutically acceptable salt, tautomer or stereoisomer thereof, wherein the oligonucleotide comprises at its 5' end a structure of formula (I): 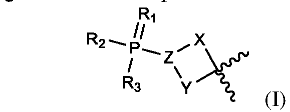 wherein each group is as defined in the context.

The present invention also relates to an oligonucleotide, or a pharmaceutically acceptable salt, tautomer or stereoisomer thereof, wherein the oligonucleotide comprises at its 5' end a structure of formula (II): 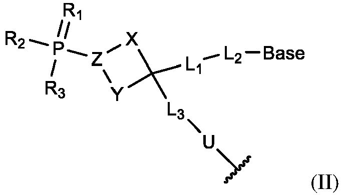 wherein each group is as defined in the context.

The present invention also relates to an oligonucleotide, or a pharmaceutically acceptable salt, tautomer or stereoisomer thereof, wherein the oligonucleotide comprises at its 5' end a nucleotide monomer of formula (I), (IV), or (V): 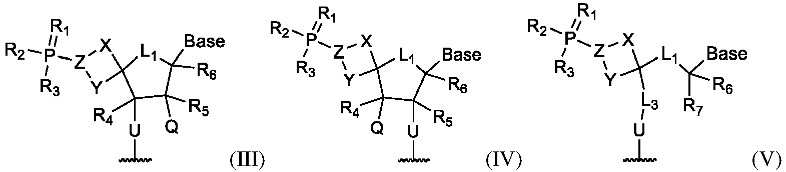 wherein each group is as defined in the context.

The present invention further relates to a compound of formula (VI), (VII), or (VIII), or a pharmaceutically acceptable salt, tautomer or stereoisomer thereof: 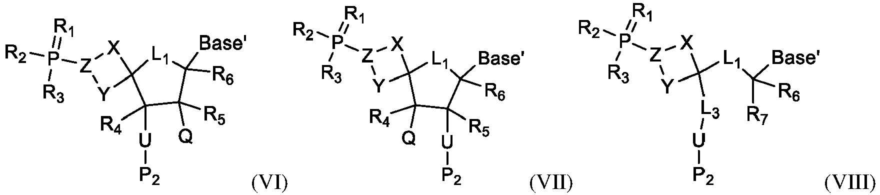 wherein each group is as defined in the context.

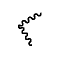

In one embodiment, 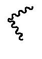 represents attachment to the remainder of the oligonucleotide; In another embodiment, 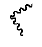 represents attachment to a modified or unmodified nucleoside.

### X. Y and Z

In one embodiment, X is CRₐR_{b}, preferably CH₂; In another embodiment, X is (CRₐR_{b})₂.

In one embodiment, Y is CRₐR_{b}, preferably CH₂; In another embodiment, Y is (CRₐR_{b})₂.

In one embodiment, Z is CRₐ, preferably CH.
wherein X and Y are optionally substituted with 1 or 2 deuterium atoms, and Z is optionally substituted with 1 deuterium atom.

### L₁. L₂ and L₃

In one embodiment, L₁ is a bond; In another embodiment, L₁ is O; In another embodiment, L₁ is S; In another embodiment, L₁ is C₁₋₄ alkylene.

In one embodiment, L₂ is C₁₋₄ alkylene, preferably C₁₋₂ alkylene.

In one embodiment, L₃ is C₁₋₄ alkylene, preferably C₁₋₂ alkylene.

In one embodiment, L₂ and L₃ are connected together and form C₅₋₆ cycloalkyl with L₁ and its adjacent carbon atom; In another embodiment, L₂ and L₃ are connected together and form 5-membered to 6-membered heterocyclyl with L₁ and its adjacent carbon atom, preferably U, L₁, L₂, and L₃ and their adjacent carbon atom form a ribose or deoxyribose, which is optionally substituted with R₅.

In one embodiment, L₁ is unsubstituted; In another embodiment, L₁ is further substituted with one R#; In another embodiment, L₁ is further substituted with 2 independently selected R#; In another embodiment, L₁ is further substituted with 3 independently selected R#; In another embodiment, L₁ is further substituted with 4 independently selected R#; In another embodiment, L₁ is further substituted with 5 independently selected R#.

In one embodiment, L₂ is unsubstituted; In another embodiment, L₂ is further substituted with one R#; In another embodiment, L₂ is further substituted with 2 independently selected R#; In another embodiment, L₂ is further substituted with 3 independently selected R#; In another embodiment, L₂ is further substituted with 4 independently selected R#; In another embodiment, L₂ is further substituted with 5 independently selected R#.

In one embodiment, L₃ is unsubstituted; In another embodiment, L₃ is further substituted with one R#; In another embodiment, L₃ is further substituted with 2 independently selected R#; In another embodiment, L₃ is further substituted with 3 independently selected R#; In another embodiment, L₃ is further substituted with 4 independently selected R#; In another embodiment, L₃ is further substituted with 5 independently selected R#.
wherein each of the aforementioned L₁, L₂ and L₃ is optionally substituted by 1, 2, 3, 4, 5, or more deuterium atoms or fully deuterated.

### U

In one embodiment, U is O; In another embodiment, U is S; In another embodiment, U is CRₐR_{b}, such as CH₂; In another embodiment, U is NR_{c}, such as NH.
wherein the aforementioned U is optionally substituted with one or more deuterium atoms or fully deuterated.

### Q

In one embodiment, Q is H; In another embodiment, Q is D; In another embodiment, Q is halogen; In another embodiment, Q is OH; In another embodiment, Q is C₁₋₆ alkyl, such as C₁₋₄ alkyl; In another embodiment, Q is C₁₋₆ haloalkyl, such as C₁₋₄ haloalkyl; In another embodiment, Q is C₁₋₆ alkoxyl, such as C₁₋₄ alkoxyl; In another embodiment, Q is C₁₋₆ haloalkoxyl, such as C₁₋₄ haloalkoxyl; In another embodiment, Q is C₂₋₆ alkenyl, such as C₂₋₄ alkenyl; In another embodiment, Q is C₂₋₆ alkynyl, such as C₂₋₄ alkynyl.
wherein the aforementioned Q is optionally substituted by 1, 2, 3, 4, 5, or more deuterium atoms or fully deuterated.

### R₁

In one embodiment, R₁ is O; In another embodiment, R₁ is S.

### R₂ and R₃

In one embodiment, R₂ is OH; In another embodiment, R₂ is SH; In another embodiment, R₂ is NH₂; In another embodiment, R₂ is C₁₋₆ alkyl; In another embodiment, R₂ is C₁₋₆ haloalkyl; In another embodiment, R₂ is C₁₋₆ alkoxyl; In another embodiment, R₂ is C₁₋₆ haloalkoxyl.

In one embodiment, R₂ is OR_{d}; In another embodiment, R₂ is OP₁; In another embodiment, R₂ is SR_{d}; In another embodiment, R₂ is SP₁; In another embodiment, R₂ is NRₑR_{f}.

In one embodiment, R₂ is unsubstituted; In another embodiment, R₂ is further substituted with one R#; In another embodiment, R₂ is further substituted with 2 independently selected R#; In another embodiment, R₂ is further substituted with 3 independently selected R#; In another embodiment, R₂ is further substituted with 4 independently selected R#; In another embodiment, R₂ is further substituted with 5 independently selected R#.

In one embodiment, R₃ is OH; In another embodiment, R₃ is SH; In another embodiment, R₃ is NH₂; In another embodiment, R₃ is C₁₋₆ alkyl; In another embodiment, R₃ is C₁₋₆ haloalkyl; In another embodiment, R₃ is C₁₋₆ alkoxyl; In another embodiment, R3 is C₁₋₆ haloalkoxyl.

In one embodiment, R₃ is OR_{d}; In another embodiment, R₃ is OP₁; In another embodiment, R₃ is SR_{d}; In another embodiment, R₃ is SP₁; In another embodiment, R₃ is NRₑR_{f}.

In one embodiment, R₃ is unsubstituted; In another embodiment, R₃ is further substituted with one R#; In another embodiment, R₃ is further substituted with 2 independently selected R#; In another embodiment, R₃ is further substituted with 3 independently selected R#; In another embodiment, R₃ is further substituted with 4 independently selected R#; In another embodiment, R₃ is further substituted with 5 independently selected R#.
wherein each of the aforementioned R₂ and R₃ is optionally substituted by 1, 2, 3, 4, 5, or more deuterium atoms or fully deuterated.

### R₄. R₅, R₆ and R₇

In one embodiment, R₄ is H; In another embodiment, R₄ is D; In another embodiment, R₄ is halogen; In another embodiment, R₄ is OH; In another embodiment, R₄ is C₁₋₆ alkyl, such as C₁₋₄ alkyl; In another embodiment, R₄ is C₁₋₆ haloalkyl, such as C₁₋₄ haloalkyl; In another embodiment, R₄ is C₁₋₆ alkoxyl, such as C₁₋₄ alkoxyl; In another embodiment, R₄ is C₁₋₆ haloalkoxyl, such as C₁₋₄ haloalkoxyl.

In one embodiment, R₅ is H; In another embodiment, R₅ is D; In another embodiment, R₅ is halogen; In another embodiment, R₅ is OH; In another embodiment, R₅ is C₁₋₆ alkyl, such as C₁₋₄ alkyl; In another embodiment, R₅ is C₁₋₆ haloalkyl, such as C₁₋₄ haloalkyl; In another embodiment, R₅ is C₁₋₆ alkoxyl, such as C₁₋₄ alkoxyl; In another embodiment, R₅ is C₁₋₆ haloalkoxyl, such as C₁₋₄ haloalkoxyl.

In one embodiment, R₆ is H; In another embodiment, R₆ is D; In another embodiment, R₆ is halogen; In another embodiment, R₆ is OH; In another embodiment, R₆ is C₁₋₆ alkyl, such as C₁₋₄ alkyl; In another embodiment, R₆ is C₁₋₆ haloalkyl, such as C₁₋₄ haloalkyl; In another embodiment, R₆ is C₁₋₆ alkoxyl, such as C₁₋₄ alkoxyl; In another embodiment, R₆ is C₁₋₆ haloalkoxyl, such as C₁₋₄ haloalkoxyl.

In one embodiment, R₇ is H; In another embodiment, R₇ is D; In another embodiment, R₇ is halogen; In another embodiment, R₇ is OH; In another embodiment, R₇ is C₁₋₆ alkyl, such as C₁₋₄ alkyl; In another embodiment, R₇ is C₁₋₆ haloalkyl, such as C₁₋₄ haloalkyl; In another embodiment, R₇ is C₁₋₆ alkoxyl, such as C₁₋₄ alkoxyl; In another embodiment, R₇ is C₁₋₆ haloalkoxyl, such as C₁₋₄ haloalkoxyl.
wherein each of the aforementioned R₄, R5, R₆ and R₇ is optionally substituted by 1, 2, 3, 4, 5, or more deuterium atoms or fully deuterated.

### Rₐ. R_{b} and R_{c}

In one embodiment, Rₐ is H; In another embodiment, Rₐ is D; In another embodiment, Rₐ is halogen; In another embodiment, Rₐ is OH; In another embodiment, Rₐ is NH₂; In another embodiment, Rₐ is CN; In another embodiment, Rₐ is C₁₋₆ alkyl, such as C₁₋₄ alkyl; In another embodiment, Rₐ is C₁₋₆ haloalkyl, such as C₁₋₄ haloalkyl; In another embodiment, Rₐ is C₁₋₆ alkoxyl, such as C₁₋₄ alkoxyl; In another embodiment, Rₐ is C₁₋₆ haloalkoxyl, such as C₁₋₄ haloalkoxyl; In another embodiment, Rₐ is C₂₋₆ alkenyl, such as C₂₋₄ alkenyl; In another embodiment, Rₐ is C₂₋₆ alkynyl, such as C₂₋₄ alkynyl.

In one embodiment, Rₐ is unsubstituted; In another embodiment, Rₐ is further substituted with one R#; In another embodiment, Rₐ is further substituted with 2 independently selected R#; In another embodiment, Rₐ is further substituted with 3 independently selected R#; In another embodiment, Rₐ is further substituted with 4 independently selected R#; In another embodiment, Rₐ is further substituted with 5 independently selected R#.

In one embodiment, R_{b} is H; In another embodiment, R_{b} is D; In another embodiment, R_{b} is halogen; In another embodiment, R_{b} is OH; In another embodiment, R_{b} is NH₂; In another embodiment, R_{b} is CN; In another embodiment, R_{b} is C₁₋₆ alkyl, such as C₁₋₄ alkyl; In another embodiment, R_{b} is C₁₋₆ haloalkyl, such as C₁₋₄ haloalkyl; In another embodiment, R_{b} is C₁₋₆ alkoxyl, such as C₁₋₄ alkoxyl; In another embodiment, R_{b} is C₁₋₆ haloalkoxyl, such as C₁₋₄ haloalkoxyl; In another embodiment, R_{b} is C₂₋₆ alkenyl, such as C₂₋₄ alkenyl; In another embodiment, R_{b} is C₂₋₆ alkynyl, such as C₂₋₄ alkynyl.

In one embodiment, R_{b} is unsubstituted; In another embodiment, R_{b} is further substituted with one R#; In another embodiment, R_{b} is further substituted with 2 independently selected R#; In another embodiment, R_{b} is further substituted with 3 independently selected R#; In another embodiment, R_{b} is further substituted with 4 independently selected R#; In another embodiment, R_{b} is further substituted with 5 independently selected R#.

In one embodiment, R_{c} is H; In another embodiment, R_{c} is C₁₋₆ alkyl; In another embodiment, R_{c} is C₁₋₆ haloalkyl; In another embodiment, R_{c} is C₂₋₆ alkenyl; In another embodiment, R_{c} is C₂₋₆ alkynyl.
wherein each of the aforementioned Rₐ, R_{b} and R_{c} is optionally substituted by 1, 2, 3, 4, 5, or more deuterium atoms or fully deuterated.

### R_{d}. Rₑ and R_{f}

In one embodiment, R_{d} is H; In another embodiment, R_{d} is C₁₋₆ alkyl, such as C₁₋₄ alkyl; In another embodiment, R_{d} is C₁₋₆ haloalkyl, such as C₁₋₄ haloalkyl; In another embodiment, R_{d} is C₂₋₆ alkenyl; In another embodiment, R_{d} is C₂₋₆ alkynyl.

In one embodiment, R_{d} is unsubstituted; In another embodiment, R_{d} is optionally substituted with one or more D or fully deuterated; In another embodiment, R_{d} is substituted with halogen; In another embodiment, R_{d} is substituted with C₁₋₆ alkyl; In another embodiment, R_{d} is substituted with C₁₋₆ haloalkyl.

In one embodiment, Rₑ is H; In another embodiment, Rₑ is C₁₋₆ alkyl, such as C₁₋₄ alkyl; In another embodiment, Rₑ is C₁₋₆ haloalkyl, such as C₁₋₄ haloalkyl.

In one embodiment, Rₑ is unsubstituted; In another embodiment, Rₑ is optionally substituted with one or more D or fully deuterated; In another embodiment, Rₑ is substituted with halogen; In another embodiment, Rₑ is substituted with C₁₋₆ alkyl; In another embodiment, Rₑ is substituted with C₁₋₆ haloalkyl.

In one embodiment, R_{f} is H; In another embodiment, R_{f} is C₁₋₆ alkyl, such as C₁₋₄ alkyl; In another embodiment, R_{f} is C₁₋₆ haloalkyl, such as C₁₋₄ haloalkyl.

In one embodiment, R_{f} is unsubstituted; In another embodiment, R_{f} is optionally substituted with one or more D or fully deuterated; In another embodiment, R_{f} is substituted with halogen; In another embodiment, R_{f} is substituted with C₁₋₆ alkyl; In another embodiment, R_{f} is substituted with C₁₋₆ haloalkyl.
wherein each of the aforementioned R_{d}, Rₑ and R_{f} is optionally substituted by 1, 2, 3, 4, 5, or more deuterium atoms or fully deuterated.

### P₁ and P₂

In one embodiment, P₁ is a protecting group; In another embodiment, P₁ is a hydroxy-protecting group.

In one embodiment, P₂ is a reactive phosphorus group, such as -P(OCH₂CH₂CN)(N(iPr)₂).

### Base and Base'

In one embodiment, Base is H; In another embodiment, Base is a modified or unmodified base or leaving group; In another embodiment, Base is selected from modified or unmodified A, U, T, G, and C, such as

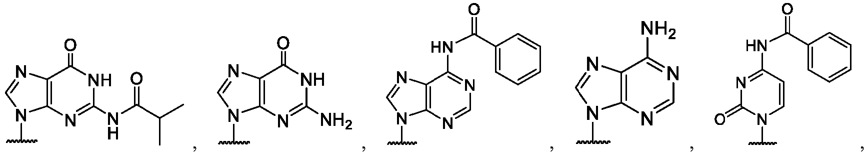

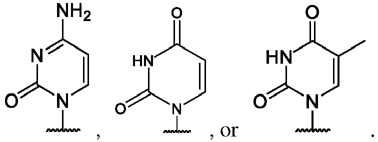

In one embodiment, Base' is H; In another embodiment, Base' is a modified or unmodified base or leaving group; In another embodiment, Base' is selected from modified or unmodified A, U, T, G, and C, such as

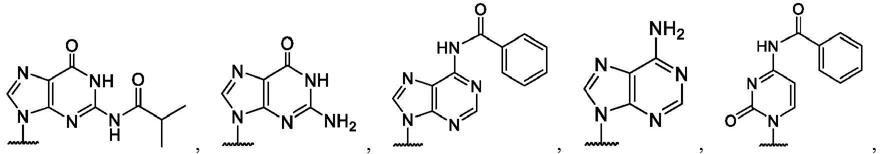

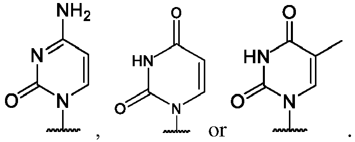

### R#

In one embodiment, R# is H; In another embodiment, R# is D; In another embodiment, R# is halogen; In another embodiment, R# is C₁₋₆ alkyl; In another embodiment, R# is C₁₋₆ haloalkyl; In another embodiment, R# is C₂₋₆ alkenyl; In another embodiment, R# is C₂₋₆ alkynyl;
wherein the aforementioned R# is optionally substituted by 1, 2, 3, 4, 5, or more deuterium atoms or fully deuterated.

### GalNAc

In one embodiment, GalNAc is a conjugation group of formula (X): 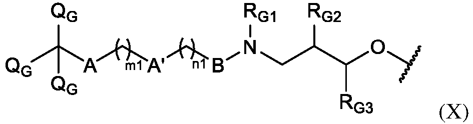 Wherein,
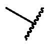 represents the position of attachment to biomolecules;
Q_{G} is independently H, 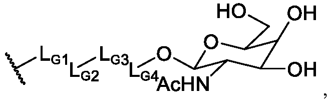 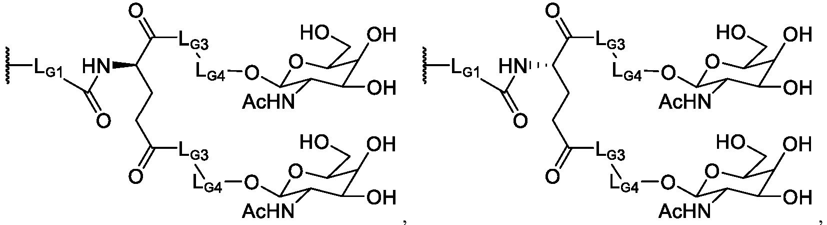 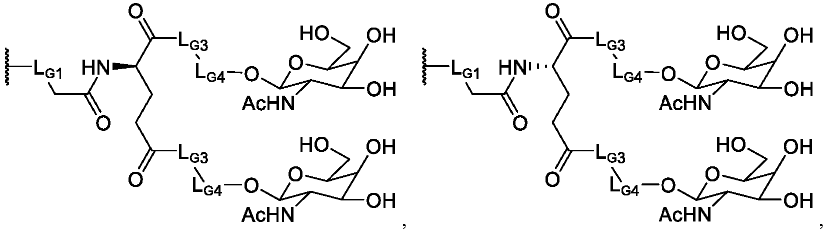 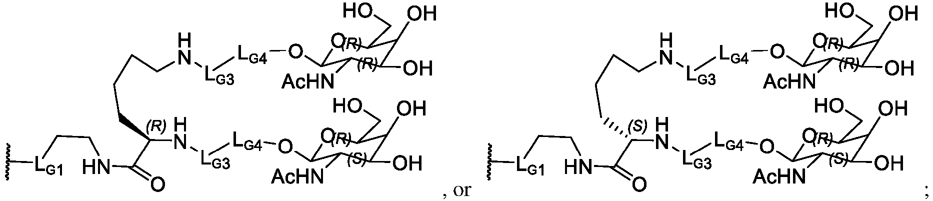
wherein L_{G1} is a bond, -CH₂-, -CH₂CH₂-, -C(O)-, -CH₂O-, -CH₂O-CH₂CH₂O-, or -NHC(O)-(CH₂NHC(O))ₐ-;
L_{G2} is a bond or -CH₂CH₂C(O)-;
L_{G3} is a bond, -(NHCH₂CH₂)_{b}-, -(NHCH₂CH₂CH₂)_{b}-, or -C(O)CH₂-;
L_{G4} is -(OCH₂CH₂)_{c}-, -(OCH₂CH₂CH₂)_{c}-, -(OCH₂CH₂CH₂CH₂)_{c}-, -(OCH₂CH₂CH₂CH₂CH₂)_{c}-, or -NHC(O)-(CH₂)_{d}-;
wherein a = 0, 1, 2, or 3;
b = 1, 2, 3, 4 or 5;
c = 1, 2, 3, 4 or 5;
d = 1, 2, 3, 4, 5, 6, 7, or 8;
A is a bond, -CH₂O-, or -NHC(O)-;
A' is a bond, -C(O)NH-, -NHC(O)-, or -O(CH₂CH₂O)ₑ-;
wherein e is 1, 2, 3, 4, or 5;
B is a bond, -CH₂-, -C(O)-, -M-, -CH₂-M-, or -C(O)-M-;
wherein M is 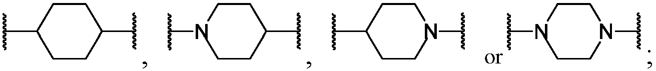
R_{G1} and R_{G2} together form -CH₂CH₂O- or -CH₂CH(RG)-O-, and R_{G3} is H;
or R_{G1} and R_{G3} together form -C₁₋₂ alkylene-, and R_{G2} is H;
wherein R_{G} is -OR_{G}', -CH₂OR_{G}', or -CH₂CH₂OR_{G}'; wherein R_{G}' is H, a hydroxy-protecting group, or a solid support, and the hydroxy-protecting group is preferably -C(O)CH₂CH₂C(O)OH or 4,4'-dimethoxytrityl;
m1 = 0, 1, 2, 3, 4, 5, 6, 7, 8, 9 or 10;
n1 = 0, 1, 2, 3, 4, 5, 6, 7, 8, 9 or 10.

In another embodiment, GalNAc is a conjugation group of formula (I'): 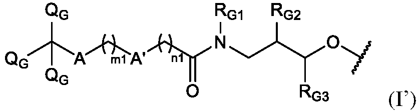 wherein, 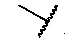
represents the position of attachment to biomolecules;
Q_{G} is independently H, 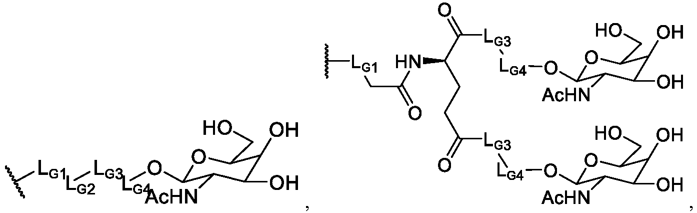 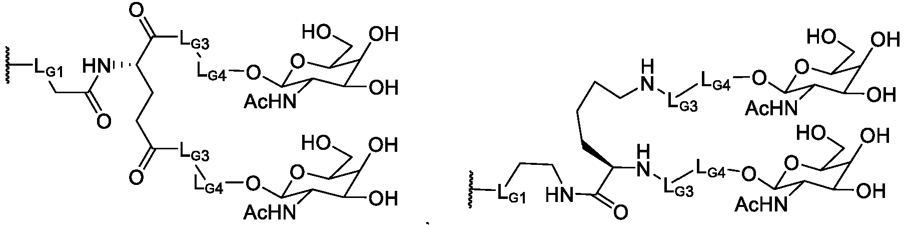 or 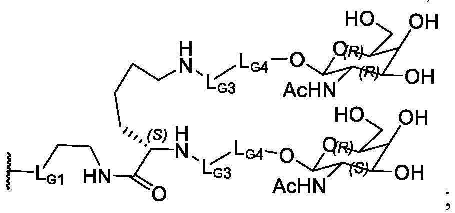
wherein L_{G1} is a bond, -CH₂-, -CH₂CH₂-, -C(O)-, -CH₂O-, -CH₂O-CH₂CH₂O-, or -NHC(O)-(CH₂NHC(O))ₐ-;
L_{G2} is a bond or -CH₂CH₂C(O)-;
L_{G3} is a bond, -(NHCH₂CH₂)_{b}-, -(NHCH₂CH₂CH₂)_{b}-, or -C(O)CH₂-;
L_{G4} is -(OCH₂CH₂)_{c}-, -(OCH₂CH₂CH₂)_{c}-, -(OCH₂CH₂CH₂CH₂)_{c}-, -(OCH₂CH₂CH₂CH₂CH₂)_{c}-, or -NHC(O)-(CH₂)_{d}-;
wherein a = 0, 1, 2, or 3;
b = 1, 2, 3, 4 or 5;
c = 1, 2, 3, 4 or 5;
d = 1, 2, 3, 4, 5, 6, 7, or 8;
A is -CH₂O- or -NHC(O)-;
A' is a bond, -C(O)NH- or -NHC(O)-;
R_{G1} and R_{G2} together form -CH₂CH₂O- or -CH₂CH(RG)-O-, and R_{G3} is H;
or R_{G1} and R_{G3} together form -C₁₋₂ alkylene-, and R_{G2} is H;
wherein R_{G} is -OR_{G}', -CH₂OR_{G}', or -CH₂CH₂OR_{G}'; wherein R_{G}' is H, a hydroxy-protecting group, or a solid support, and the hydroxy-protecting group is preferably -C(O)CH₂CH₂C(O)OH or 4,4'-dimethoxytrityl;
m1 = 0, 1, 2, 3, 4, 5, 6, 7, 8, 9 or 10;
n1 = 0, 1, 2, 3, 4, 5, 6, 7, 8, 9 or 10.

In another embodiment, GalNAc is a conjugation group of formula (X), wherein
Q_{G} is independently H, 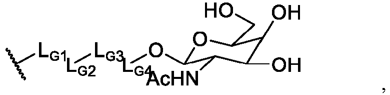 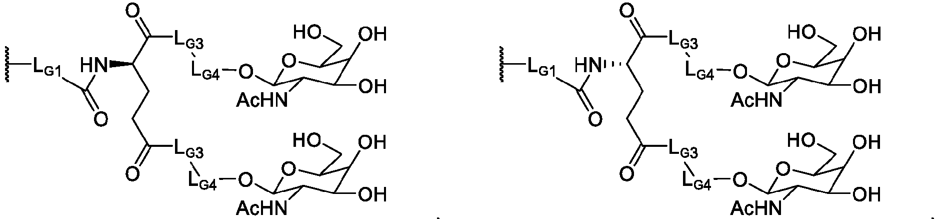 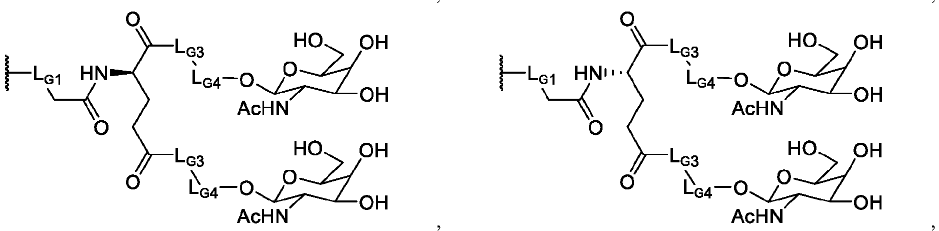 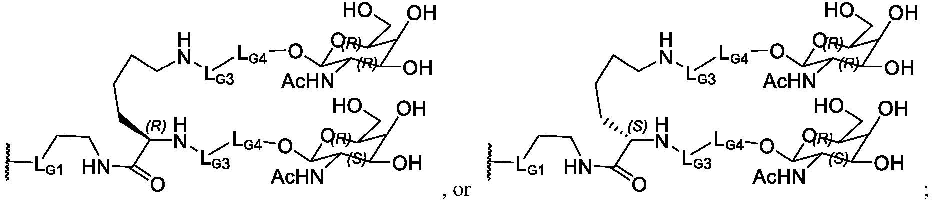
wherein L_{G1} is a bond, -CH₂-, -CH₂CH₂-, -C(O)-, -CH₂O-, -CH₂O-CH₂CH₂O-, or -NHC(O)-(CH₂NHC(O))ₐ-;
L_{G2} is a bond or -CH₂CH₂C(O)-;
L_{G3} is a bond, -(NHCH₂CH₂)_{b}-, -(NHCH₂CH₂CH₂)_{b}-, or -C(O)CH₂-;
L_{G4} is -(OCH₂CH₂)_{c}-, -(OCH₂CH₂CH₂)_{c}-, -(OCH₂CH₂CH₂CH₂)_{c}-, -(OCH₂CH₂CH₂CH₂CH₂)_{c}-, or -NHC(O)-(CH₂)_{d}-;
wherein a = 0, 1, 2, or 3;
b = 1, 2, 3, 4 or 5;
c = 1, 2, 3, 4 or 5;
d = 1, 2, 3, 4, 5, 6, 7, or 8;
A is a bond, -CH₂O-, or -NHC(O)-;
A' is a bond, -C(O)NH-, -NHC(O)-, or -O(CH₂CH₂O)ₑ-;
wherein e is 1, 2, 3, 4, or 5;
B is a bond, -CH₂-, -M-, -CH₂-M-, or -C(O)-M-;
wherein M is 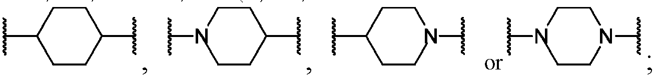
R_{G1} and R_{G2} together form -CH₂CH₂O- or -CH₂CH(RG)-O-, and R_{G3} is H;
or R_{G1} and R_{G3} together form -C₁₋₂ alkylene-, and R_{G2} is H;
wherein R_{G} is -OR_{G}', -CH₂OR_{G}', or -CH₂CH₂OR_{G}'; wherein R_{G}' is H, a hydroxy-protecting group, or a solid support, and the hydroxy-protecting group is preferably -C(O)CH₂CH₂C(O)OH or 4,4'-dimethoxytrityl;
m1 = 0, 1, 2, 3, 4, 5, 6, 7, 8, 9 or 10;
n1 = 0, 1, 2, 3, 4, 5, 6, 7, 8, 9 or 10.

In another embodiment, GalNAc is a conjugation group of formula (X), wherein:
Q_{G} is independently H, 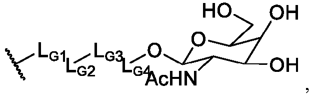 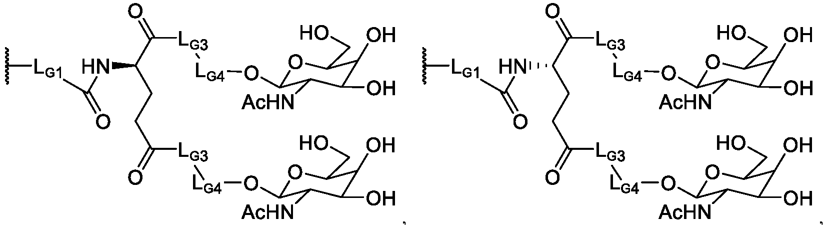 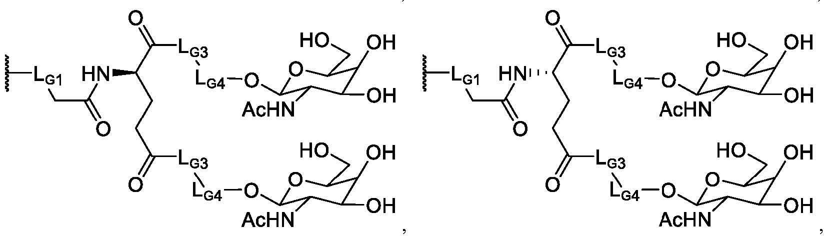 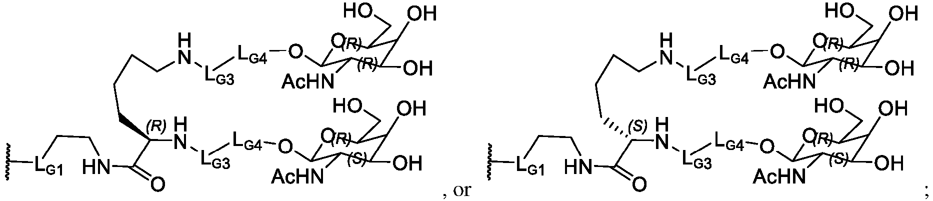
wherein L_{G1} is a bond, -CH₂-, -CH₂CH₂-, -C(O)-, -CH₂O-, -CH₂O-CH₂CH₂O-, or -NHC(O)-(CH₂NHC(O))ₐ-;
L_{G2} is a bond or -CH₂CH₂C(O)-;
L_{G3} is a bond, -(NHCH₂CH₂)_{b}-, -(NHCH₂CH₂CH₂)_{b}-, or -C(O)CH₂-;
L_{G4} is -(OCH₂CH₂)_{c}-, -(OCH₂CH₂CH₂)_{c}-, -(OCH₂CH₂CH₂CH₂)_{c}-, -(OCH₂CH₂CH₂CH₂CH₂)_{c}-, or -NHC(O)-(CH₂)_{d}-;
wherein a = 0, 1, 2, or 3;
b = 1, 2, 3, 4 or 5;
c = 1, 2, 3, 4 or 5;
d = 1, 2, 3, 4, 5, 6, 7, or 8;
A is a bond, -CH₂O-, or -NHC(O)-;
A' is -O(CH₂CH₂O)ₑ-;
wherein e is 1, 2, 3, 4, or 5;
B is a bond, -CH₂-, -C(O)-, -M-, -CH₂-M-, or -C(O)-M-;
wherein M is 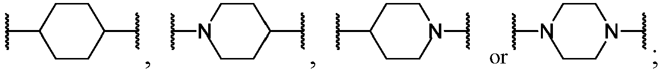
R_{G1} and R_{G2} together form -CH₂CH₂O- or -CH₂CH(RG)-O-, and R_{G3} is H;
or R_{G1} and R_{G3} together form -C₁₋₂ alkylene-, and R_{G2} is H;
wherein R_{G} is -OR_{G}', -CH₂OR_{G}', or -CH₂CH₂OR_{G}'; wherein R_{G}' is H, a hydroxy-protecting group, or a solid support, and the hydroxy-protecting group is preferably -C(O)CH₂CH₂C(O)OH or 4,4'-dimethoxytrityl;
m1 = 0, 1, 2, 3, 4, 5, 6, 7, 8, 9 or 10;
n1 = 0, 1, 2, 3, 4, 5, 6, 7, 8, 9 or 10.

Any technical solution or any combination thereof in any of the above specific embodiments can be combined with any technical solution or any combination thereof in other specific embodiments. For example, any technical solution or any combination thereof of X can be combined with any technical solution or any combination thereof of Y, Z, L₁, L₂, L₃, R₁, R₂, R₃, R₄, R₅, R₆, R₇, U, Q, P₁, P₂, Base, and Base'. The present invention is intended to include all combinations of these technical solutions, which are not listed one by one due to space limitations.

The present invention also provides a vector comprising a nucleotide sequence encoding the siRNA of the invention. The vector of the present invention can amplify or express a nucleotide encoding the siRNA of the invention linked thereto.

For example, a siRNA targeting the PCSK9 gene can be expressed from a transcription unit inserted into a DNA or RNA vector. Expression can be transient (within hours to weeks) or sustained (weeks to months or longer), depending on the particular construct used and the target tissue or cell type. A nucleotide encoding the siRNA can be introduced into a linear construct, a circular plasmid, or a viral vector. A nucleotide encoding the siRNA can be stably expressed by integration into the cell genome, or can be stably inherited and expressed extrachromosomally. Generally speaking, a vector expressing the siRNA is usually a DNA plasmid or a viral vector.

Viral vector systems comprising a sequence encoding the siRNA include, but are not limited to: (a) adenoviral vectors; (b) retroviral vectors; (c) adeno-associated viral vectors; (d) herpes simplex virus vectors; (e) SV40 vectors; (f) polyomavirus vectors; (g) papillomavirus vectors; (h) picornavirus vectors; (i) poxvirus vectors; and (j) helper virus-dependent or gutless adenoviral vectors.

The present invention also provides a cell comprising the siRNA or vector of the invention, wherein the siRNA or vector of the invention can be transcribed in the cell.

In another aspect, the present invention relates to an oligonucleotide, or a pharmaceutically acceptable salt, tautomer or stereoisomer thereof, wherein the oligonucleotide comprises at its 5' end a structure of the following formula: 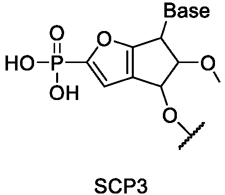 wherein 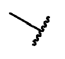 represents attachment to the remainder of the oligonucleotide; Base is as defined in the context.

The present invention specifically relates to the following technical solutions:
1. An oligonucleotide, or a pharmaceutically acceptable salt, tautomer or stereoisomer thereof, wherein the oligonucleotide comprises at its 5' end a structure of formula (I): 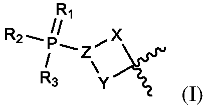 wherein, 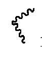
   represents attachment to the remainder of the oligonucleotide, preferably to a modified or unmodified nucleoside;
   X and Y are each independently selected from CRₐR_{b} or (CRₐR_{b})₂, preferably CRₐR_{b};
   Z is CRₐ;
   Rₐ and R_{b} are independently selected from H, D, halogen, OH, NH₂, CN, C₁₋₆ alkyl, C₁₋₆ haloalkyl, C₁₋₆ alkoxyl, C₁₋₆ haloalkoxyl, C₂₋₆ alkenyl, or C₂₋₆ alkynyl; wherein the Rₐ and R_{b} are optionally further substituted with 1, 2, 3, 4, or 5 independently selected R#;
   R₁ is selected from O or S;
   R₂ and R₃ are independently selected from OH, SH, NH₂, C₁₋₆ alkyl, C₁₋₆ haloalkyl, C₁₋₆ alkoxyl, or C₁₋₆ haloalkoxyl; wherein the R₂ and R₃ are optionally further substituted with 1, 2, 3, 4, or 5 independently selected R#;
   R# is selected from H, D, halogen, C₁₋₆ alkyl, C₁₋₆ haloalkyl, C₂₋₆ alkenyl, or C₂₋₆ alkynyl;
   wherein each of the aforementioned groups is optionally substituted by 1, 2, 3, 4, 5, or more deuterium atoms or fully deuterated.
2. An oligonucleotide according to technical solution 1, or a pharmaceutically acceptable salt, tautomer, or stereoisomer thereof, wherein: 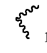
   represents attachment to the remainder of the oligonucleotide, preferably to a modified or unmodified nucleoside;
   X and Y are each independently selected from CRₐR_{b} or (CRₐR_{b})₂, preferably CRₐR_{b};
   Z is CRₐ;
   Rₐ and R_{b} are independently selected from H, D, halogen, OH, NH₂, CN, C₁₋₆ alkyl, C₁₋₆ haloalkyl, C₁₋₆ alkoxyl, or C₁₋₆ haloalkoxyl;
   R₁ is selected from O or S;
   R₂ and R₃ are independently selected from OH, SH, C₁₋₆ alkoxyl, or C₁₋₆ haloalkoxyl; wherein the R₂ and R₃ are optionally further substituted with 1, 2, or 3 independently selected R#;
   R# is selected from H, D, halogen, C₁₋₆ alkyl, and C₁₋₆ haloalkyl.
3. An oligonucleotide according to technical solution 1 or 2, or a pharmaceutically acceptable salt, tautomer, or stereoisomer thereof, wherein: 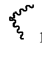
   represents attachment to the remainder of the oligonucleotide, preferably to a modified or unmodified nucleoside;
   X and Y are each independently selected from CRₐR_{b} or (CRₐR_{b})₂, preferably CRₐR_{b};
   Z is CRₐ;
   Rₐ and R_{b} are independently selected from H, D, halogen, C₁₋₄ alkyl, or C₁₋₄ haloalkyl;
   R₁ is selected from O or S;
   R₂ and R₃ are independently selected from OH or SH, preferably OH.
4. An oligonucleotide according to any one of technical solutions 1 to 3, or a pharmaceutically acceptable salt, tautomer, or stereoisomer thereof, wherein 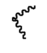 represents attachment to a group at the 5'-end of the oligonucleotide, preferably to a modified or unmodified nucleoside;
   X and Y are CH₂;
   Z is CH;
   R₁ is O;
   R₂ and R₃ are OH.
5. An oligonucleotide according to technical solution 1, or a pharmaceutically acceptable salt, tautomer, or stereoisomer thereof, which has a structure of formula (II) : 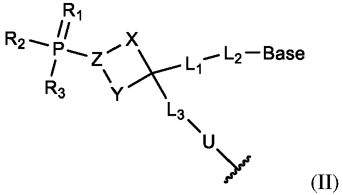 wherein,
   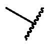 represents attachment to the remainder of the oligonucleotide;
   X and Y are CRₐR_{b};
   Z is CRₐ;
   L₁ is selected from a bond, O, S, or C₁₋₄ alkylene;
   L₂ and L₃ are independently selected from C₁₋₄ alkylene;
   L₁, L₂, and L₃ are optionally independently substituted with 1, 2, 3, 4, or 5 independently selected R#;
   U is selected from O, S, CRₐR_{b}, or NR_{c};
   Rₐ and R_{b} are independently selected from H, D, halogen, OH, NH₂, CN, C₁₋₆ alkyl, C₁₋₆ haloalkyl, C₁₋₆ alkoxyl, C₁₋₆ haloalkoxyl, C₂₋₆ alkenyl or C₂₋₆ alkynyl;
   R_{c} is independently selected from H, C₁₋₆ alkyl, C₁₋₆ haloalkyl, C₂₋₆ alkenyl, or C₂₋₆ alkynyl;
   Rₐ, R_{b}, and R_{c} are optionally further substituted with 1, 2, 3, 4, or 5 independently selected R#;
   alternatively, L₂ and L₃ are connected together and form a C₅₋₆ cycloalkyl or 5-membered to 6-membered heterocyclyl with Li and its adjacent carbon atom, preferably U, L₁, L₂, and L₃ and their adjacent carbon atom form a ribose or deoxyribose, which is optionally substituted with R₅;
   R₁ is selected from O or S;
   R₂ and R₃ are independently selected from OH, SH, NH₂, C₁₋₆ alkyl, C₁₋₆ haloalkyl, C₁₋₆ alkoxyl, or C₁₋₆ haloalkoxyl; wherein R₂ and R₃ are optionally substituted with 1, 2, 3, 4, or 5 independently selected R#;
   R₅ is selected from H, D, halogen, C₁₋₆ alkoxyl, or C₁₋₆ haloalkoxyl;
   R# is selected from H, D, halogen, C₁₋₆ alkyl, C₁₋₆ haloalkyl, C₂₋₆ alkenyl, or C₂₋₆ alkynyl;
   Base is selected from H, or a modified or unmodified base;
   wherein each of the aforementioned groups is optionally substituted by 1, 2, 3, 4, 5, or more deuterium atoms or fully deuterated.
**6.** An oligonucleotide according to technical solution 5, or a pharmaceutically acceptable salt, tautomer, or stereoisomer thereof, wherein: 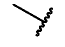
   represents attachment to the remainder of the oligonucleotide;
   X and Y are CRₐR_{b};
   Z is CRₐ;
   Rₐ and R_{b} are independently selected from H, D, halogen, OH, NH₂, CN, C₁₋₆ alkyl, C₁₋₆ haloalkyl, C₁₋₆ alkoxyl, or C₁₋₆ haloalkoxyl;
   L₁ is selected from a bond, O, or C₁₋₄ alkylene;
   L₂ and L₃ are independently selected from C₁₋₄ alkylene;
   L₁, L₂, and L₃ are optionally independently substituted with 1, 2, or 3 independently selected R#;
   U is selected from O, S, NH, or CH₂;
   alternatively, L₂ and L₃ are connected together with and form a C₅₋₆ cycloalkyl or 5-membered to 6-membered heterocyclyl with L₁ and its adjacent carbon atom, preferably U, L₁, L₂, and L₃ and their adjacent carbon atom form a ribose or deoxyribose, which is optionally substituted with R₅;
   R₁ is selected from O or S;
   R₂ and R₃ are independently selected from OH, SH, C₁₋₆ alkoxyl, or C₁₋₆ haloalkoxyl; wherein R₂ and R₃ are optionally substituted with 1, 2, or 3 independently selected R#;
   R₅ is selected from H, D, C₁₋₆ alkyl, C₁₋₆ haloalkyl, C₁₋₆ alkoxyl, or C₁₋₆ haloalkoxyl;
   R# is selected from H, D, halogen, C₁₋₆ alkyl, and C₁₋₆ haloalkyl;
   Base is selected from H, or a modified or unmodified base.
7. An oligonucleotide according to technical solution 5 or 6, or a pharmaceutically acceptable salt, tautomer, or stereoisomer thereof, wherein: 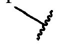
   represents attachment to the remainder of the oligonucleotide;
   X and Y are CRₐR_{b};
   Z is CRₐ;
   Rₐ and R_{b} are independently selected from H, D, halogen, C₁₋₄ alkyl, or C₁₋₄ haloalkyl;
   L₁ is selected from a bond, or C₁₋₄ alkylene;
   L₂ and L₃ are independently selected from C₁₋₄ alkylene;
   U is selected from O or S;
   R₁ is selected from O or S;
   R₂ and R₃ are independently selected from OH or SH, preferably OH;
   Base is selected from 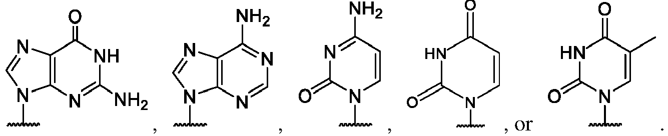
8. An oligonucleotide according to any one of technical solutions 5 to 7, or a pharmaceutically acceptable salt, tautomer, or stereoisomer thereof, wherein:
   represents attachment to the remainder of the oligonucleotide;
   X and Y are CH₂;
   Z is CH;
   L₁ is a bond;
   L₂ and L₃ are independently selected from C₁₋₂ alkylene;
   U is O;
   R₁ is O;
   R₂ and R₃ are OH;
   Base is selected from 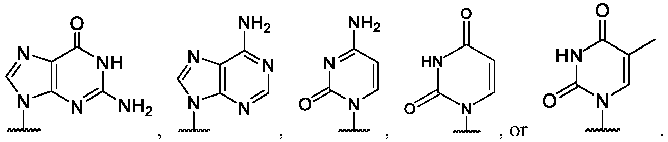
9. An oligonucleotide of technical solution 1, or a pharmaceutically acceptable salt, tautomer or stereoisomer thereof, wherein the oligonucleotide comprises at its 5' end a nucleotide monomer of formula (I), (IV), or (V): 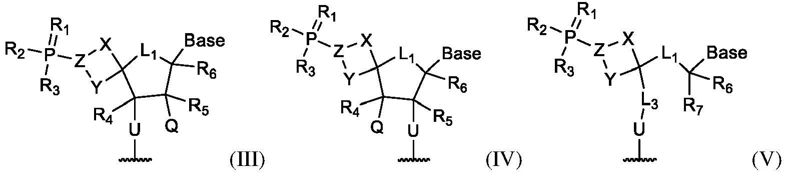
   wherein Q is selected from H, D, halogen, OH, C₁₋₆ alkyl, C₁₋₆ haloalkyl, C₁₋₆ alkoxyl, C₁₋₆ haloalkoxyl, C₂₋₆ alkenyl, or C₂₋₆ alkynyl;
   R₄. R₅, R₆ and R₇ are independently selected from H, D, halogen, OH, C₁₋₆ alkyl, C₁₋₆ haloalkyl, C₁₋₆ alkoxyl, C₁₋₆ haloalkoxyl, C₂₋₆ alkenyl or C₂₋₆ alkynyl;
   Base is selected from H, or a modified or unmodified base;
   L₁, L₃, X, Y, Z, R₁, R₂, R₃, and U are as defined in any one of technical solutions 5 to 8;
   wherein each of the aforementioned groups is optionally substituted by 1, 2, 3, 4, 5, or more deuterium atoms or fully deuterated.
10. An oligonucleotide according to technical solution 9, or a pharmaceutically acceptable salt, tautomer, or stereoisomer thereof, wherein:
   Q is selected from H, D, halogen, OH, C₁₋₆ alkyl, C₁₋₆ haloalkyl, C₁₋₆ alkoxyl, or C₁₋₆ haloalkoxyl;
   R₄. R₅, R₆ and R₇ are independently selected from H, D, halogen, C₁₋₆ alkyl, C₁₋₆ haloalkyl, C₂₋₆ alkenyl, or C₂₋₆ alkynyl;
   Base is selected from H, or a modified or unmodified base, preferably 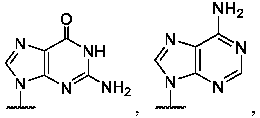 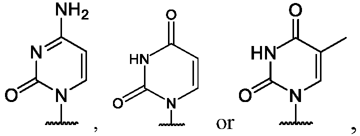
   L₁, L₃, X, Y, Z, R₁, R₂, R₃, and U are as defined in any one of technical solutions 5 to 8.
11. An oligonucleotide according to technical solution 9 or 10, or a pharmaceutically acceptable salt, tautomer, or stereoisomer thereof, wherein:
   Q is selected from H, halogen, or C₁₋₄ alkoxyl, preferably H, F, or methoxyl;
   R₄. R₅, R₆, and R₇ are independently selected from H, halogen, C₁₋₄ alkyl, or C₁₋₄ haloalkyl, preferably H;
   Base is selected from 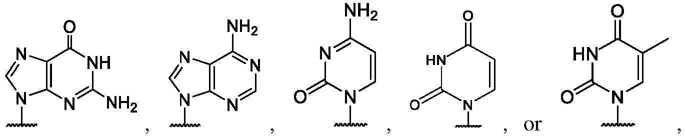 preferably 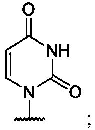
   L₁, L₃, X, Y, Z, R₁, R₂, R₃, and U are as defined in any one of technical solutions 5 to 8.
12. An oligonucleotide according to technical solutions 9 to 11, or a pharmaceutically acceptable salt, tautomer, or stereoisomer thereof, wherein the oligonucleotide comprises at its 5' end a nucleotide monomer having the following structure: 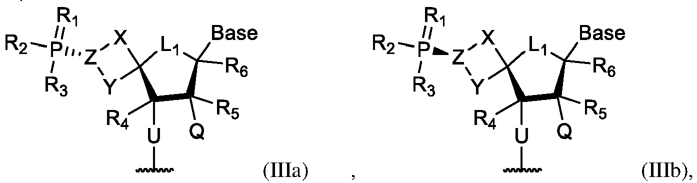 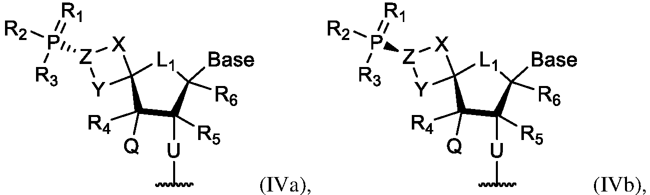 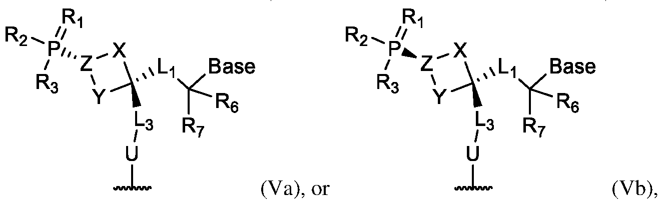 wherein, each group is as defined in any one of technical solutions 9 to 11.
13. An oligonucleotide according to technical solutions 9 to 12, or a pharmaceutically acceptable salt, tautomer, or stereoisomer thereof, wherein the oligonucleotide comprises at its 5' end a nucleotide monomer having the following structure: 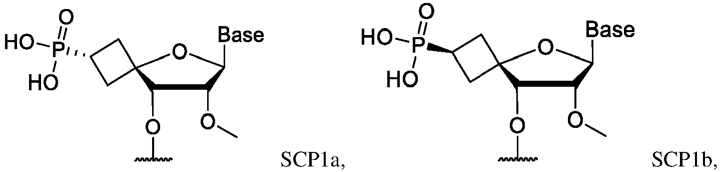 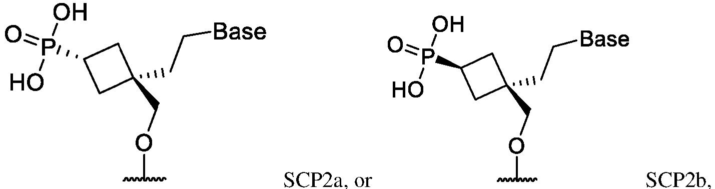 wherein, Base is selected from 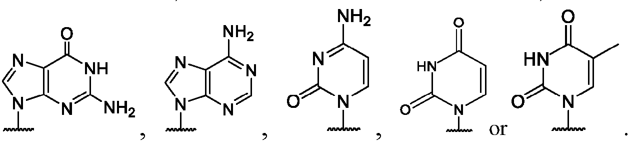
**14.** A compound of formula (VI), (VII), or (VIII), or a pharmaceutically acceptable salt, tautomer or stereoisomer thereof: 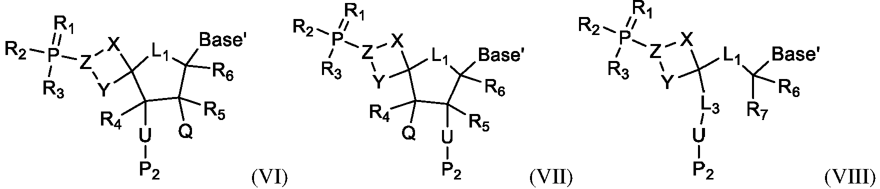 Wherein,
   R₁ is selected from O or S;
   R₂ and R₃ are independently selected from OR_{d}, OP₁, SR_{d}, SP₁, or NRₑR_{f};
   R_{d} is selected from H, C₁₋₆ alkyl, C₁₋₆ haloalkyl, C₂₋₆ alkenyl, or C₂₋₆ alkynyl, wherein R_{d} is optionally substituted with D, halogen, C₁₋₆ alkyl, or C₁₋₆ haloalkyl or fully deuterated;
   Rₑ and R_{f} are independently selected from H, C₁₋₆ alkyl, or C₁₋₆ haloalkyl, wherein Rₑ and R_{f} may be optionally substituted with D, halogen, C₁₋₆ alkyl, or C₁₋₆ haloalkyl or fully deuterated;
   P₁ is selected from a protecting group, preferably a hydroxy-protecting group;
   P₂ is selected from a reactive phosphorus group, preferably -P(OCH₂CH₂CN)(N(iPr)₂);
   Base' is independently selected from H, a modified or unmodified base or leaving group;
   L₁, L₃, X, Y, Z, U, Q, R₄, R₅, R₆, and R₇ are as defined in any one of technical solutions 5 to 11;
   wherein each of the aforementioned groups is optionally substituted by 1, 2, 3, 4, 5, or more deuterium atoms or fully deuterated.
15. A compound according to technical solution 14, or a pharmaceutically acceptable salt, tautomer, or stereoisomer thereof, wherein:
   R₁ is selected from O or S;
   R₂ and R₃ are independently selected from OR_{d}, OP₁, SR_{d}, SP₁, or NRₑR_{f};
   R_{d} is selected from H, C₁₋₄ alkyl, or C₁₋₄ haloalkyl, wherein R_{d} is optionally substituted with D or halogen or fully deuterated;
   Rₑ and R_{f} are independently selected from H, C₁₋₄ alkyl, or C₁₋₄ haloalkyl, wherein Rₑ and R_{f} may be optionally substituted with D or halogen or fully deuterated;
   P₁ is selected from a protecting group, preferably a hydroxy-protecting group;
   P₂ is selected from a reactive phosphorus group, preferably -P(OCH₂CH₂CN)(N(iPr)₂);
   Base' is selected from 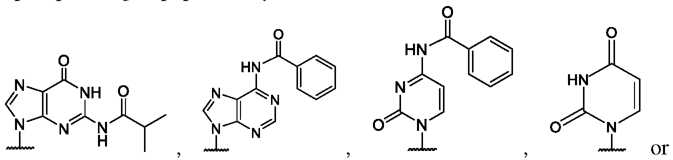 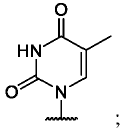
   L₁, L₃, X, Y, Z, U, Q, R₄, R₅, R₆, and R₇ are as defined in any one of technical solutions 5 to 11.
16. A compound according to technical solution 14 or 15, or a pharmaceutically acceptable salt, tautomer, or stereoisomer thereof, wherein:
   R₁ is selected from O or S, preferably O;
   R₂ and R₃ are independently selected from OR₄ or OP₁;
   R_{d} is selected from H, C₁₋₄ alkyl, or C₁₋₄ haloalkyl;
   Rₑ and R_{f} are independently selected from H, C₁₋₄ alkyl, or C₁₋₄ haloalkyl;
   P₁ is selected from a protecting group, preferably a hydroxy-protecting group;
   P₂ is selected from a reactive phosphorus group, preferably -P(OCH₂CH₂CN)(N(iPr)₂);
   Base' is selected from 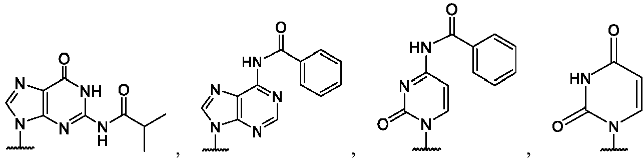 or 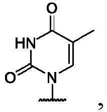 more preferably 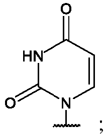
   L₁, L₃, X, Y, Z, U, Q, R₄, R₅, R₆, and R₇ are as defined in any one of technical solutions 5 to 11.
17. A compound according to any one of technical solutions 14 to 16, or a pharmaceutically acceptable salt, tautomer or stereoisomer thereof, wherein the compound is selected from: 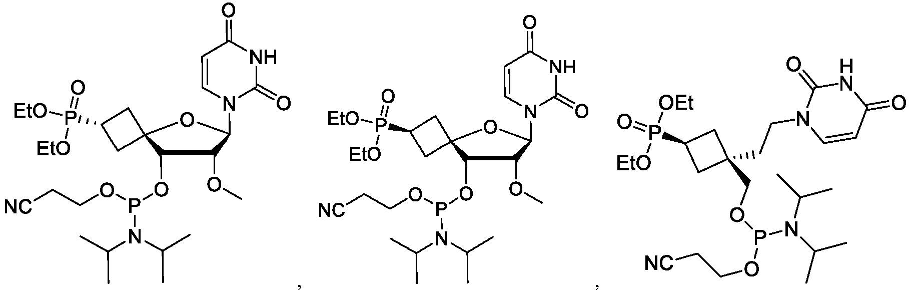 or 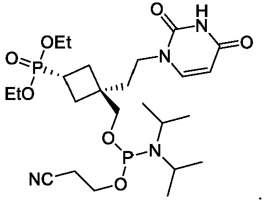
**18.** An oligonucleotide according to any one of technical solutions 1 to 13, wherein the oligonucleotide is single-stranded with 14 to 30 nucleotides.
**19.** An oligonucleotide according to any one of technical solutions 1 to 13, wherein the oligonucleotide is a double-stranded RNA comprising a sense strand and an antisense strand, each strand having 14 to 30 nucleotides; wherein the antisense strand has a sequence sufficiently complementary to the sense strand and target mRNA.
**20.** An oligonucleotide according to technical solution 19, wherein the double-stranded RNA comprises in its antisense strand a structure of formula (I), (II), (III), (IV), (V), (wyin'c), (IIIb), (IVa), (IVb), (Va), or (Vb) according to any one of technical solutions 1 to 12 or a nucleotide monomer according to technical solution 13.
**21.** An oligonucleotide according to any one of technical solutions 19 to 20, wherein the double-stranded RNA is further conjugated to a ligand; preferably, the ligand comprises one or more GalNAc.
**22.** A nucleic acid molecule comprising in its nucleotide sequence one or more nucleotide monomers according to any one of technical solutions 9 to 13.
**23.** A nucleic acid molecule according to technical solution 22, wherein the nucleic acid is selected from DNA, RNA, and a DNA/RNA hybrid.
**24.** A nucleic acid molecule according to technical solution 23, wherein the nucleic acid molecule is single- or double-stranded.
**25.** A nucleic acid molecule according to any one of technical solutions 22 to 24, wherein the nucleic acid molecule is selected from small interfering RNA (siRNA) and short hairpin RNA (shRNA).
**26.** A vector comprising a nucleotide sequence encoding a dsRNA of any one of technical solutions 19 to 21.
**27.** A cell comprising a dsRNA of any one of technical solutions 19 to 21 or a vector of technical solution 24.
**28.** A pharmaceutical composition comprising the double-stranded RNA molecule according to any one of technical solutions 19 to 21 and a pharmaceutically acceptable carrier or excipient.
**29.** A kit comprising the double-stranded RNA molecule according to any one of technical solutions 19 to 21.
**30**. A method for inhibiting the expression of a target gene in a cell, comprising a step of introducing the double-stranded RNA molecule according to any one of technical solutions 19 to 21 into the cell.

Without wishing to be bound by any particular theory, the stereoconfigurations of formulae (IIIa), (IVa), and (Va) enable the oligonucleotide (particularly the phosphonate group at its 5' end) of the present invention to better bind to the Ago2 protein, thereby improving the activity of the oligonucleotide.

### EXAMPLES

The following examples are intended to illustrate the invention and do not limit the scope of the invention.

### EXAMPLE 1 Preparation of Compounds E1-1 and E1-2

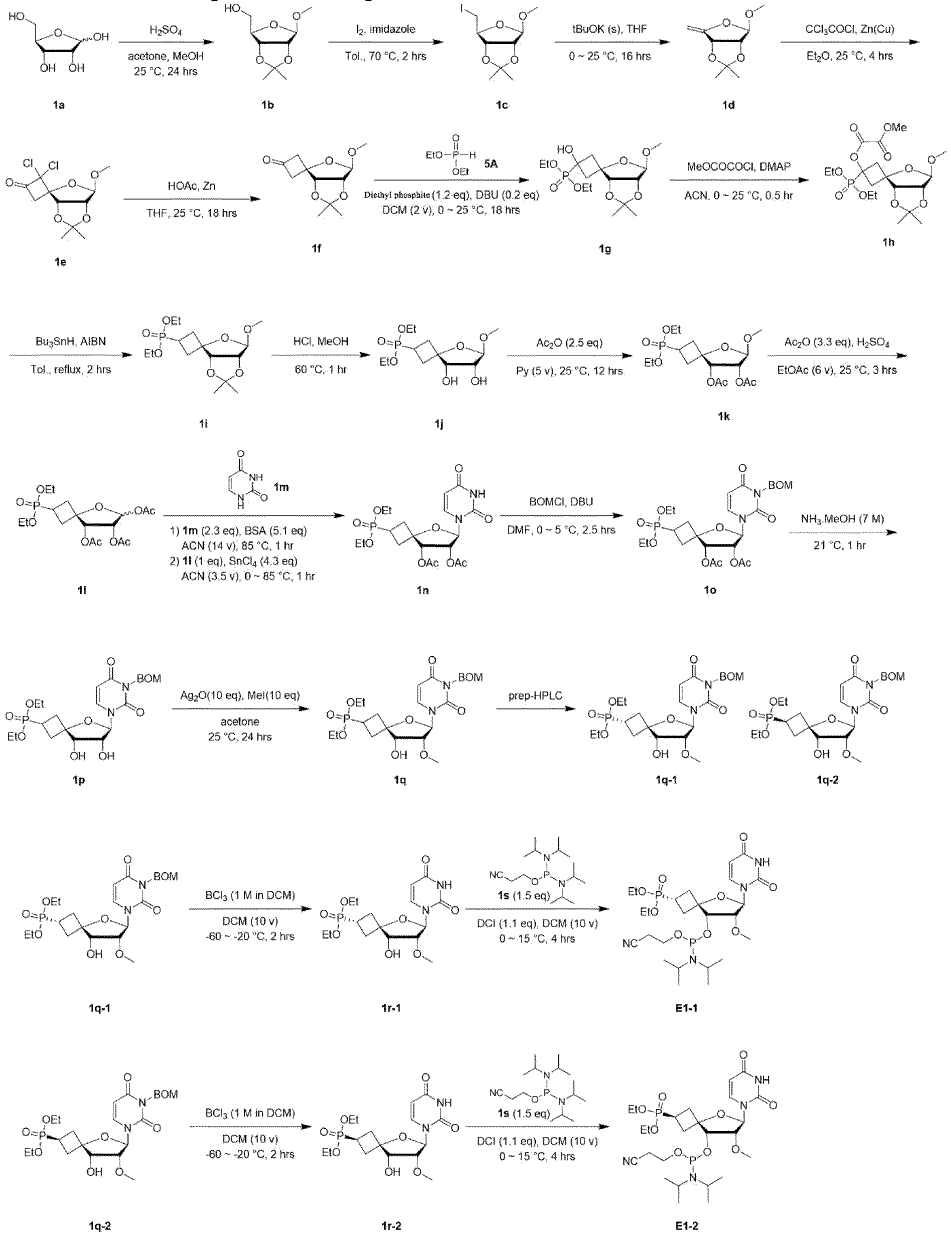

### 1. Preparation of Compound 1b

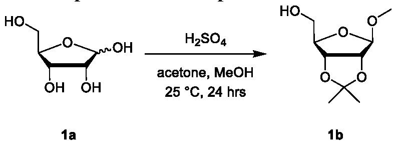

Compound **1a** (200 g, 1.33 mol, 1.00 eq) was suspended in a mixed solution of anhydrous acetone (1.00 L) and anhydrous methanol (1.00 L). Concentrated sulfuric acid (20.0 mL, 0.27 eq) was added dropwise, and the mixture was allowed to react at 25°C for 24 h. The reaction mixture was neutralized with saturated sodium bicarbonate and concentrated. The residue was dissolved in ethyl acetate and washed three times with saturated brine (500 mL). The organic phase was dried over anhydrous sodium sulfate and concentrated to obtain compound **1b** (242 g, 88.9%).

**¹H NMR:** 400 MHz CDCl₃ *δ* 4.97 (s, 1H), 4.84 (d, *J =* 6.0 Hz, 1H), 4.59 (d, *J =* 6.0 Hz, 1H), 4.44-4.43 (m, 1H), 3.72-3.59 (m, 2H), 3.44 (s, 3H), 1.49 (s, 3H), 1.32 (s, 3H).

### 2. Preparation of Compound 1c

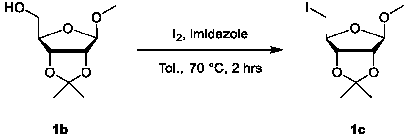

Compound **1b** (310 g, 1.52 mol, 1.00 eq), imidazole (206 g, 3.02 mol, 2.00 eq), and triphenylphosphine (476 g, 1.82 mol, 1.20 eq) were dissolved in toluene (2.1 L), and elemental iodine (446 g, 1.76 mmol, 1.16 eq) was added in portions at room temperature. The mixture was heated up to 70°C and stirred for 1.5 h until TLC showed that the reaction was complete. The reaction mixture was quenched with methanol (60 mL). After cooling, saturated aqueous sodium thiosulfate solution (2.1 L) was added. The organic phase was separated and collected, washed twice with saturated brine (1.5 L), dried over anhydrous sodium sulfate, filtered, and concentrated. The residue was pulped with methyl tert-butyl ether and filtered. The filtrate was concentrated, and the obtained crude product was purified by silica gel column chromatography (eluent: petroleum ether/ethyl acetate) to obtain compound **1c** (401 g, 1.27 mol, 91.1%).

**¹H NMR:** 400 MHz CDCl₃ *δ* 4.98 (s, 1H), 4.69 (d, J = 6.0 Hz, 1H), 4.56 (d, J = 6.0 Hz, 1H), 4.39-4.35 (m, 1H), 3.30 (s, 3H), 3.24-3.20 (m, 1H), 3.09 (t, J = 10.0 Hz, 1H) , 1.41 (s, 3H), 1.26 (s, 3H).

### 3. Preparation of Compound 1d

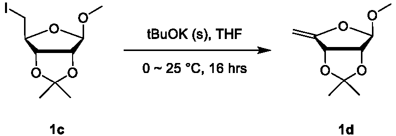

Compound **1c** (203 g, 646 mmol, 1.00 eq) was dissolved in tetrahydrofuran (2.03 L), and potassium tert-butoxide (145 g, 1.29 mol, 1.29 eq) was added in portions at 0°C. The mixture was stirred at 25°C for 16 h and then cooled to 5°C. The mixture was quenched with ice water (1.1 L), extracted with methyl tert-butyl ether (2.0 L), washed with saturated brine (2.0 L), dried over anhydrous sodium sulfate, filtered, and concentrated. The obtained crude product was purified by silica gel column chromatography (eluent: petroleum ether/ethyl acetate) to obtain compound **1d** (160 g, 91.3%).

**¹H NMR:** 400 MHz CDCl₃ δ 5.11 (s, 1H), 5.02 (d, J = 5.6 Hz, 1H), 4.06 (d, J = 1.6 Hz, 1H), 4.50 (d, J = 5.6 Hz, 1H), 4.39 (d, J = 1.6 Hz, 1H), 3.41 (s, 3H), 3.24-3.20 (m, 1H), 3.09 (t, J = 10.0 Hz, 1H) , 1.47 (s, 3H), 1.35 (s, 3H).

### 4. Preparation of Compound 1e

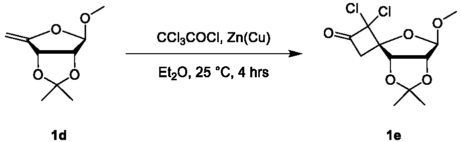

A zinc-copper couple (90.0 g, 1.37 mol, 5.50 eq) was dispersed in diethyl ether (200 mL). Compound **1d** (60 g, 241 mmol, 1.00 eq) was added at 25°C, and a solution of trichloroacetyl chloride (61.5 g, 338 mmol, 1.40 eq) in diethyl ether (200 mL) was added dropwise. The mixture was stirred for 1 h. After filtration, the filter cake was washed with methyl tert-butyl ether (500 mL), and the filtrate was poured into a saturated aqueous sodium bicarbonate solution (2.50 L) and filtered. The filtrate was washed three times with saturated brine (500 mL), dried over anhydrous sodium sulfate, filtered, and concentrated to obtain crude compound **1e** (71.8 g).

**¹H NMR:** 400 MHz CDCl₃ *δ* 5.12 (d, J = 5.6 Hz, 1H), 5.09 (s, 1H), 4.70 (d, J = 6.0 Hz, 1H), 3.70-3.55 (m, 2H), 3.54 (s, 3H), 1.45 (s, 3H), 1.36 (s, 3H).

### 5. Preparation of Compound 1f

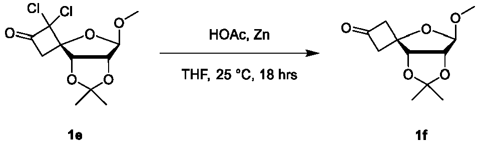

Compound **1e** (71.8 g, 242 mmol, 1.00 eq) was dissolved in tetrahydrofuran (1.60 L). Glacial acetic acid (69.1 mL, 1.20 mol, 5.00 eq) was added, and zinc powder (142 g, 2.17 mol, 9.00 eq) was added in portions. Stirred at 25°C for 18 h. After the reaction was complete, the reaction mixture was filtered, and the filtrate was concentrated to obtain a crude product. The crude product was dissolved in methyl tert-butyl ether, poured into a saturated aqueous sodium bicarbonate solution (2.0 L), and filtered. The filtrate was washed twice with saturated brine (500 mL), dried over anhydrous sodium sulfate, filtered, and concentrated. The obtained crude product was purified by silica gel column chromatography (eluent: petroleum ether/ethyl acetate/dichloromethane) to obtain compound **1f** (31 g, 56.3% yield in two steps).

**¹H NMR:** 400 MHz CDCl₃ *δ* 4.97 (s, 1H), 4.70-4.67 (m, 2H), 3.54-3.48 (m, 2H), 3.37-3.31 (m, 4H), 3.16-3.10 (m, 1H), 3.09-3.03 (m, 1H), 1.43 (s, 3H), 1.34 (s, 3H).

### 6. Preparation of Compound 1g

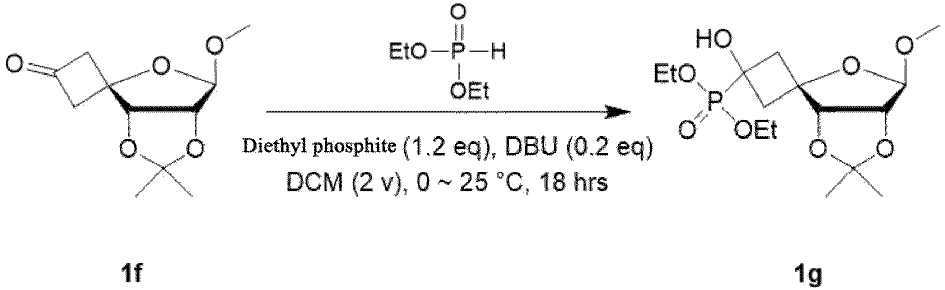

Compound **1f** (50 g, 219 mmol, 1.00 eq) and diethyl phosphite (33.2 g, 240 mmol, 1.20 eq) were dissolved in dichloromethane (100 mL); 1,8-diazabicycloundec-7-ene (DBU, 6.67 g, 43.8 mmol, 0.20 eq) was added at 0°C and stirred at 25°C for 18 h. The reaction mixture was washed three times with saturated NH₄Cl (100 mL) and once with saturated brine (100 mL), dried over anhydrous sodium sulfate, and concentrated. The obtained crude product was purified by silica gel column chromatography (eluent: petroleum ether/dichloromethane) to obtain compound **1g** (70 g, 87.2%).

**¹H NMR:** 400 MHz CDCl₃ *δ* 5.27 (s, 1H), 5.01 (brs, 1H), 4.78 (s, 1H), 4.65 (d, J = 5.6 Hz, 1H), 4.49 (d, J = 6.0 Hz, 1H), 4.20-4.05 (m, 5H), 3.31 (s, 1H), 3.29-3.05 (m, 1H), 2.75-2.68 (m, 1H), 2.50-2.40 (m, 1H), 2.35-2.25 (m, 1H), 1.36-1.25 (m, 12 H).

### 7. Preparation of Compound 1h

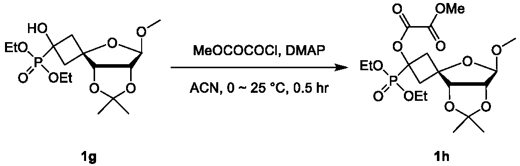

Compound **1g** (80.2 g, 219 mmol, 1.00 eq) and DMAP (40.1 g, 328 mmol, 1.50 eq) were dissolved in acetonitrile (562 mL). Methyl oxalyl chloride (40.2 g, 328 mmol, 1.50 eq) was added at 5°C. Stirred at 25°C for half an hour. The reaction mixture was concentrated. The obtained crude product was dissolved in ethyl acetate, washed five times with saturated ammonium chloride (300 mL), once with water (200 mL), and once with brine (200 mL), dried over anhydrous sodium sulfate, filtered, and concentrated to obtain crude compound **1h** (99 g).

**¹H NMR:** 400 MHz CDCl₃ *δ* 4.88-4.80 (m, 2H), 4.67-4.53 (m, 2H), 4.24-4.12 (m, 4H), 3.89 (s, 3H), 3.51-3.43 (m, 1H), 3.35-3.33 (m, 3H), 3.15-3.07 (m, 1H), 2.92-2.80 (m, 1H), 2.69-2.61 (m, 1H), 1.39-1.29 (m, 12H).

### 8. Preparation of Compound 1i

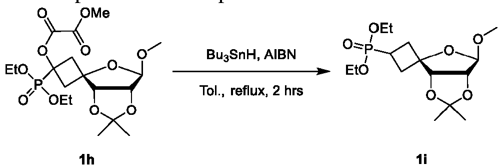

Compound **1h** (99 g, 218 mmol, 1.00 eq) was dissolved in anhydrous toluene (1.00 L). Tri-n-butyltin hydride (76.4 g, 262 mmol, 1.20 eq) and azobisisobutyronitrile (AIBN, 1.08 g, 6.56 mmol, 0.03 eq) were added. Refluxed for 2 h. The reaction mixture was concentrated. The obtained crude product was purified by silica gel column chromatography (eluent: petroleum ether/ethyl acetate) to obtain crude compound **1i** (146 g).

**¹H NMR:** 400 MHz CDCl₃ *δ* 4.75-4.49 (m, 3H), 4.05-3.95 (m, 4H), 3.26-3.24 (m, 3H), 2.76-2.07 (m, 5H), 1.39-1.29 (m, 12H).

### 9. Preparation of Compound 1j

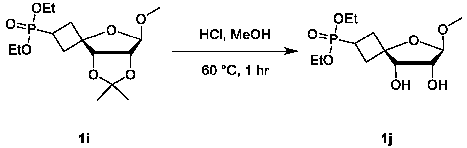

Compound **1i** (141 g, 201 mmol, 1.00 eq) was dissolved in methanol (1.41 L). An aqueous solution of HCl (704 mL, 1.40 mol, 2 M, 7.00 eq) was added and stirred at 60°C for 1 h. The reaction mixture was extracted with a mixture of methyl tert-butyl ether/petroleum ether. The aqueous phase was collected, adjusted to pH 8 with saturated sodium bicarbonate, and concentrated. Tetrahydrofuran was added to the residue and filtered. The filtrate was concentrated to obtain crude compound **1j** (62.4 g, 201 mmol).

### 10. Preparation of Compound 1k

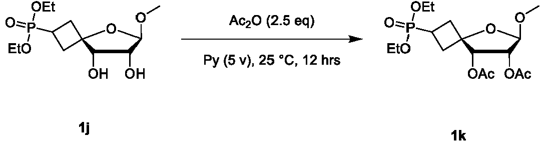

Compound **1j** (62.4 g, 201 mmol, 1.00 eq) was dissolved in pyridine (300 mL). Acetic anhydride (47.4 mL, 502 mmol, 2.50 eq) was added and stirred at 25°C for 12 h. The reaction mixture was diluted with saturated sodium bicarbonate (1.00 L), extracted twice with ethyl acetate (600 mL), dried over anhydrous sodium sulfate, filtered, and concentrated. The obtained crude product was purified by silica gel column chromatography (eluent: petroleum ether/ethyl acetate) to obtain compound **1k** (74 g, 93.3%).

**¹H NMR:** 400 MHz CDCl₃ δ 5.38-5.10 (m, 3H), 4.07-4.03 (m, 4H), 3.36-3.32 (m, 3H), 2.41-2.05 (m, 5H), 2.04-1.98 (m, 9H), 1.27-1.23 (m, 6H).

### 11. Preparation of Compound 1l

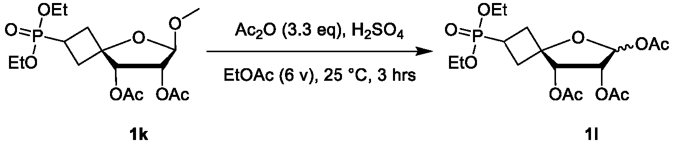

Compound **1k** (79.3 g, 201 mmol, 1.00 eq) was dissolved in ethyl acetate (476 mL). Acetic anhydride (62.6 mL, 663 mmol, 3.30 eq) and concentrated sulfuric acid (5.38 mL, 100 mmol, 0.50 eq) were added. Stirred at 25°C for 3 h. The reaction mixture was neutralized with saturated aqueous sodium bicarbonate solution, and extracted twice with ethyl acetate (1.00 L). The organic phases were combined, washed twice with saturated brine (500 mL), dried over anhydrous sodium sulfate, filtered, and concentrated. The crude product was purified by silica gel column chromatography to obtain compound **1l** (43 g, 50.6%).

**¹H NMR:** 400 MHz CDCl₃ δ 6.11 (d, J = 2.8 Hz, 1H), 5.44-5.39 (m, 1H), 5.33-5.32 (m, 1H), 4.10-4.05 (m, 5H), 2.80-2.35 (m, 4H), 2.12-2.02 (m, 9H), 1.32-1.23 (m, 6H).

### 12. Preparation of Compound 1n

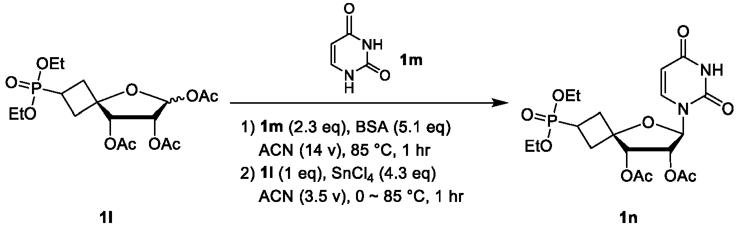

Compound **1m** (8.55 g, 76.2 mmol, 2.30 eq) and bis(trimethylsilyl)acetamide (BSA, 34.3 g, 169 mmol, 5.10 eq) were suspended in acetonitrile (200 mL) and stirred at 85°C for 1 h. After cooling, a solution of compound **1l** (14.0 g, 33.1 mmol, 1.00 eq) in acetonitrile (50.0 mL) was added, followed by tin tetrachloride (37.1 g, 142 mmol, 4.30 eq). The mixture was stirred at 25°C for 15 min and then stirred in an oil bath at 85°C for 45 min. After cooling, the reaction mixture was poured into a saturated aqueous sodium bicarbonate solution (1.50 L), and extracted three times with dichloromethane (700 mL). The organic phases were combined, washed with saturated brine (500 mL), dried over anhydrous sodium sulfate, filtered, and concentrated. The crude product was purified by silica gel column chromatography (eluent: dichloromethane/methanol) to obtain compound **1n** (14.4 g, 84.3%).

**¹H NMR:** 400 MHz CDCl₃ *δ* 9.70 (brs, 0.46H), 9.60 (brs, 0.60H), 7.17 (d, J = 8.0 Hz, 0.60H), 7.12 (d, J = 8.0 Hz, 0.49H), 5.98 (d, J = 6.4 Hz, 0.43H), 5.93 (d, J = 5.2 Hz, 0.55H), 5.78 (dt, J1 = 8.0 Hz, J2 = 2.0 Hz, 1H), 5.50-5.45 (m, 2H), 4.45-4.02 (m, 5H), 2.77-2.42 (m, 6H), 2.18 (s, 1.22H), 2.16 (s, 1.74 H), 2.04 (s, 1.31H), 2.02 (s, 1.67H), 1.32-1.28 (m, 6H).

### 13. Preparation of Compound 1o

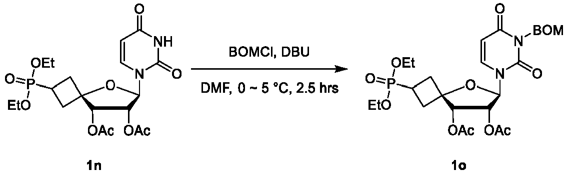

Compound **1n** (8.30 g, 17.4 mmol, 1.00 eq) was dissolved in DMF (40.0 mL). DBU (2.93 g, 19.2 mmol, 1.10 eq) was added, and benzyl chloromethyl ether (BOMCl, 3.01 g, 19.2 mmol, 1.10 eq) was added after cooling. The mixture was stirred at 0-5°C for 2.5 h. The reaction mixture was diluted with saturated ammonium chloride (160 mL), and extracted twice with dichloromethane (80.0 mL). The organic phases were combined, washed with saturated brine (80 mL), dried over anhydrous sodium sulfate, filtered, and concentrated. The crude product was purified by silica gel column chromatography (eluent: dichloromethane/methanol) to obtain compound **1o** (12.0 g, 92.3%).

**¹H NMR:** 400 MHz CDCl₃ *δ* 7.36-7.24 (m, 5H), 7.15-7.09 (m, 1H), 5.96-5.92 (m, 1H), 5.81-5.79 (m, 1H), 5.52-5.42 (m, 4H), 4.67 (s, 2H), 4.15-4.05 (m, 4H), 2.80-2.45 (m, 5H), 2.19-2.17 (m, 3H), 2.05-2.03 (m, 3H), 1.34-1.30 (m, 6H).

### 14. Preparation of Compound 1p

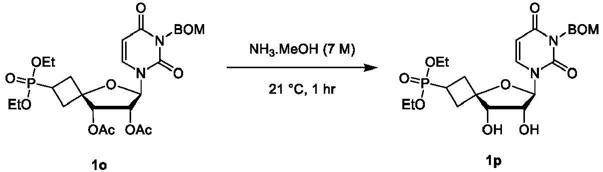

Compound **1o** (10.4 g, 17.4 mmol, 1.00 eq) was dissolved in ammonia methanol solution (29.9 mL, 7 M) and stirred at 21°C for 1 h. The crude product was obtained by concentration and purified by silica gel column chromatography (eluent dichloromethane/methanol) to obtain compound **1p** (8.00 g, 62.7%).

**¹H NMR:** 400 MHz CDCl₃ *δ* 7.34-7.06 (m, 5H), 5.83 (s, 0.52H), 5.71 (d, J = 8.4 Hz, 0.41H), 5.661 (d, J = 8.4 Hz, 0.53H), 5.60 (d, J = 2.0 Hz, 0.59H), 5.53 (d, J = 5.2 Hz, 0.41H), 5.42-5.36 (m, 2H), 4.62 (s, 2H), 4.51 (d, J = 3.6 Hz, 0.45H), 3.72 (d, J = 2.8 Hz, 0.36H), 3.54 (d, J = 2.0 Hz, 0.48H), 2.93-2.40 (m, 5H), 2.24-2.13 (m, 1H), 1.27-1.23 (m, 6H).

### 15. Preparation of Compound 1q

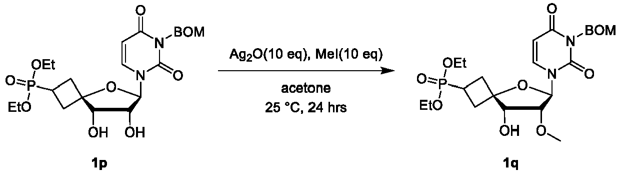

Compound **1p** (1 g, 1.95 mmol, 1.00 eq) was dissolved in acetone (19.6 mL). Silver oxide (3.63 g, 15.6 mmol, 10.0 eq) and methyl iodide (1.22 mL, 19.5 mmol, 10.0 eq) were added and stirred at 25°C for 24 h. The reaction mixture was filtered and concentrated to obtain crude compound **1q** (1.36 g).

**¹H NMR:** 400 MHz CDCl₃ δ 5.11 (s, 1H), 5.02 (d, J = 5.6 Hz, 1H), 4.06 (d, J = 1.6 Hz, 1H), 4.50 (d, J = 5.6 Hz, 1H), 4.39 (d, J = 1.6 Hz, 1H), 3.41 (s, 3H), 3.24-3.20 (m, 1H), 3.09 (t, J = 10.0 Hz, 1H) , 1.47 (s, 3H), 1.35 (s, 3H).

### 16. Preparation of Compounds 1q-1 and 1q-2

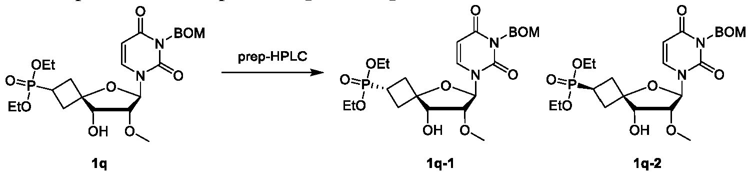

Crude compound **1q** (10.9 g, 20.7 mmol, 1.00 eq) was purified by C18 reversed-phase column chromatography (aqueous ammonium bicarbonate solution/acetonitrile) to obtain compound **1q-1** (1.50 g, 11.5%) and compound **1q-2** (1.50 g, 11.5%).

### Compound 1q-1:

**m/z:** ES+ [M+H]+ 525.2
**HPLC:** Retention time: 0.820 min (Column: XBridge C18 2.1*50mm,5um; Mobile phase: A: 10mM NH₄HCO₃ aqueous solution B: Acetonitrile; Gradient: 0-0.01 min 5% B, 0.01-0.7 min 5-95% B, 0.7-1.16 min 95% B, 1.16-1.5min, 95%-5% B; Flow rate: 1.5 mL/min; Column temp.: 40°C)
**¹H NMR:** 400 MHz CDCl₃ *δ* 7.37-7.08 (m, 5H), 7.09 (d, J = 8.0 Hz, 1H), 5.76-5.74 (m, 2H), 5.49-5.44 (m, 2H), 4.73-4.67 (m, 2H), 4.15-4.06 (m, 4H), 4.02 (d, J = 4.8 Hz, 1H), 3.87-3.85 (m, 1H), 3.56 (s, 3H), 2.81-2.64 (m, 2H), 2.50-2.39 (m, 2H), 2.36-2.27 (m, 1H), 1.34-1.30 (m, 6H).

### Compound 1q-2:

m/z: ES+ [M+H]+ 525.2
**HPLC:** Retention time: 0.836 min (Column: XBridge C18 2.1*50mm,5um; Mobile phase: A: 10mM NH₄HCO₃ aqueous solution B: Acetonitrile; Gradient: 0-0.01 min 5% B, 0.01-0.7 min 5-95% B, 0.7-1.16 min 95% B, 1.16-1.5min, 95%-5% B; Flow rate: 1.5 mL/min; Column temp.: 40°C)
**¹H NMR:** 400 MHz CDCl₃ *δ* 7.37-7.27 (m, 5H), 7.08 (d, J = 8.0 Hz, 1H), 5.75-5.73 (m, 2H), 5.50-5.45 (m, 2H), 4.73-4.67 (m, 2H), 4.18-4.08 (m, 4H), 4.06 (d, J = 4.8 Hz, 1H), 3.88-3.86 (m, 1H), 3.56 (s, 3H), 3.06-2.95 (m, 1H), 2.83-2.41 (m, 5H), 1.34-1.31 (m, 6H).

### 17. Preparation of Compound 1r-1

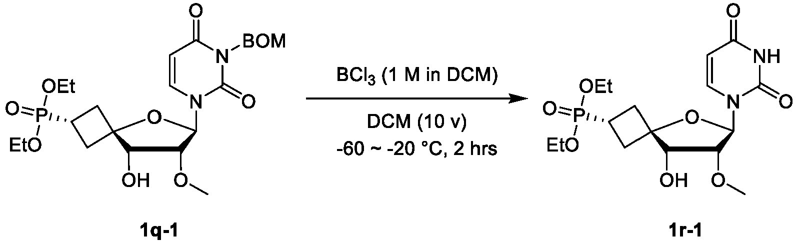

Compound **1q-1** (1.00 g, 1.90 mmol, 1.00 eq) was dissolved in anhydrous dichloromethane (DCM, 20.0 mL) and cooled to -60°C. Boron trichloride (7.62 mL, 7.62 mmol, 1 M in dichloromethane, 4.00 eq) was added and stirred at -20°C for 1 h. The reaction mixture was quenched with absolute ethanol (10.0 mL), neutralized to about pH 7 with concentrated ammonia, and concentrated. The crude product was added to a mixed solution of dichloromethane and ethanol and filtered. The filtrate was concentrated and the residue was purified by C18 reversed-phase column chromatography (aqueous ammonium bicarbonate solution/acetonitrile) to obtain compound **1r-1** (400 mg, 51.9%).
m/z: ES+ [M+H]+ 405.3
**¹H NMR:** 400 MHz CDCl₃ *δ* 8.51-8.43 (m, 1H), 7.15 (d, J = 8.4 Hz, 1H), 5.78 (d, J = 3.2 Hz, 1H), 5.76 (dd, J1 = 8.0 Hz, J2 = 2.4 Hz, 1H), 4.16-4.07 (m, 5H), 3.94 (dd, J1 = 4.8 Hz, J2 = 3.2 Hz, 1H), 3.55 (s, 3H), 2.96-2.94 (m, 1H),2.79-2.66 (m, 2H), 2.52-2.41 (m, 2H), 2.35-2.26 (m, 1H), 1.34-1.31 (m, 6H).

### 18. Preparation of Compound 1r-2

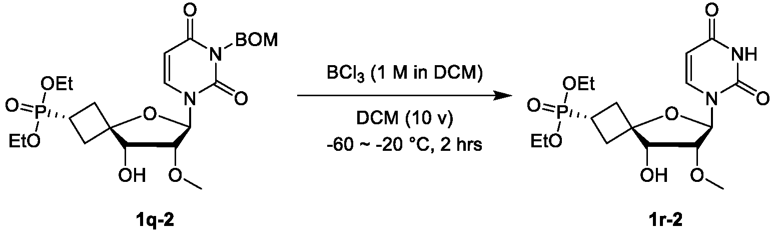

Compound **1r-2** was obtained by the same synthetic method as compound **1r-1.**

**¹H NMR:** 400 MHz CDCl₃ *δ* 9.16-8.81 (m, 1H), 7.15 (d, J = 8.0 Hz, 1H), 5.78 (d, J = 2.8 Hz, 1H), 5.76 (dd, J1 = 8.0 Hz, J2 = 2.0 Hz, 1H), 4.65 (brs, 1H), 4.18-4.08 (m, 5H), 3.96 (dd, J1 = 4.8 Hz, J2 = 3.2 Hz, 1H), 3.53 (s, 3H), 3.05-2.95 (m, 1H), 2.81-2.41 (m, 2H), 1.34-1.30 (m, 6H).

### 19. Preparation of Compound E1-1

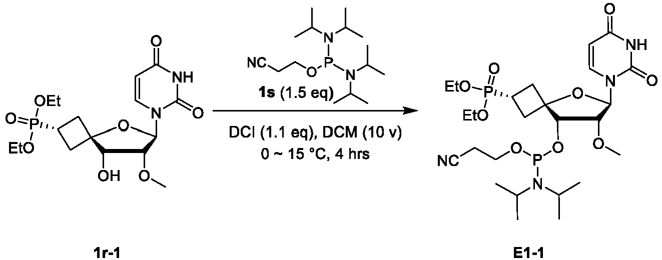

Compound **1r-1** (500 mg, 1.23 mmol, 1.00 eq) was dissolved in dichloromethane (5.00 mL). Compound **1s** (0.59 mL, 1.85 mmol, 1.50 eq) was added, and 4,5-dicyanoimidazole (160 mg, 1.36 mmol, 1.10 eq) was added after cooling to 0°C and stirred at 15°C for 4 h. The reaction mixture was quenched with saturated sodium bicarbonate (10.0 mL) and extracted twice with dichloromethane (10.0 mL). The crude product was obtained by concentration and purified by silica gel column chromatography (eluent: dichloromethane/acetone) to obtain compound **E1-1** (530 mg, 70.9%).

**¹H NMR:** 400 MHz CDCl₃ *δ* 9.17 (brs, 1H), 7.24 (d, J = 8.4 Hz, 1H), 5.83-5.79 (m, 1H), 5.64-5.61 (m, 1H), 4.43-4.33 (m, 1H), 4.09-3.62 (m, 9H), 3.43-3.38 (m, 3H), 2.86-2.66 (m, 3H), 2.57-2.21 (m, 4H), 1.28-1.18 (m, 18H).

### 20. Preparation of Compound E1-2

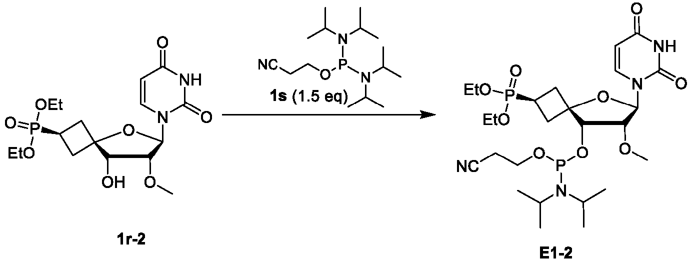

Compound **1r-2** (500 mg, 1.23 mmol, 1.00 eq) was dissolved in dichloromethane (5.00 mL). Compound **1s** (0.59 mL, 1.85 mmol, 1.50 eq) was added, and 4,5-dicyanoimidazole (160 mg, 1.36 mmol, 1.10 eq) was added after cooling to 0°C and stirred at 15°C for 4 h. The reaction mixture was quenched with saturated sodium bicarbonate (10.0 mL) and extracted twice with dichloromethane (10.0 mL). The crude product was obtained by concentration, and purified by silica gel column chromatography (eluent: dichloromethane/acetone) to obtain compound **E1-2** (480 mg, 64.0%).

**¹H NMR:** 400 MHz CDCl₃ *δ* 9.06 (brs, 1H), 7.33-7.31 (m, 1H), 5.89-5.88 (m, 1H), 5.66-5.64 (m, 1H), 4.49-4.40 (m, 1H), 4.08-3.64 (m, 9H), 3.40-3.36 (m, 3H), 3.00-2.19 (m, 7H), 1.29-1.18 (m, 18H).

### EXAMPLE 2 Preparation of Compounds E2-1 and E2-2

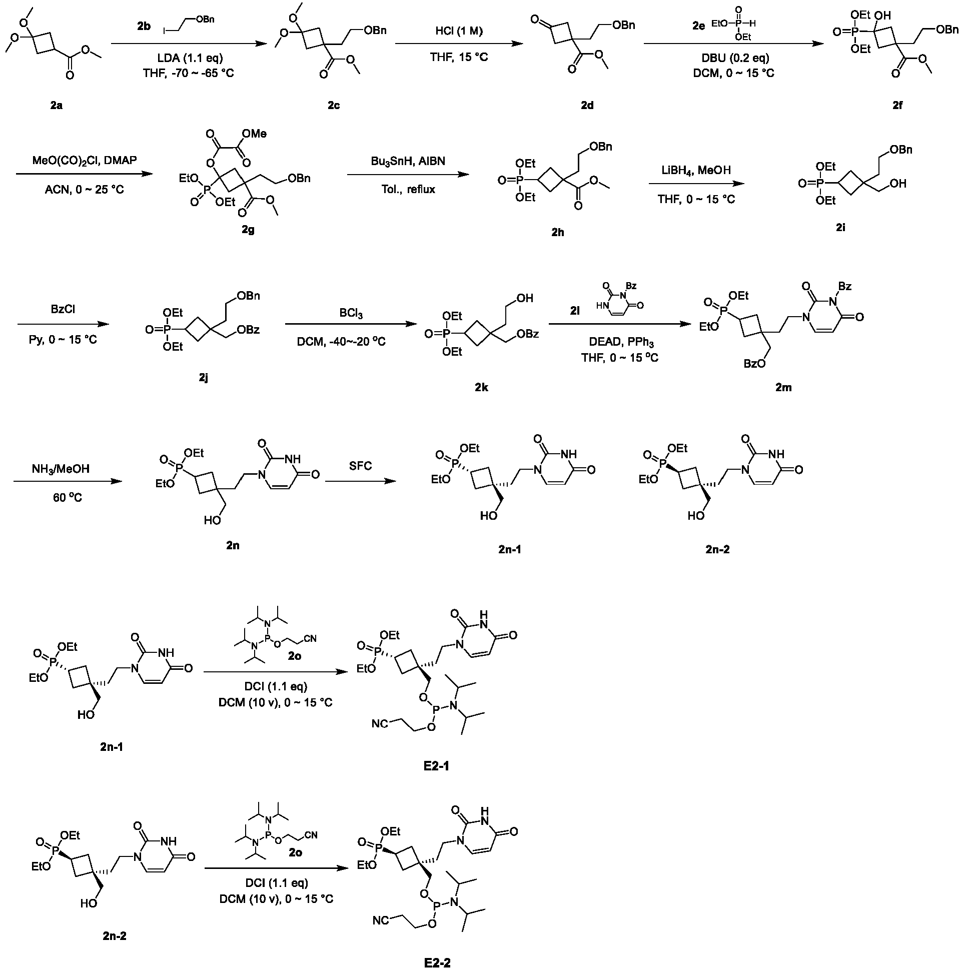

### 1. Preparation of Compound 2c

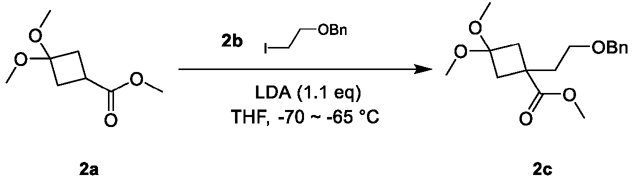

A solution of compound 2a (86.0 g, 493 mmol) in THF (250 mL) was dropped into a solution of LDA in THF (271 mL, 543 mmol, 2 M) at -70°C to -65°C under a nitrogen atmosphere. After 30 min, a solution of compound 2b (142 g, 543 mmol) in THF (250 mL) was added and stirred for 1 h. After the reaction was complete, the reaction mixture was poured into a saturated aqueous ammonium chloride solution (2 L), and extracted three times with methyl tert-butyl ether. The organic phases were combined, washed with saturated brine, dried over anhydrous sodium sulfate, and concentrated under reduced pressure. The residue was purified by silica gel column chromatography (eluent: petroleum ether/ethyl acetate) to obtain compound 2c (113 g, 70.6%).

¹H NMR: 400 MHz DMSO-*d₆ δ* 7.35-7.31 (m, 2H), 7.28-7.24 (m, 3H), 4.36 (s, 2H), 3.52 (s, 3H), 3.39-3.36 (m, 2H), 3.03 (s, 3H), 3.00 (s, 3H), 2.46-2.45 (m, 1H), 2.44-2.43 (m, 1H), 2.12-2.11 (m, 1H), 2.10-2.09 (m, 1H), 2.03-2.00(m, 2H).

### 2. Preparation of Compound 2d

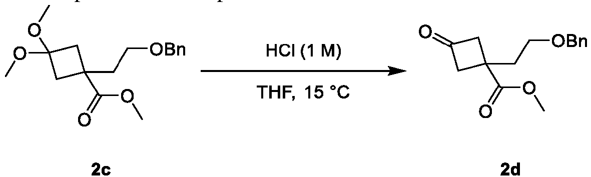

1 M hydrochloric acid (217 mL, 217 mmol) was added to compound 2c (13.4 g, 43.4 mmol) in THF (200 mL) at 15°C and stirred for 24 h. The reaction mixture was extracted three times with ethyl acetate. The organic phases were combined, washed twice with saturated aqueous sodium bicarbonate solution, washed with saturated brine, dried over anhydrous sodium sulfate, and concentrated under reduced pressure. The residue was purified by silica gel column chromatography (eluent: petroleum ether/ethyl acetate) to obtain compound 2d (9.9 g, 76.7%).

¹H NMR: 400 MHz CDCl₃ *δ* 7.36-7.26 (m, 5H), 4.45 (s, 2H), 3.68 (s, 3H), 3.59-3.56 (m, 3H), 3.53-3.49 (m, 1H), 3.10-3.08 (m, 1H), 3.06-3.04 (m, 1H), 2.26 (t, *J* = 6.0 Hz, 2H).

### 3. Preparation of Compound 2f

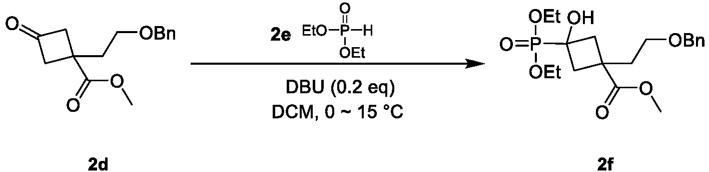

DBU (1.15 g, 7.54 mmol) was added to compound 2d (9.90 g, 37.7 mmol) and compound 2e (6.25 g, 45.2 mmol) in dichloromethane (20 mL) at 0°C-5°C and stirred at 15°C for 24 h. The reaction mixture was concentrated under reduced pressure. The residue was purified by silica gel column chromatography (eluent: petroleum ether/ethyl acetate/dichloromethane) to obtain compound 2f (15.5 g, 92.3%).

¹H NMR: 400 MHz CDCl₃ *δ* 7.34-7.26 (m, 5H), 4.42-4.41 (m, 2H), 4.20-4.11 (m, 5H), 3.64-3.60 (m, 3H), 3.49-3.45 (m, 1H), 3.44-3.41 (m, 1H), 3.11-3.04 (m, 1H), 2.70-2.56 (m, 2H), 2.28-2.25 (m, 1H), 2.20-2.04 (m, 2H), 1.38-1.30 (m, 6H).

### 4. Preparation of Compound 2g

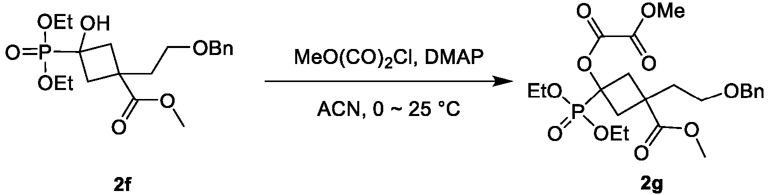

Compound 2f (14.5 g, 36.2 mmol) and DMAP (6.64 g, 54.3 mmol) were dissolved in acetonitrile (100 mL). Methyl oxalyl chloride (5.00 mL, 54.3 mmol) was added at 5°C. Stirred at 15°C for half an hour. The reaction mixture was concentrated. The obtained crude product was dissolved in ethyl acetate, washed with saturated ammonium chloride and brine in sequence, dried over anhydrous sodium sulfate, and concentrated under reduced pressure to obtain crude compound 2g (16.4 g).

### 5. Preparation of Compound 2h

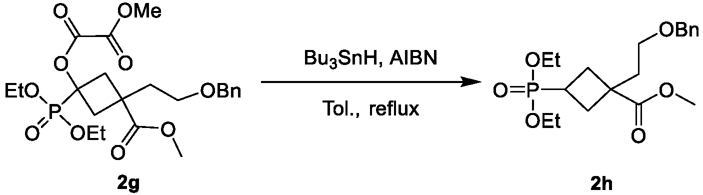

Compound 2g (17.5 g, 35.9 mmol) was dissolved in anhydrous toluene (175 mL). Tri-n-butyltin hydride (12.5 g, 43.1 mmol) and azobisisobutyronitrile (AIBN, 0.30 g, 1.79 mmol) were added. Refluxed for 2 h. The reaction mixture was concentrated. The obtained crude product was purified by silica gel column chromatography (eluent: petroleum ether/ethyl acetate) to obtain compound 2h (11.3 g, 79.9% in two steps).

¹H NMR: 400 MHz CDCl₃ δ 7.34-7.24 (m, 5H), 4.43-4.41 (m, 2H), 3.63-3.60 (m, 3H), 3.49-3.40 (m, 2H), 2.79-2.59 (m, 3H), 2.39-2.28 (m, 1H), 2.17-2.13 (m, 2H), 1.32-1.29 (m, 6H).

### 6. Preparation of Compound 2i

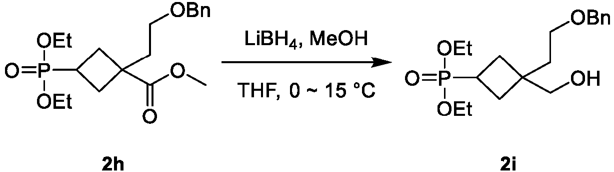

Compound 2h (8.80 g, 22.8 mmol) was dissolved in THF (176 mL) and methanol (3.1 mL, 76 mmol). Lithium borohydride (2.49 g, 114 mmol) was added at 0°C and stirred at 15°C for 2 h. The reaction mixture was added with water (100 mL), adjusted to pH 3 with 1 M hydrochloric acid, and extracted three times with dichloromethane. The organic phases were combined, washed with saturated aqueous sodium bicarbonate solution and saturated brine in sequence, dried over anhydrous sodium sulfate, and concentrated. The obtained crude product was purified by silica gel column chromatography (eluent: dichloromethane/methanol) to obtain compound 2i (7.86 g, 52.4%).

¹H NMR: 400 MHz DMSO-d₆ δ 7.35-7.25 (m, 5H), 4.70-4.68 (m, 1H) 4.44-4.42 (m, 2H), 3.97-3.92 (m, 4H), 3.49-3.26 (m, 3H), 2.60-2.50 (m, 1H), 1.96-1.67 (m, 5H), 1.21-1.18 (m, 6H).

### 7. Preparation of Compound 2j

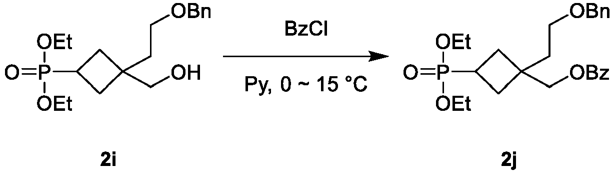

Compound 2i (7.86 g, 22.0 mmol) was dissolved in pyridine (47 mL). Benzoyl chloride (2.81 mL, 24.2 mmol) was added at 5°C. Stirred at 15°C for 1 h. The reaction mixture was concentrated. The residue was dissolved in ethyl acetate (40 mL), washed with 2 M hydrochloric acid, saturated aqueous sodium bicarbonate solution, and saturated brine in sequence, dried over anhydrous sodium sulfate, filtered, and concentrated to obtain crude compound 2j (10 g).

¹H NMR: 400 MHz CDCl₃ δ 8.03-7.98 (m, 2H), 7.54-7.43 (m, 1H), 7.42-7.39 (m, 2H), 7.27-7.23 (m, 5H), 4.45-4.44 (m, 2H), 4.33-4.22 (m, 2H), 4.11-4.03 (m, 4H), 3.56-3.51 (m, 2H), 2.72-2.62 (m, 1H), 2.45-2.05 (m, 4H), 1.97-1.93 (m, 1H), 1.30-1.27 (m, 6H).

### 8. Preparation of Compound 2k

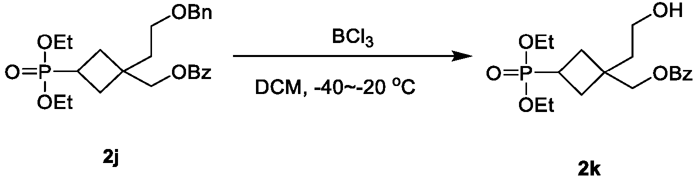

Compound 2j (8.00 g, 17.3 mmol) was dissolved in dichloromethane (160 mL). A solution of boron trichloride (69.4 mL, 69.4 mmol) in dichloromethane (1 M) was added at -60°C and reacted at -20°C for 1 h. To the reaction mixture, ethanol (40.5 mL, 694 mmol) and saturated aqueous sodium bicarbonate solution were added in sequence. The organic phase was collected, washed with saturated aqueous sodium bicarbonate solution and saturated brine in sequence, dried over anhydrous sodium sulfate, filtered, and concentrated. The residue was purified by silica gel column chromatography (eluent: petroleum ether/ethyl acetate, dichloromethane/methanol) to obtain compound 2k (6.10 g, 85.3%).

¹H NMR: 400 MHz CDCl₃ δ 8.06-7.99 (m, 2H), 7.59-7.53 (m, 1H), 7.47-7.42 (m, 2H), 4.35-4.28 (m, 2H), 4.16-4.03 (m, 4H), 3.83-3.74 (m, 2H), 2.78-2.65 (m, 1H), 2.50-2.33 (m, 2H), 2.21-2.06 (m, 2H), 2.00-1.97 (m, 1H), 1.86-1.82 (m, 1H), 1.37-1.27 (m, 6H).

### 9. Preparation of Compound 2m

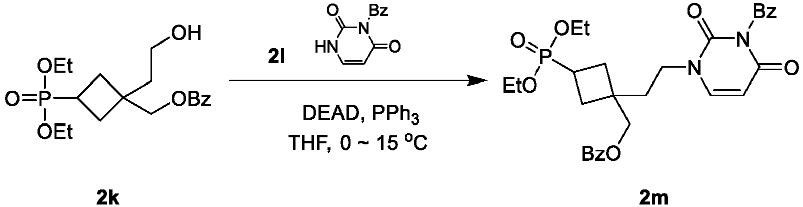

Compound 2k (5.80 g, 15.6 mmol), compound 2l (3.55 g, 16.4 mmol), and triphenylphosphine (4.93 g, 18.7 mmol) were dissolved in tetrahydrofuran (58 mL). Diethyl azodicarboxylate (DEAD, 3.27 g, 18.7 mmol) was added at 5°C and stirred at 15°C for 2 h. The reaction mixture was added with water and extracted twice with ethyl acetate. The organic phases were combined, washed with saturated brine, dried over anhydrous sodium sulfate, filtered, and concentrated under reduced pressure. The residue was extracted with a mixture of methyl tert-butyl ether/petroleum ether. The aqueous phase was collected, adjusted to pH 8 with saturated sodium bicarbonate, and concentrated. The residue was purified by silica gel column chromatography (eluent: petroleum ether/ethyl acetate, dichloromethane/methanol) to obtain compound 2m (7.40 g, 61.9%).

¹H NMR: 400 MHz CDCl₃ δ 8.06-8.00 (m, 2H), 7.94-7.91 (m, 2H), 7.66-7.51 (m, 2H), 7.42-7.32 (m, 5H), 7.32-7.29 (m, 1H), 5.80-5.77 (m, 1H), 4.39-4.38 (m, 2H), 4.12-4.02 (m, 5H), 3.87-3.83 (m, 2H), 2.79-2.67 (m, 1H), 2.44-1.95 (m, 6H), 1.33-1.25 (m, 6H).

### 10. Preparation of Compound 2n

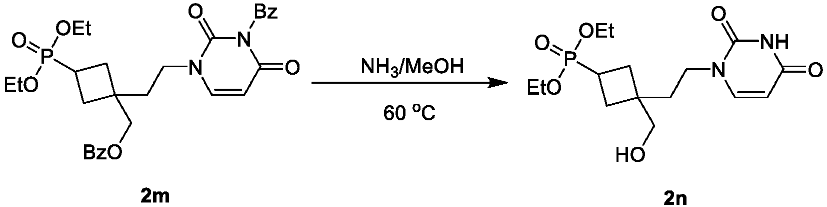

Compound 2m (3.00 g, 5.27 mmol) was dissolved in ammonia methanol solution (150 mL, 7 M) and reacted at 60°C for 48 h. The reaction mixture was concentrated. The residue was purified by silica gel column chromatography (eluent: dichloromethane/methanol) to obtain crude compound 2n (3.10 g).

¹H NMR: 400 MHz DMSO-d₆ δ 11.21 (brs, 1H), 7.69-7.63 (m, 1H), 5.56-5.52 (m, 1H), 4.80-4.77 (m, 1H), 3.98-3.93 (m, 4H), 3.60-3.69 (m, 2H), 3.42 (d, J = 5.2 HZ, 1H), 2.75-2.55 (m, 1H), 1.93-1.87 (m, 6H), 1.22-1.18 (m, 6H).

### 11. Preparation of Compounds 2n-1 and 2n-2

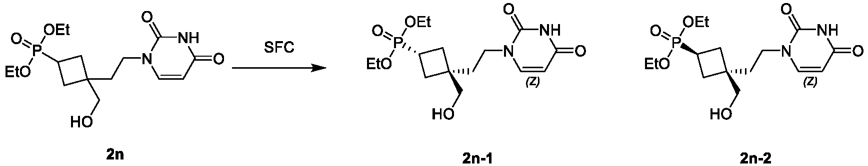

Compound 2n (3.70 g, 10.2 mmol) was purified by SFC to obtain compound 2n-1 (2.00 g, 50.7%) and compound 2n-2 (1.00 g, 24.1%). Purification column: DAICEL CHIRALPAK AD (250 mm*30 mm, 10 µm); Mobile phase: [A: CO₂; B: IPA]; B%: 40.00%-40.00%, 3.00 min.

### Compound 2n-1:

HPLC: Retention time: 3.065 min (column: Chiralpak AD-3, 150×4.6 mm I.D., 3 µm; Mobile phase: A: CO₂, B: IPA (0.2% NH₃ (7 M methanol solution), v/v); Gradient: 0-0.5 min 10% B, 0.5-3.5 min 10-50% B, 3.5-4.5 min 50% B, 4.5-5.0 min 50%-10% B; Flow rate: 2.5 mL/min; Column temperature: 35°C)
¹H NMR: 400 MHz DMSO-d₆ δ 11.20 (brs, 1H), 7.64 (d, J = 8.0 Hz, 1H), 5.53 (d, J = 8.0 Hz, 1H), 4.78 (t, J = 5.2 Hz, 1H), 3.99-3.93 (m, 4H), 3.55-3.75 (m, 2H), 3.42 (d, J = 5.6 Hz, 2H), 2.70-2.60 (m, 1H), 1.93-1.87 (m, 4H), 1.68-1.67 (m, 2H), 1.22-1.18 (m, 6H).

### Compound 2n-2:

HPLC: Retention time: 3.626 min (column: Chiralpak AD-3, 150×4.6 mm I.D., 3 µm; Mobile phase: A: CO₂, B: IPA (0.2% NH₃ (7 M methanol solution), v/v); Gradient: 0-0.5 min 10% B, 0.5-3.5 min 10-50% B, 3.5-4.5 min 50% B, 4.5-5.0 min 50%-10% B; Flow rate: 2.5 mL/min; Column temperature: 35°C)
¹H NMR: 400 MHz DMSO-*d₆ δ* 11.22 (brs, 1H), 7.68 (d, J = 8.0 Hz, 1H), 5.56-5.53 (m, 1H), 4.68 (t, J = 5.2 Hz, 1H), 4.00-3.91 (m, 4H), 3.68-3.64 (m, 2H), 3.31-3.30 (m, 2H), 2.74-2.62 (m, 1H), 2.03-1.93 (m, 2H), 1.88-1.75 (m, 4H), 1.22-1.19 (m, 6H).

### 12. Preparation of Compound E2-1

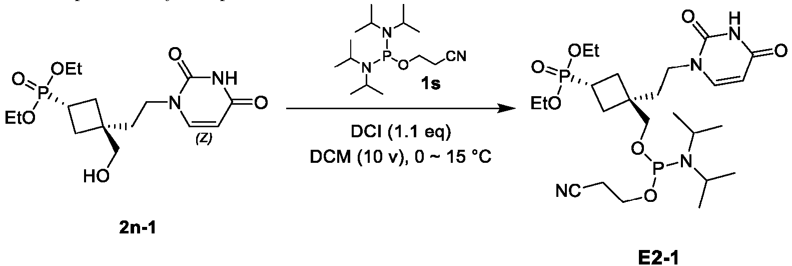

Compound 2n-1 (1.00 g, 2.77 mmol) was dissolved in dichloromethane (10 mL). Compound 1s (1.32 mL, 4.16 mmol) was added, and 4,5-dicyanoimidazole (DCI, 360 mg, 3.05 mmol) was added after cooling to 0°C and stirred at 15°C for 1 h. The reaction mixture was quenched with saturated sodium bicarbonate (20.0 mL), extracted with dichloromethane (20.0 mL), washed with saturated brine, dried over anhydrous sodium sulfate, and concentrated. The obtained crude product was purified by silica gel column chromatography (eluent: dichloromethane/acetone) to obtain compound E2-1 (1.41 g, 89.5%).

¹H NMR: 400 MHz DMSO*-d₆ δ* 11.21 (brs, 1H), 7.63 (d, *J* = 8.0 Hz, 1H), 5.55 (d, *J* = 8.0 Hz, 1H), 3.98-3.94 (m, 4H), 3.80-3.62 (m, 8H), 2.79-2.76 (m, 3H), 2.06-1.93 (m, 4H), 1.80-1.75 (m, 2H), 1.21-1.14 (m, 18H).

### 13. Preparation of Compound E2-2

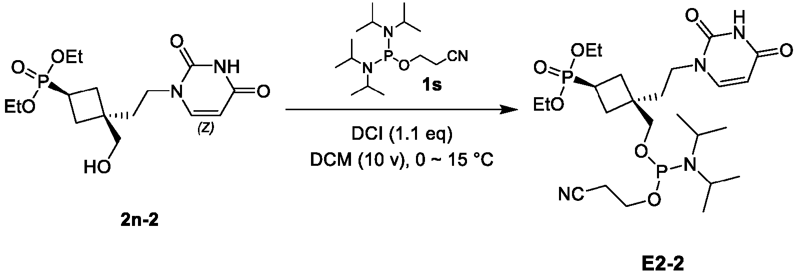

Compound 2n-2 (780 mg, 2.16 mmol) was dissolved in dichloromethane (7.8 mL). Compound 1s (936 mg, 3.24 mmol) was added, and 4,5-dicyanoimidazole (280 mg, 2.37 mmol) was added after cooling to 0°C and stirred at 15°C for 1 h. The reaction mixture was quenched with saturated sodium bicarbonate (20.0 mL), extracted with dichloromethane (20.0 mL), washed with saturated brine, dried over anhydrous sodium sulfate, and concentrated. The obtained crude product was purified by silica gel column chromatography (eluent: dichloromethane/acetone) to obtain compound E2-2 (860 mg, 60.7%).

¹H NMR: 400 MHz DMSO-*d₆ δ* 11.21 (brs, 1H), 7.66 (d, J = 8.0 HZ, 1H), 5.55 (d, J = 8.0 Hz, 1H), 4.03-3.90 (m, 4H), 3.81-3.66 (m, 4H), 3.61-3.31 (m, 4H), 2.80-2.67 (m, 3H), 2.05-1.84 (m, 6H), 1.21 (t, J = 7.2 Hz, 6H), 1.15-1.13 (m, 12H).

### EXAMPLE 3 Preparation of Compound E3

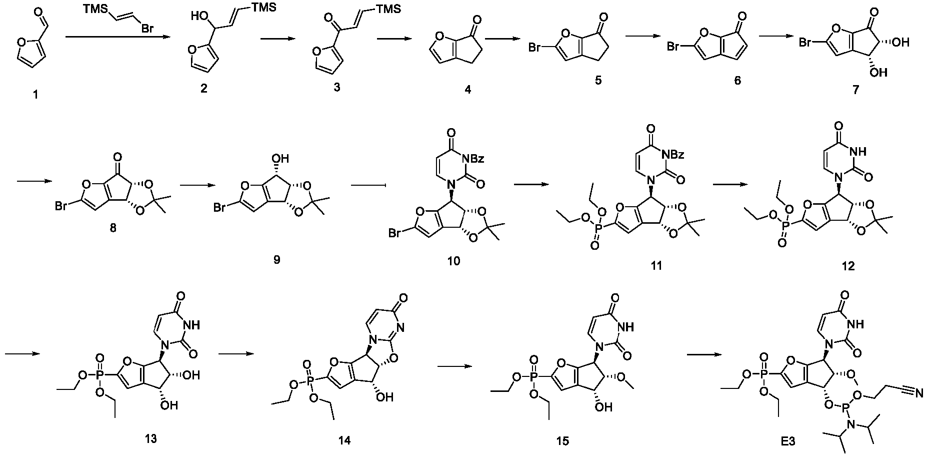

Compound E3 was prepared according to the steps described above.

### EXAMPLE 4 Preparation of siRNA

The siRNA of the present invention was prepared using the solid-phase phosphoramidite method well known in the art. The specific methods can be referred to, for example, PCT Publication Nos. WO2016081444 and WO2019105419, and are briefly described below.

### 1 Synthesis of Sense Strand (SS)

Using the solid-phase phosphoramidite method, a blank CPG solid support or solid support conjugated with L96 was used as the starting cycle, and nucleoside monomers were linked one by one according to the arrangement of sense strand nucleotides from the 3' to 5' direction. Each linkage of a nucleoside monomer involved four steps of deprotection, coupling, capping, and oxidation or thiolation to synthesize 5 µmol oligonucleotide with the synthesis conditions as follows:
Nucleoside monomers were provided in a 0.05 mol/L acetonitrile solution. The conditions for each step were identical: 25°C; deprotection for 3 times using a 3% trichloroacetic acid-dichloromethane solution; coupling twice using a 0.25 mol/L ETT-acetonitrile solution as an activator; capping twice using 10% acetic anhydride-acetonitrile and pyridine/N-methylimidazole/acetonitrile (10:14:76, v/v/v); oxidation twice using 0.05 mol/L of iodine in tetrahydrofuran/pyridine/water (70:20:10, v/v/v); thiolation twice using 0.2 mol/L PADS in acetonitrile/3-methylpyridine (1:1, v/v).

### 2 Synthesis of Antisense Strand (AS)

Using the solid-phase phosphoramidite method, a blank CPG solid support was used as the starting cycle, and nucleoside monomers or nucleotide dimers of the present invention were linked one by one according to the arrangement of antisense strand nucleotides from the 3' to 5' direction. Each linkage of a nucleoside monomer or a nucleotide dimer of the present invention involved four steps of deprotection, coupling, capping, and oxidation or thiolation. The conditions for the synthesis of a 5 µmol oligonucleotide for the antisense strand were identical to those for the sense strand.

### 3 Purification and Annealing of Oligonucleotides

### 3.1 Ammonolysis

The synthesized solid support (sense or antisense strand) was transferred to a 5 mL centrifuge tube, followed by the addition of 3% diethylamine/ammonia (v/v). The mixture was allowed to react in a thermostatic water bath at 35°C (or 55°C) for 16 h (or 8 h), and then filtered. The solid support was washed three times with ethanol/water, 1 mL each time. The filtrate was concentrated by centrifugation, and the crude product was purified.

### 3.2 Purification

The methods for purification and desalting are well known to those skilled in the art. For example, a strong anionic packing column can be used; a sodium chloride-sodium hydroxide system can be used for elution and purification. The product can be collected in tubes and desalted using a gel packing purification column. The elution system can be pure water.

### 3.3 Annealing

The sense strand (SS) was mixed with the antisense strand (AS) at a molar ratio (SS/AS = 1/1.05) according to the table below. The mixture was heated in a water bath to 70-95°C for 3-5 min, and then allowed to cool naturally to room temperature. The system was freeze-dried to obtain the product.

The siRNA sequences used in the present invention are as follows:

| **Double Strand No.** | **Sequence (5'->3') (The first one is the sense strand, and the second one is the antisense strand.)** |
|---|---|
| DR005961 | CfsCmsUfGmGfAmCfAmUfUfCfAmGfAmAfCmAfAmGfAmAf-L96 |
| | UmsUfsCmUfUmGfUmUfCmUfGmAmAmUfGmUfCmCfAmGfGmsGfsUm |
| DR005962 | CfsCmsUfGmGfAmCfAmUfUfCfAmGfAmAfCmAfAmGfAmAf-L96 |
| | (VPUm)sUfsCmUfUmGfUmUfCmUfGmAmAmUfGmUfCmCfAmGfGmsGfsUm |
| DR007066 | CfsCmsUfGmGfAmCfAmUfUfCfAmGfAmAfCmAfAmGfAmAf-L96 |
| | (SCP1a-U)sUfsCmUfUmGfUmUfCmUfGmAmAmUfGmUfCmCfAmGfGmsGfsUm |
| DR007067 | CfsCmsUfGmGfAmCfAmUfUfCfAmGfAmAfCmAfAmGfAmAf-L96 |
| | (SCP1b-U)sUfsCmUfUmGfUmUfCmUfGmAmAmUfGmUfCmCfAmGfGmsGfsUm |
| DR005969 | CmsAmsCmAmGmAmAfAmCfUfCfAmAmUmAmAmAmGmUmGmAm-L96 |
| | UmsCfsAmCmUmUfUmAfUfUmGmAmGmUfUmUfCmUmGmUmGmsCmsCm |
| DR005970 | CmsAmsCmAmGmAmAfAmCfUfCfAmAmUmAmAmAmGmUmGmAm-L96 |
| | (VPUm)CfAmCmUmUfUmAfUfUmGmAmGmUfUmUfCmUmGmUmGmsCmsCm |
| DR005971 | CmsAmsCmAmGmAmAfAmCfUfCfAmAmUmAmAmAmGmUmGmAm-L96 |
| | (VPUm)sCfsAmCmUmUfUmAfUfUmGmAmGmUfUmUfCmUmGmUmGmsCmsCm |
| DR007075 | CmsAmsCmAmGmAmAfAmCfUfCfAmAmUmAmAmAmGmUmGmAm-L96 |
| | (SCP1a-U)sCfsAmCmUmUfUmAfUfUmGmAmGmUfUmUfCmUmGmUmGmsCmsCm |
| DR007076 | CmsAmsCmAmGmAmAfAmCfUfCfAmAmUmAmAmAmGmUmGmAm-L96 |
| | (SCP1b-U)sCfsAmCmUmUfUmAfUfUmGmAmGmUfUmUfCmUmGmUmGmsCmsCm |
| DR007179 | CfsCmsUfGmGfAmCfAmUfUfCfAmGfAmAfCmAfAmGfAmAf-L96 |
| | (SCP2a-U)sUfsCmUfUmGfUmUfCmUfGmAmAmUfGmUfCmCfAmGfGmsGfsUm |
| DR007798 | CfsCmsUfGmGfAmCfAmUfUfCfAmGfAmAfCmAfAmGfAmAf-L96 |
| | (SCP2b-U)sUfsCmUfUmGfUmUfCmUfGmAmAmUfGmUfCmCfAmGfGmsGfsUm |
| DR007187 | CmsAmsCmAmGmAmAfAmCfUfCfAmAmUmAmAmAmGmUmGmAm-L96 |
| | (SCP2a-U)sCfsAmCmUmUfUmAfUfUmGmAmGmUfUmUfCmUmGmUmGmsCmsCm |
| DR007797 | CmsAmsCmAmGmAmAfAmCfUfCfAmAmUmAmAmAmGmUmGmAm-L96 |
| | (SCP2b-U)sCfsAmCmUmUfUmAfUfUmGmAmGmUfUmUfCmUmGmUmGmsCmsCm |

The abbreviations used herein have the meaning as follows:
A. U, G, and C represent a natural adenine ribonucleotide, a uracil ribonucleotide, a guanine ribonucleotide, and a cytosine ribonucleotide, respectively.

m represents that the left nucleotide is a 2'-OCH₃ modified nucleotide. For example, Am, Um, Gm, and Cm represent 2'-OCH₃ modified A, U, G, and C, respectively.

f represents that the left nucleotide is a 2'-F modified nucleotide. For example, Af, Uf, Gf, and Cf represent 2'-F modified A, U, G, and C, respectively.

"s" or "s-" represents that the flanking two nucleotides and/or delivery moiety are linked by a phosphorothioate group.

L96 represents a GalNAc delivery moiety of the following structure well known in the art, wherein 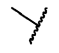 represents a position linked to siRNA by a phosphate group or a phosphorothioate group. See, for example, PCT Publication Nos. WO2009073809 and WO2009082607.

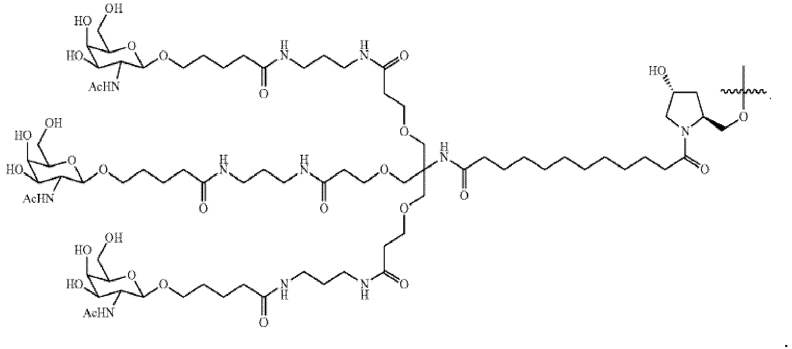

VP represents that the nucleotide adjacent to the right thereof is a vinylphosphonate modified nucleotide. See, for example, PCT Publication Nos. WO2011139702, WO2013033230, and WO2019105419.

SCP1a, SCP1b, SCP2a, and SCP2b represent nucleotide substitutions of the structure described above, wherein Base can be any base. For example, SCP1a-U represents that the Base is uracil.

### EXAMPLE 5 Activity Screening Assay in Primary Mouse Hepatocytes (PMHs) From C57BL/6 Wild-type Mice

### 1. Free Uptake or Transfection

Primary mouse hepatocytes (PMHs) were isolated from C57BL/6 wild-type mice and counted. PMHs were loaded in a 24-well plate at 900 µL/well (i.e., 8×10⁴ cells/well), and in a 96-well plate at 100 µL/well (i.e., 1×10⁴ cells/well). Free uptake or transfection was then selected.

Free uptake: 10 µL of diluted compound was added to 90 µL of Opti-MEM and mixed well. The mixture was added to the corresponding wells and incubated in a 5% CO₂ incubator at 37°C for 24 h. No siRNA was added to control wells.

Transfection: 10 µL of diluted compound was added to 40 µL of Opti-MEM and mixed well. Similarly, 3 µL of RNAiMAX was added to 47 µL of Opti-MEM, mixed well, and incubated for 5 min. The incubated RNAiMAX was then mixed with the diluted compound, allowed to stand at room temperature for 10 min, transferred to the corresponding wells, and incubated in a 5% CO₂ incubator at 37°C for 24 h. No siRNA was added to control wells.

### 2. Fluorescence Quantitative PCR

Total RNA was extracted by the magnetic bead method using a high-throughput nucleic acid extractor (FireGen, FG0412; Hangzhou Allsheng, Auto-pure96), and determined by real-time quantitative PCR assay (TagMan^{™} Fast Advanced Master Mix (ABI, 4444965)) after reverse transcription (PrimeScript^{™} II 1st Strand cDNA Synthesis Kit (Takara, 6210B)).

**Table 1 Primer Information**

| Primer Name | Sequence Information (5'-3') | Fluorophore |
|---|---|---|
| mAPOB-PF | GAGGCTTGTCACCCTTCTTT | |
| mAPOB-PR | GCCTTGTGAGCACCAGTATTA | |
| mAPOB-P | ATCCCTCACTTCCCAGGGTGTAGAAT | 5'6-FAM, 3'BHQ1 |
| mFXII-PF-MGB | AACTACCTGGCTTGGATCCAGAA | |
| mFXII-PR-MGB | CCCAAGGAGCACACAAGGA | |
| mFXII-P-MGB | CATATTGCTTCATAACTAAC | 5'6-FAM, 3'MGB |
| mGAPDH-PF | CGGCAAATTCAACGGCACAG | |
| mGAPDH-PR | CCACGACATACTCAGCACCG | |
| mGAPDH-P | ACCATCTTCCAGGAGCGAGACCCCACT | 5'TET, 3'BHQ2 |

### 3. Data Statistics

The 2^{-△△Ct} value was calculated and converted into a percentage to obtain the residual inhibition; △△Ct = [(Cttarget gene of experimental group - Ctinternal reference of experimental group) - (Cttarget gene of control group - Ctinternal reference of control group)]. wherein the target gene was mAPOB or mFXII, and the control gene was mGAPDH.

### 4. Test Results of mAPOB

Using the transfection method, primary mouse hepatocytes (PMHs) from C57BL/6 wild-type mice were selected and loaded into a 24-well plate. The compound was serially diluted 10-fold, starting from an initial concentration of 40 nM, to obtain five concentration points (40 nM, 4 nM, 0.4 nM, 0.04 nM, and 0.004 nM) for screening IC₅₀ activity at 5 concentration points in PMHs from C57BL/6 wild-type mice. The test results are shown in Table 2.

**Table 2 IC₅₀ Activity Screening Results at Five Concentration Points for siRNA Compound Targeting mAPOB Gene with Nucleotides of the Present Invention in PMHs**

| Transfection | **Duplex No.** | 40nM | 4nM | 0.4nM | 0.04nM | 0.004nM | **IC₅₀ (nM)** |
|---|---|---|---|---|---|---|---|
| 1 | DR005961 | 3.6% | 6.8% | 29.5% | 54.4% | 87.3% | 0.0617 |
| 2 | DR005962 | 4.1% | 5.9% | 10.5% | 35.6% | 68.8% | 0.0174 |
| 3 | DR007066 | 3.2% | 5.4% | 9.8% | 35.5% | 58.2% | 0.0123 |
| 4 | DR007067 | 4.9% | 8.0% | 24.5% | 62.1% | 83.3% | 0.0813 |

### 5. Test Results of mFXII gene

Using the free uptake method, primary mouse hepatocytes (PMHs) from C57BL/6 wild-type mice were selected and loaded into a 96-well plate. The compound was serially diluted 10-fold, starting from an initial concentration of 100 nM, to obtain five concentration points (100 nM, 10 nM, 1 nM, 0.1 nM, and 0.01 nM) for screening IC₅₀ activity-free uptake at 5 concentration points in PMHs from C57BL/6 wild-type mice. The test results are shown in Table 3.

**Table 3 IC₅₀ Activity Screening Results at Five Concentration Points for siRNA Compound Targeting mFXII Gene with Nucleotides of the Present Invention in PMHs**

| Free Uptake | **Duplex No.** | 100nM | 10nM | 1nM | 0.1nM | 0.01nM | **IC₅₀ (nM)** |
|---|---|---|---|---|---|---|---|
| 1 | DR005969 | 0.48 | 0.63 | 0.94 | 0.97 | 1.05 | 50.119 |
| 2 | DR005970 | 0.03 | 0.04 | 0.11 | 0.55 | 0.87 | 0.1230 |
| 3 | DR005971 | 0.03 | 0.05 | 0.18 | 0.64 | 0.98 | 0.1820 |
| 4 | DR007075 | 0.02 | 0.04 | 0.14 | 0.62 | 0.92 | 0.1622 |
| 5 | DR007076 | 0.13 | 0.29 | 0.65 | 0.92 | 0.89 | 2.5704 |

### EXAMPLE 6 Validation of Compound Efficacy in C57BL/6 Mouse Model

C57BL/6 mice (male, 18-21 g, 6-8 weeks old, SiPeiFu (Suzhou) Biotechnology Co., Ltd.) were randomized into 9 animals per group (with livers removed from the mice euthanized on Days 14 and 28, N=3/group). The dose for each animal was calculated based on body weight and administered subcutaneously as a single dose. The siRNA conjugate was provided to CRO in a 10 mg/mL solution (0.9% aqueous sodium chloride solution as solvent). Specifically, before the experiment, the siRNA conjugate was dissolved and diluted to the desired concentration and volume with 0.9% aqueous sodium chloride solution. The dose volume was 5 mL/kg for both the normal saline and siRNA conjugate.

Blood samples were collected before administration (designated as Day -2) for testing. Animals were grouped based on LDL levels on Day 2, and the remaining samples were retained for detection of the target protein. On Days 7, 14, and 21 after administration (with the day of administration designated as Day 0), blood samples were collected from the orbital venous plexus of mice (starved for 5 h before each blood collection). The concentrations of LDL in serum (LDL Cholesterol Detection Agent, MedicalSystem Biotechnology Co., Ltd.) were detected at Anling Biomed (Suzhou) Co., Ltd. using the direct method (automatic biochemistry analyzer, NT-1000, Neusoft Cloud Technology Co., Ltd.) at each time point. The remaining samples were retained for detection of the target protein by ELISA (Mouse ApoB ELISA Kit, Abcam, ab230932). On Days 14, 28, and 42, 10 mg of liver (n=3/group/time point) was removed and placed in RNAlater solution, frozen at -80°C, and sent on dry ice for extraction and detection of liver mRNA expression (see Table 4 for detection primers, to be updated for subsequent time points). The test results are shown in Table 5.

**Table 4 Primer Information**

| Primer Name | Sequence Information (5'-3') | Fluorophore |
|---|---|---|
| mAPOB-PF | GAGGCTTGTCACCCTTCTTT | |
| mAPOB-PR | GCCTTGTGAGCACCAGTATTA | |
| mAPOB-P | ATCCCTCACTTCCCAGGGTGTAGAAT | 5'6-FAM, 3'BHQ1 |
| mGAPDH-PF | CGGCAAATTCAACGGCACAG | |
| mGAPDH-PR | CCACGACATACTCAGCACCG | |
| mGAPDH-P | ACCATCTTCCAGGAGCGAGACCCCACT | 5'TET, 3'BHQ2 |

**Table 5 Efficacy of Three siRNA Compounds in C57BL/6 Mouse Model**

| | | Mean LDL Level (Normalized to Pre-dose Level) | | | | | | | |
|---|---|---|---|---|---|---|---|---|---|
| | | 2 Days Predose | | Day 7 | | Day 14 | | Day 21 | |
| | Compound Name | Mean Percentage | SD | Mean Percentage | SD | Mean Percentage | SD | Mean Percentage | SD |
| 1 | Saline | 100.0% | 30.9% | 94.4% | 20.5% | 85.5% | 17.7% | 73.8% | 7.3% |
| 2 | DR005961 | 100.0% | 21.4% | 55.6% | 13.6% | 81.2% | 13.0% | 73.7% | 9.1% |
| 3 | DR005962 | 100.0% | 21.1% | 36.1% | 9.5% | 47.7% | 11.2% | 54.1% | 5.3% |
| 4 | DR007066 | 100.0% | 20.4% | 29.3% | 11.6% | 42.5% | 13.7% | 59.1% | 12.7% |

### EXAMPLE 7 Activity Screening Assay in Primary Mouse Hepatocytes (PMHs) From C57BL/6 Wild-type Mice

Further compounds of the present invention were tested according to the procedure of Example 3.

**Table 6 IC₅₀ Activity Screening Results at Five Concentration Points for siRNA Compound Targeting mAPOB Gene with Compounds of the Present Invention in PMHs**

| Transfection | Duplex No. | 40nM | 4nM | 0.4nM | 0.04nM | 0.004nM | IC₅₀ (nM) |
|---|---|---|---|---|---|---|---|
| 1 | DR005961 | 17.4% | 27.0% | 76.3% | 93.8% | 99.7% | 0.8841 |
| 2 | DR005962 | 5.5% | 6.9% | 20.0% | 87.7% | 99.5% | 0.1339 |
| 3 | DR007179 | 6.3% | 6.6% | 17.9% | 94.0% | 99.6% | 0.1549 |
| 4 | DR007798 | 10.6% | 15.2% | 48.5% | 92.7% | 98.3% | 0.3149 |

**Table 7 IC₅₀ Activity Screening Results at Five Concentration Points for siRNA Compound Targeting mFXII Gene with Compounds of the Present Invention in PMHs**

| Free Uptake | Duplex No. | 100nM | 10nM | 1nM | 0.1nM | 0.01nM | IC₅₀ (nM) |
|---|---|---|---|---|---|---|---|
| 1 | DR005969 | 9.7% | 30.4% | 82.4% | 112.7% | 140.4% | 4.0272 |
| 2 | DR005971 | 1.2% | 1.5% | 4.6% | 36.2% | 80.2% | 0.0571 |
| 3 | DR007187 | 1.0% | 1.3% | 3.0% | 25.5% | 78.9% | 0.0375 |
| 4 | DR007797 | 2.3% | 2.7% | 10.9% | 55.9% | 78.3% | 0.1282 |

### EXAMPLE 8 Molecular Simulation

Through molecular simulation, we have observed that the compounds of the present invention lock the phosphonate in an *ap* conformation highly similar to the unmodified 5'-phosphate-bound Ago2, wherein the phosphonate group perfectly matches Arg812, Lys570, Lys566, and Lys533, forming multiple salt bridges and hydrogen bonds, as well as two hydrogen bonds with Tyr529 and Cys526, respectively. In addition, the base (uracil) in the spirocyclic phosphonate compound forms two hydrogen bonds with the backbone amides of Thr526 and Gly524, respectively. The combined action of these structures stabilizes the binding of the compounds of the present invention to the Ago2 protein.

The molecular simulation has shown that, similar to spiro compounds, the sugar ring fused with a highly rigid aromatic ring (including five-membered and six-membered aromatic rings) locks the phosphonategroup at the 5' end in a conformation that closely mimics the *ap* conformation of natural 5'-phosphate, with the phosphonate group perfectly matching Arg812, Lys570, Lys566, and Lys533, forming multiple salt bridges and hydrogen bonds, as well as two hydrogen bond with Tyr529 and Cys526, respectively. The base uracil on the sugar ring similarly forms two hydrogen bond with the backbone amides of Thr526 and Gly524, respectively. The combined action of these structures stabilizes the binding of the compounds of the present invention to the Ago2 protein.

## Claims

1. An oligonucleotide, or a pharmaceutically acceptable salt, tautomer or stereoisomer thereof, wherein the oligonucleotide comprises at its 5' end a structure of formula (I): 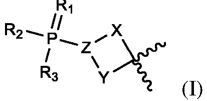 wherein, 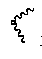
represents attachment to the remainder of the oligonucleotide, preferably to a modified or unmodified nucleoside;
X and Y are each independently selected from CRₐR_{b} or (CRₐR_{b})₂, preferably CRₐR_{b};
Z is CRₐ;
Rₐ and R_{b} are independently selected from H, D, halogen, OH, NH₂, CN, C₁₋₆ alkyl, C₁₋₆ haloalkyl, C₁₋₆ alkoxyl, C₁₋₆ haloalkoxyl, C₂₋₆ alkenyl, or C₂₋₆ alkynyl; wherein Rₐ and R_{b} are optionally further substituted with 1, 2, 3, 4, or 5 independently selected R#;
R₁ is selected from O or S;
R₂ and R₃ are independently selected from OH, SH, NH₂, C₁₋₆ alkyl, C₁₋₆ haloalkyl, C₁₋₆ alkoxyl, or C₁₋₆ haloalkoxyl; wherein R₂ and R₃ are optionally further substituted with 1, 2, 3, 4, or 5 independently selected R#;
R# is selected from H, D, halogen, C₁₋₆ alkyl, C₁₋₆ haloalkyl, C₂₋₆ alkenyl, or C₂₋₆ alkynyl;
wherein each of the aforementioned groups is optionally substituted by 1, 2, 3, 4, 5, or more deuterium atoms or fully deuterated.

2. An oligonucleotide according to claim 1, or a pharmaceutically acceptable salt, tautomer, or stereoisomer thereof, wherein: 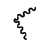
represents attachment to the remainder of the oligonucleotide, preferably to a modified or unmodified nucleoside;
X and Y are each independently selected from CRₐR_{b} or (CRₐR_{b})₂, preferably CRₐR_{b};
Z is CRₐ;
Rₐ and R_{b} are independently selected from H, D, halogen, OH, NH₂, CN, C₁₋₆ alkyl, C₁₋₆ haloalkyl, C₁₋₆ alkoxyl, or C₁₋₆ haloalkoxyl;
R₁ is selected from O or S;
R₂ and R₃ are independently selected from OH, SH, C₁₋₆ alkoxyl, or C₁₋₆ haloalkoxyl; wherein R₂ and R₃ are optionally further substituted with 1, 2, or 3 independently selected R#;
R# is selected from H, D, halogen, C₁₋₆ alkyl, or C₁₋₆ haloalkyl.

3. An oligonucleotide according to claim 1 or 2, or a pharmaceutically acceptable salt, tautomer, or stereoisomer thereof, wherein: 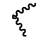
represents attachment to the remainder of the oligonucleotide, preferably to a modified or unmodified nucleoside;
X and Y are each independently selected from CRₐR_{b} or (CRₐR_{b})₂, preferably CRₐR_{b};
Z is CRₐ;
Rₐ and R_{b} are independently selected from H, D, halogen, C₁₋₄ alkyl, or C₁₋₄ haloalkyl;
R₁ is selected from O or S;
R₂ and R₃ are independently selected from OH or SH, preferably OH.

4. An oligonucleotide according to any one of claims 1 to 3, or a pharmaceutically acceptable salt, tautomer, or stereoisomer thereof, wherein: 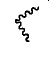
represents attachment to the 5'-end group of the oligonucleotide, preferably to a modified or unmodified nucleoside;
X and Y are CH₂;
Z is CH;
R₁ is O;
R₂ and R₃ are OH.

5. An oligonucleotide according to claim 1, or a pharmaceutically acceptable salt, tautomer, or stereoisomer thereof, which has a structure of formula (II): 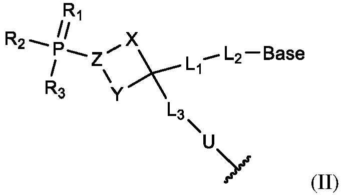 wherein, 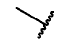
represents attachment to the remainder of the oligonucleotide;
X and Y are CRₐR_{b};
Z is CRₐ;
L₁ is selected from a bond, O, S, or C₁₋₄ alkylene;
L₂ and L₃ are independently selected from C₁₋₄ alkylene;
L₁, L₂, and L₃ are optionally independently substituted with 1, 2, 3, 4, or 5 independently selected R#;
U is selected from O, S, CRₐR_{b}, or NR_{c};
Rₐ and R_{b} are independently selected from H, D, halogen, OH, NH₂, CN, C₁₋₆ alkyl, C₁₋₆ haloalkyl, C₁₋₆ alkoxyl, C₁₋₆ haloalkoxyl, C₂₋₆ alkenyl or C₂₋₆ alkynyl;
R_{c} is independently selected from H, C₁₋₆ alkyl, C₁₋₆ haloalkyl, C₂₋₆ alkenyl, or C₂₋₆ alkynyl;
Rₐ, R_{b}, and R_{c} are optionally further substituted with 1, 2, 3, 4, or 5 independently selected R#;
alternatively, L₂ and L₃ are connected together, and form a C₅₋₆ cycloalkyl or 5-membered to 6-membered heterocyclyl with L₁ and its adjacent carbon atom; preferably U, L₁, L₂, and L₃ and their adjacent carbon atom form a ribose or deoxyribose, which is optionally substituted with R₅;
R₁ is selected from O or S;
R₂ and R₃ are independently selected from OH, SH, NH₂, C₁₋₆ alkyl, C₁₋₆ haloalkyl, C₁₋₆ alkoxyl, or C₁₋₆ haloalkoxyl; wherein the R₂ and R₃ are optionally substituted with 1, 2, 3, 4, or 5 independently selected R#;
R₅ is selected from H, D, halogen, C₁₋₆ alkoxyl, or C₁₋₆ haloalkoxyl;
R# is selected from H, D, halogen, C₁₋₆ alkyl, C₁₋₆ haloalkyl, C₂₋₆ alkenyl, or C₂₋₆ alkynyl;
Base is selected from H, or a modified or unmodified base;
wherein each of the aforementioned groups is optionally substituted by 1, 2, 3, 4, 5, or more deuterium atoms or fully deuterated.

6. An oligonucleotide according to claim 5, or a pharmaceutically acceptable salt, tautomer, or stereoisomer thereof, wherein: 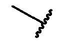
represents attachment to the remainder of the oligonucleotide;
X and Y are CRₐR_{b};
Z is CRₐ;
Rₐ and R_{b} are independently selected from H, D, halogen, OH, NH₂, CN, C₁₋₆ alkyl, C₁₋₆ haloalkyl, C₁₋₆ alkoxyl, or C₁₋₆ haloalkoxyl;
L₁ is selected from a bond, O, or C₁₋₄ alkylene;
L₂ and L₃ are independently selected from C₁₋₄ alkylene;
L₁, L₂, and L₃ are optionally independently substituted with 1, 2, or 3 independently selected R#;
U is selected from O, S, NH, or CH₂;
alternatively, L₂ and L₃ are connected together, and form a C₅₋₆ cycloalkyl or 5-membered to 6-membered heterocyclyl with L₁ and its adjacent carbon atom; preferably U, L₁, L₂, and L₃ and their adjacent carbon atom form a ribose or deoxyribose, which is optionally substituted with R₅;
R₁ is selected from O or S;
R₂ and R₃ are independently selected from OH, SH, C₁₋₆ alkoxyl, or C₁₋₆ haloalkoxyl; wherein the R₂ and R₃ are optionally substituted with 1, 2, or 3 independently selected R#;
R₅ is selected from H, D, C₁₋₆ alkyl, C₁₋₆ haloalkyl, C₁₋₆ alkoxyl, or C₁₋₆ haloalkoxyl;
R# is selected from H, D, halogen, C₁₋₆ alkyl, and C₁₋₆ haloalkyl;
Base is selected from H, or a modified or unmodified base.

7. An oligonucleotide according to claim 5 or 6, or a pharmaceutically acceptable salt, tautomer, or stereoisomer thereof, wherein: 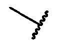
represents attachment to the remainder of the oligonucleotide;
X and Y are CRₐR_{b};
Z is CRₐ;
Rₐ and R_{b} are independently selected from H, D, halogen, C₁₋₄ alkyl, or C₁₋₄ haloalkyl;
L₁ is selected from a bond, or C₁₋₄ alkylene;
L₂ and L₃ are independently selected from C₁₋₄ alkylene;
U is selected from O or S;
R₁ is selected from O or S;
R₂ and R₃ are independently selected from OH or SH, preferably OH;
Base is selected from

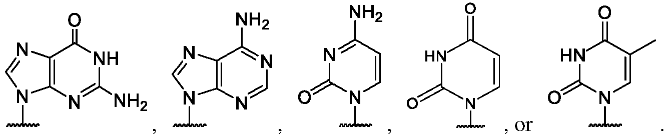

8. An oligonucleotide according to any one of claims 5 to 7, or a pharmaceutically acceptable salt, tautomer, or stereoisomer thereof, wherein: 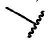
represents attachment to the remainder of the oligonucleotide;
X and Y are CH₂;
Z is CH;
L₁ is a bond;
L₂ and L₃ are independently selected from C₁₋₂ alkylene;
U is O;
R₁ is O;
R₂ and R₃ are OH;
Base is selected from

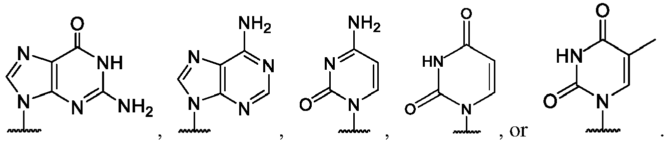

9. An oligonucleotide of claim 1, or a pharmaceutically acceptable salt, tautomer or stereoisomer thereof, wherein the oligonucleotide comprises at its 5' end a nucleotide monomer of formula (I), (IV), or (V): 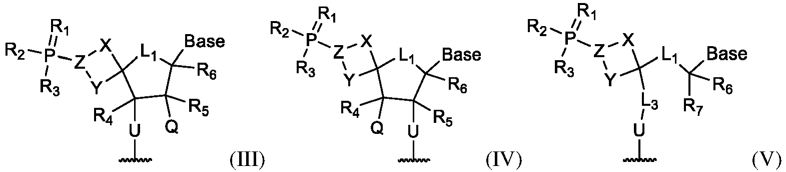 wherein,
Q is selected from H, D, halogen, OH, C₁₋₆ alkyl, C₁₋₆ haloalkyl, C₁₋₆ alkoxyl, C₁₋₆ haloalkoxyl, C₂₋₆ alkenyl, or C₂₋₆ alkynyl;
R₄. R₅, R₆ and R₇ are independently selected from H, D, halogen, OH, C₁₋₆ alkyl, C₁₋₆ haloalkyl, C₁₋₆ alkoxyl, C₁₋₆ haloalkoxyl, C₂₋₆ alkenyl or C₂₋₆ alkynyl;
Base is selected from H, or a modified or unmodified base;
L₁. L₃, X, Y, Z, R₁, R₂, R₃, and U are as defined in any one of claims 5 to 8;
wherein each of the aforementioned groups is optionally substituted by 1, 2, 3, 4, 5, or more deuterium atoms or fully deuterated.

10. An oligonucleotide according to claim 9, or a pharmaceutically acceptable salt, tautomer, or stereoisomer thereof, wherein:
Q is selected from H, D, halogen, OH, C₁₋₆ alkyl, C₁₋₆ haloalkyl, C₁₋₆ alkoxyl, or C₁₋₆ haloalkoxyl;
R₄. R₅, R₆ and R₇ are independently selected from H, D, halogen, C₁₋₆ alkyl, C₁₋₆ haloalkyl, C₂₋₆ alkenyl, or C₂₋₆ alkynyl;
Base is selected from H, or a modified or unmodified base, preferably 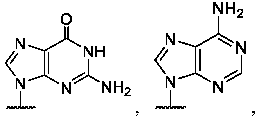 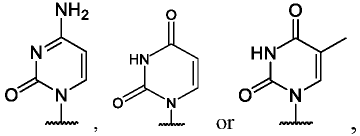
L₁. L₃, X, Y, Z, R₁, R₂, R₃, and U are as defined in any one of claims 5 to 8.

11. An oligonucleotide according to claim 9 or 10, or a pharmaceutically acceptable salt, tautomer, or stereoisomer thereof, wherein:
Q is selected from H, halogen, or C₁₋₄ alkoxyl, preferably H, F, or methoxyl;
R₄. R₅, R₆, and R₇ are independently selected from H, halogen, C₁₋₄ alkyl, or C₁₋₄ haloalkyl, preferably H;
Base is selected from 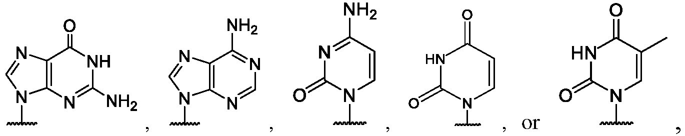 preferably 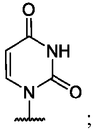
L₁. L₃, X, Y, Z, R₁, R₂, R₃, and U are as defined in any one of claims 5 to 8.

12. An oligonucleotide according to claims 9 to 11, or a pharmaceutically acceptable salt, tautomer, or stereoisomer thereof, wherein the oligonucleotide comprises at its 5' end a nucleotide monomer having the following structure: 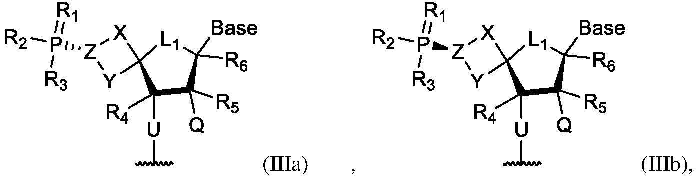 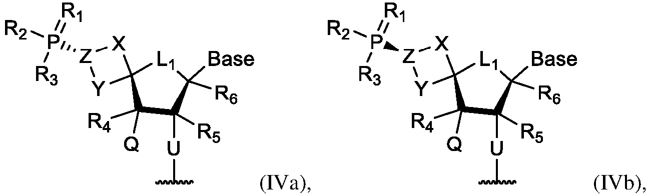 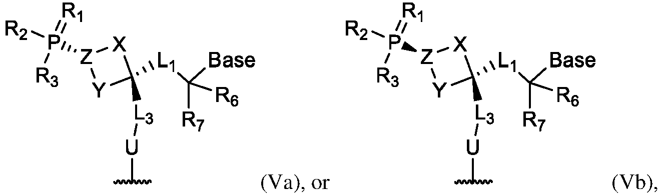 wherein each group is as defined in any one of claims 9 to 11.

13. An oligonucleotide according to claims 9 to 12, or a pharmaceutically acceptable salt, tautomer, or stereoisomer thereof, wherein the oligonucleotide comprises at its 5' end a nucleotide monomer having the following structure: 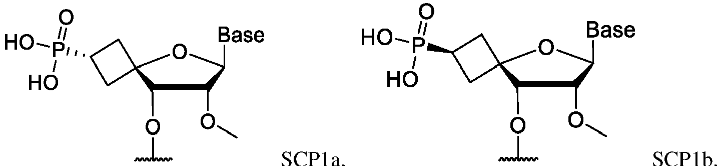 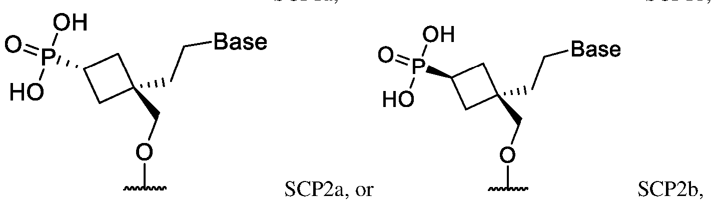 wherein, Base is selected from

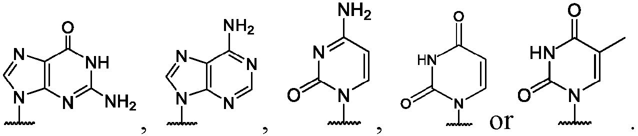

14. A compound of formula (VI), (VII), or (VIII), or a pharmaceutically acceptable salt, tautomer or stereoisomer thereof: 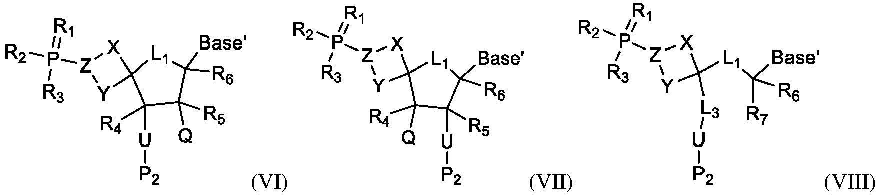 wherein,
R₁ is selected from O or S;
R₂ and R₃ are independently selected from OR_{d}, OP₁, SR_{d}, SP₁, or NRₑR_{f};
R_{d} is selected from H, C₁₋₆ alkyl, C₁₋₆ haloalkyl, C₂₋₆ alkenyl, or C₂₋₆ alkynyl, wherein the R_{d} is optionally substituted with D, halogen, C₁₋₆ alkyl, or C₁₋₆ haloalkyl or fully deuterated;
Rₑ and R_{f} are independently selected from H, C₁₋₆ alkyl, or C₁₋₆ haloalkyl, wherein the Rₑ and R_{f} may be optionally substituted with D, halogen, C₁₋₆ alkyl, or C₁₋₆ haloalkyl or fully deuterated;
P₁ is selected from a protecting group, preferably a hydroxy-protecting group;
P₂ is selected from a reactive phosphorus group, preferably -P(OCH₂CH₂CN)(N(iPr)₂);
Base' is independently selected from H, a modified or unmodified base or leaving group;
L₁, L₃, X, Y, Z, U, Q, R₄, R₅, R₆, and R₇ are as defined in any one of claims 5 to 11;
wherein each of the aforementioned groups is optionally substituted by 1, 2, 3, 4, 5, or more deuterium atoms or fully deuterated.

15. A compound according to claim 14, or a pharmaceutically acceptable salt, tautomer, or stereoisomer thereof, wherein:
R₁ is selected from O or S;
R₂ and R₃ are independently selected from OR_{d}, OP₁, SR_{d}, SP₁, or NRₑR_{f};
R_{d} is selected from H, C₁₋₄ alkyl, or C₁₋₄ haloalkyl, wherein the R_{d} is optionally substituted with D or halogen or fully deuterated;
Rₑ and R_{f} are independently selected from H, C₁₋₄ alkyl, or C₁₋₄ haloalkyl, wherein the Rₑ and R_{f} may be optionally substituted with D or halogen or fully deuterated;
P₁ is selected from a protecting group, preferably a hydroxy-protecting group;
P₂ is selected from a reactive phosphorus group, preferably -P(OCH₂CH₂CN)(N(iPr)₂);
Base' is selected from 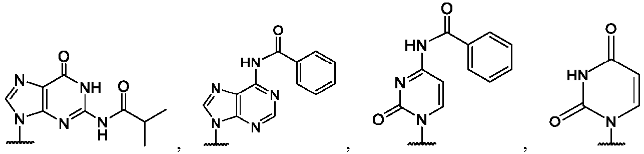 or 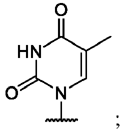
L₁, L₃, X, Y, Z, U, Q, R₄, R₅, R₆, and R₇ are as defined in any one of claims 5 to 11.

16. A compound according to claim 14 or 15, or a pharmaceutically acceptable salt, tautomer, or stereoisomer thereof, wherein:
R₁ is selected from O or S, preferably O;
R₂ and R₃ are independently selected from OR_{d} or OP₁;
R_{d} is selected from H, C₁₋₄ alkyl, or C₁₋₄ haloalkyl;
Rₑ and R_{f} are independently selected from H, C₁₋₄ alkyl, or C₁₋₄ haloalkyl;
P₁ is selected from a protecting group, preferably a hydroxy-protecting group;
P₂ is selected from a reactive phosphorus group, preferably -P(OCH₂CH₂CN)(N(iPr)₂);
Base' is selected from 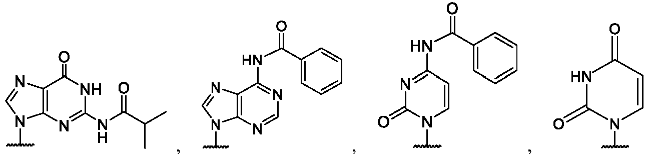 or 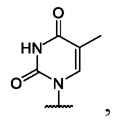 more preferably 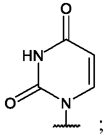
L₁, L₃, X, Y, Z, U, Q, R₄, R₅, R₆, and R₇ are as defined in any one of claims 5 to 11.

17. **A** compound according to any one of claims 14 to 16, or a pharmaceutically acceptable salt, tautomer or stereoisomer thereof, wherein the compound is selected from: 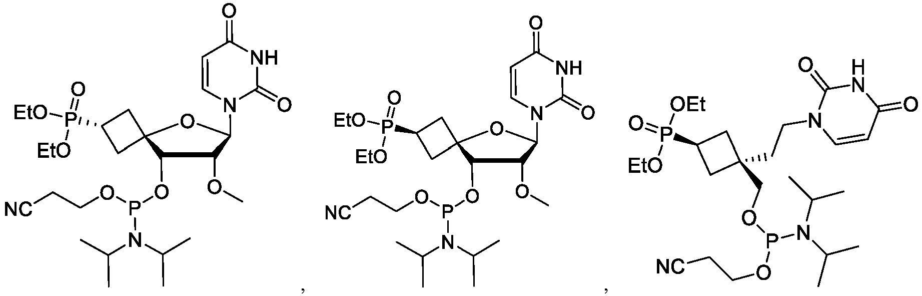 or

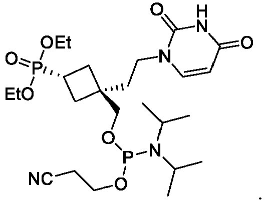

18. An oligonucleotide according to any one of claims 1 to 13, wherein the oligonucleotide is single stranded with 14 to 30 nucleotides.

19. An oligonucleotide according to any one of claims 1 to 13, wherein the oligonucleotide is a double-stranded RNA comprising a sense strand and an antisense strand, each strand having 14 to 30 nucleotides; wherein the antisense strand has a sequence sufficiently complementary to the sense strand and target mRNA.

20. An oligonucleotide according to claim 19, wherein the double-stranded RNA comprises in its antisense strand a structure of formula (I), (II), (III), (IV), (V), (IIIa), (IIIb), (IVa), (IVb), (Va), or (Vb) according to any one of claims 1 to 12 or a nucleotide monomer according to claim 13.

21. An oligonucleotide according to any one of claims 19 to 20, wherein the double-stranded RNA is further conjugated to a ligand; preferably, the ligand comprises one or more GalNAc.

22. A nucleic acid molecule comprising in its nucleotide sequence one or more nucleotide monomers according to any one of claims 9 to 13.

23. A nucleic acid molecule according to claim 22, wherein the nucleic acid is selected from DNA, RNA, and a DNA/RNA hybrid.

24. A nucleic acid molecule according to claim 23, wherein the nucleic acid molecule is single- or double-stranded.

25. A nucleic acid molecule according to any one of claims 22 to 24, wherein the nucleic acid molecule is selected from small interfering RNA (siRNA) and short hairpin RNA (shRNA).

26. A cell comprising a dsRNA of any one of claims 19 to 21.

27. A pharmaceutical composition comprising the double-stranded RNA molecule according to any one of claims 19 to 21 and a pharmaceutically acceptable carrier or excipient.

28. A kit comprising the double-stranded RNA molecule according to any one of claims 19 to 21.

29. A method for inhibiting the expression of a target gene in a cell, comprising a step of introducing the double-stranded RNA molecule according to any one of claims 19 to 21 into the cell.
